(19) Europäisches Patentamt
European Patent Office
Office européen des brevets

(11) EP 2 392 668 B1

(12) # EUROPÄISCHE PATENTSCHRIFT

(45) Veröffentlichungstag und Bekanntmachung des Hinweises auf die Patenterteilung:
**29.10.2014 Patentblatt 2014/44**

(51) Int Cl.:
***C12Q 1/68*** *(2006.01)*

(21) Anmeldenummer: **10196834.5**

(22) Anmeldetag: **28.02.2008**

(54) **Kontrollgene zur Normalisierung von Genexpressionsanalysedaten**

Control gene for normalising gene expression analysis data

Gènes de contrôle pour la normalisation de données d'analyse d'expression génique

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **02.03.2007 DE 102007010252**

(43) Veröffentlichungstag der Anmeldung:
**07.12.2011 Patentblatt 2011/49**

(62) Dokumentnummer(n) der früheren Anmeldung(en) nach Art. 76 EPÜ:
**08716110.5 / 2 118 315**

(73) Patentinhaber: **Analytik Jena AG**
**07745 Jena (DE)**

(72) Erfinder:
- **Rußwurm, Stefan**
  **07743, Jena (DE)**
- **Saluz, Hans Peter**
  **07743, Jena (DE)**
- **Deigner, Hans-Peter**
  **68623, Lampertheim (DE)**

(74) Vertreter: **Winter, Brandl, Fürniss, Hübner, Röss, Kaiser, Polte - Partnerschaft mbB**
**Patent- und Rechtsanwaltskanzlei**
**Alois-Steinecker-Strasse 22**
**85354 Freising (DE)**

(56) Entgegenhaltungen:
**WO-A-2004/087949 DE-A1- 10 336 511**

- **DATABASE EMBL [Online] 10. Dezember 2003 (2003-12-10), "Homo sapiens proline rich Gla (G-carboxyglutamic acid) 4 (transmembrane), mRNA (cDNA clone MGC:74683 IMAGE:5183337), complete cds.", XP002660952, gefunden im EBI accession no. EM_HUM:BC063393 Database accession no. BC063393**
- **VANDESOMPELE J. ET AL.: "Accurate normalization of real-time quantitative RT-PCR data by geometric averaging of multiple internal control genes", GENOME BIOLOGY, BIOMED CENTRAL LTD., LONDON, GB, Bd. 3, Nr. 7, 18. Juni 2002 (2002-06-18), XP021021149, ISSN: 1465-6906**
- **SILVER NICHOLAS ET AL.: "Selection of housekeeping genes for gene expression studies in human reticulocytes using real-time PCR", BMC MOLECULAR BIOLOGY, Bd. 7, Oktober 2006 (2006-10), XP002492790, ISSN: 1471-2199**
- **CHINNAIYAN A.M. ET AL.: "MOLECULAR SIGNATURES OF SEPSIS MULTIORGAN GENE EXPRESSION PROFILES OF SYSTEMIC INFLAMMATION", AMERICAN JOURNAL OF PATHOLOGY, PHILADELPHIA, PA, US, Bd. 159, Nr. 4, 1. Oktober 2001 (2001-10-01), Seiten 1199-1209, XP008037039, ISSN: 0002-9440**
- **WARRINGTON J.A. ET AL.: "Comparison of human adult and fetal expression and identification of 535 housekeeping/maintenance genes", PHYSIOLOGICAL GENOMICS, Bd. 2, Mai 2000 (2000-05), Seiten 143-147, XP002492791, ISSN: 1094-8341**
- **"Human Genome U95Av2", INTERNET CITATION, 2 October 2002 (2002-10-02), XP002215481, Retrieved from the Internet: URL:http: //www.affymetrix.com [retrieved on 2002-10-02]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- "GeneChip Human Genome U133 Set", INTERNET CITATION, 26 February 2003 (2003-02-26), XP002232760, Retrieved from the Internet: URL:http://www.affymetrix.com/support/tech nical/datasheets/hgu133_datasheet.pdf [retrieved on 2003-02-26]
- CONSTANTINE L ET AL: "Use of genechip high-density oligonucleotide arrays for gene expression monitoring", LIFE SCIENCE NEWS, AMERSHAM LIFE SCIENCE, US, 1 January 1998 (1998-01-01), pages 11-14, XP002964122, ISSN: 0969-0190

SEQUENZ Protokoll

<110> SIRS-Lab GmbH

<120> Kontrollgene zur Normalisierung von Genexpressionsanalysedaten

<130> SL0630

<140>
<141>

<160> 107

<170> Patent Prepare 0.5.2

<210> 1
<211> 67
<212> DNA
<213> Homo sapiens

<400> 1

```
gagttagagg ccagcctggc gaaaccccat ctctactaaa aatacaaaat ccaggcgtgg    60
tggcaca                                                              67
```

<210> 2
<211> 69
<212> DNA
<213> Homo sapiens

<400> 2

ttataggtgt gagctactgt acccagcctt aacctgtttc acagttgatt atacttcatg 60

ctgttttcc 69

<210> 3
<211> 69
<212> DNA
<213> Homo sapiens

<400> 3

ccacactacc acattaaaaa aattagaaag tagccacgta tggtggctca tgtctataat 60

cccagcact 69

<210> 4
<211> 64
<212> DNA
<213> Homo sapiens

<400> 4

cccaaatgct gggattacag acatgaacca ccacgcctgg ctggaatact tactcttgtc 60

ggga 64

<210> 5
<211> 65
<212> DNA
<213> Homo sapiens

<400> 5

acgtagatag aggtggagac aggaaaaaga ctaagccaga cgtggtggct cacacctgta 60

atccc 65

<210> 6
<211> 70
<212> DNA
<213> Homo sapiens

<400> 6

gttcaaaacg aagactagct attaaaattt catgccgggc gcagtggctc acgcctgtaa 60

tcccagccct 70

<210> 7
<211> 70
<212> DNA
<213> Homo sapiens

<400> 7

cttggcctcc caaagtgcta gtattatggg cgtgaaccac catgcccagc cgaaaagctt 60

ttgaggggct 70

<210> 8
<211> 19
<212> DNA
<213> Homo sapiens

<400> 8
tgacagagcc agtgggaag 19

<210> 9
<211> 19
<212> DNA
<213> Homo sapiens

<400> 9
aggtgtgagc tactgtacc 19

<210> 10
<211> 20
<212> DNA
<213> Homo sapiens

<400> 10
gctaaattcc acactaccac 20

<210> 11
<211> 20
<212> DNA
<213> Homo sapiens

<400> 11
ccacgctcgt ctccaactcc 20

<210> 12
<211> 20
<212> DNA
<213> Homo sapiens

<400> 12
cactgtgcct gagctctgac 20

<210> 13
<211> 20
<212> DNA
<213> Homo sapiens

<400> 13
gatgaattgg gggatagatc 20

<210> 14
<211> 19
<212> DNA
<213> Homo sapiens

<400> 14
gagatggggt ttcaccatc 19

<210> 15
<211> 19

<212> DNA
<213> Homo sapiens

<400> 15
caattctcct acctcaacc 19

<210> 16
<211> 19
<212> DNA
<213> Homo sapiens

<400> 16 19
ggattacagg catgcaacc 19

<210> 17
<211> 20
<212> DNA
<213> Homo sapiens

<400> 17
ttgagtgcag cggtgtgaac 20

<210> 18
<211> 21
<212> DNA
<213> Homo sapiens

<400> 18
ccacagcata atgaattctg c 21

<210> 19
<211> 20
<212> DNA
<213> Homo sapiens

<400> 19
tgttggccag gctggtttcg 20

<210> 20
<211> 19
<212> DNA
<213> Homo sapiens

<400> 20
cctgacctct ggtgatctg 19

<210> 21
<211> 21
<212> DNA
<213> Homo sapiens

<400> 21
ttagaaaagt cctagaaatg c 21

<210> 22
<211> 2015
<212> RNA
<213> Homo sapiens

<400> 22

cccggaccga ggcaggacct caccccgcgc gtgttccccg ggcgcccctc tgcgaacccc 60
aggcccttcc caggtttgcg cgcgggggcc atccagaccc tgcggagagc gaggcccgga 120
gcgtcgccga ggtttgaggg cgccggagac cgagggcctg gcggccgaag gaaccgcccc 180
aagaagagcc tctggcccgg gggctgctgg aacatgtgcg gggggacaca gtttgtttga 240
cagttgccag actatgttta cgcttctggt tctactcagc caactgccca cagttaccct 300
ggggtttcct cattgcgcaa gaggtccaaa ggcttctaag catgcgggag aagaagtgtt 360
tacatcaaaa gaagaagcaa actttttcat acatagacgc cttctgtata atagatttga 420
tctggagctc ttcactcccg gcaacctaga aagagagtgc aatgaagaac tttgcaatta 480
tgaggaagcc agagagattt ttgtggatga agataaaacg attgcatttt ggcaggaata 540
ttcagctaaa ggaccaacca caaaatcaga tggcaacaga gagaaaatag atgttatggg 600
ccttctgact ggattaattg ctgctggagt atttttggtt atttttggat tacttggcta 660
ctatcttttgt atcactaagt gtaataggct acaacatcca tgctcttcag ccgtctatga 720
aagggggagg cacactccct ccatcatttt cagaagacct gaggaggctg ccttgtctcc 780
attgccgcct tctgtggagg atgcaggatt accttcttat gaacaggcag tggcgctgac 840
cagaaaacac agtgtttcac caccaccacc atatcctggg cacacaaaag gatttagggt 900
atttaaaaaa tctatgtctc tcccatctca ctgactacct tgtcattttg gtataagaaa 960
tttgtgttat ttgataggcc gggcatggtg gctcatgcct gtaatcccag cactttggga 1020
ggccaggagt tcgagaccag cctggccaac atggtgaaac ccggtctcta ctaaaaattc 1080
aaaaattacc taggcgtcat ggggcatgcc tgtagtccca cctacttggg aggctgaagc 1140
aggagaattg ctcgaacctg ggaggcagag gttgcagtaa gctgagatca cgccactgca 1200
ttccagcctg ggcgacagag caagactcca tctcaaaaat aaaataaaaa aagaaagaaa 1260
gaaaagaaga agaaaagaga agaaggagaa ggagatgaag gaggaggagg aggagaagga 1320
gaagaagaag aagaagaaga ccacaaaaga catgactatc caacttttta tgacaaactg 1380
caaggaataa aggaagaata agtccatgta ctgtaccaca gaagttctgt ctgcatcttg 1440

gacctgaact tgatcattat cagcttgata agagactttt tgactctata tccttgcagt 1500

taagaagaaa gcactttttt gtaatgtttg ttttaatggt tcaaaaaaaa tctttcttat 1560

aaagagcata ggtagaatta gtgaactctt tggatccttt gtacagataa aggttataga 1620

tttcttgtgt tgaatattaa aaaagcaagg atgtctaacc attaagatta tccaaagtca 1680

ggctgggcgc agtggctcac gcctgtaatc ccagcacttt gggagggata ggtgggcgga 1740

tcacctgagg tcaggagttt gagaccagcc tggccaacat ggcaaaaccc cgtctctaca 1800

aaaatacaaa agaaattagc cagacatgat ggcgggtgcc tctaatccca gctactgggg 1860

aggctgaggt gggagaatcg cttgaactcg ggaggtggag gttgtagtga ggcgagattg 1920

tgccattgca ctccaacctg ggcgacagag tgagactcca tctcaaaaaa aaaaaaaaaa 1980

aaaaaaaaaa aaaaaaaaaa aaaaaaaaaa aaaaa 2015

<210> 23
<211> 356
<212> RNA
<213> Homo sapiens

<400> 23

gttttgtttg ttttgttttt ttttttaata ttttttaaga gctgtaaaga aggagaagag 60

gaatgagaaa atgagaaaga attattatta ttattggtgg tagtagtgat agagactgta 120

tgtggcctat aaaggctaac atattcactg tctgaccctt tagagaaagt ttgtcaaccc 180

ctggcctaga acatgggtgg cttcttacta gggctcagta agtgtctgaa tgaaggaagg 240

aacagtttaa aactcagctt tgccgggcgc agtggctcac gcctgcaatc ccagcaccct 300

gggaggccga ggcgggcgga tcatgaggtc aggagttcga gaccagcccg gccaac 356

<210> 24
<211> 451
<212> RNA
<213> Homo sapiens

<400> 24

aatggagacg agttcttact atgttgccca ggcaagtctc aaactcctgg gttcaagcga 60

ctctcccacc tcactctccc aaagtgttgg gattacaggc gtgaggcact gcacctggcc 120

taatccacaa actgtctaga agcaaacaac caaacatatc gagaattttt ctgagtgtaa 180

aaataaatct ctttgtggca tgattctatt acagatcact ggtatgcctg attaaagtgg 240

actacaataa agattacata caccagactt taaataattg caatccactg aaataacagc 300

atttactaat ctcagcgaat gctcaattta ttgagcattt acacctgacc aaatgtctta 360

attcaacctt ttactcaatc ctgaatcatc tgtataaatt ggaaataaca gttgtcatac 420

aaactttaag taattccttc actgggtacc n 451

<210> 25

<211> 397
<212> RNA
<213> Homo sapiens

<400> 25

```
tttttttttt tttttgagac gtagtctttc tctgtcacct aggttgaagt gcagtggtgc      60
aatcttggct cactgcaacc tccacctccc aggttgaagc gattctcctg cctcagcctc     120
ctgagtagct gggattacag gcatgcacca tcacacctga ctttgtattt ttagtagaga     180
cggggtttcg ccatgttgcc aggctggtct caaactcctg agctcagcca atctgcccgc     240
cttggcctcc caaaatgctg ggattacagg cgtgacacta gtgcctggcc tggtctttca     300
gtaccatata caagcctgca ataaatctgt ttagacataa tgtcatagaa gtgagtgtat     360
ctgtgggaca aatccctaga attgctgggt caaaggg                              397
```

<210> 26
<211> 457
<212> RNA
<213> Homo sapiens

<400> 26

```
tgaccaggat ctcactcagt cactcaggct agagtgcagt ggcatgatca tggctcacca      60
cagacttgac ctcccagact caggtgattc tcccacctca gcctcccgag tagctacgac     120
tacaggcgtg cgccaccacg cctggactaa tttttccata gaaacggggt tttaccatgt     180
tgccccaggc tggtctcgaa ctcttgtgct taagagatcc tcctgcctca cactcccaaa     240
gtgctgggat tacaggtgtg agccacggtg cctggcctat actatctctt tcaactctct     300
caataactta caaatgaaga aactagggct tacagaggtt aagggttaag taggggcaca     360
tggtaggaaa tcagaattct aacctacatc tatgcaaccc cgacatctgt gctccttcca     420
ttccattaaa aacatgtagg ctgcaaaaaa ccacagg                              457
```

<210> 27
<211> 2811
<212> RNA
<213> Homo sapiens

<400> 27

acaaggcagg atgtgtgcgt gggaggaaga ttgacagtga ctgagcctgg acgggggaga 60
ccaggtatga ggtctgaagc acctggaaca gaaaggacag gacagatgtg ggcacactgc 120
acgtgtagaa tcaaaggact gacagcaggt cgaatgtgag gaatgaggga gggaaagaat 180
caggactcaa gtgccatcct ggctgcctca aaaaatgata ctgtcttcca gagggaaagg 240
aaagataaca atagttactg ctttgtggcg tacatgtgat gaatttcatt ttggacattc 300
cagtaggata tccaagtgga aatgcccagt aagccttaga cataaggatc tggatctcaa 360
gagaaaaatt gaggttgaac cataatatgt ctttcccctc gaatcatgta ggtttctctt 420
ttgccttctt tcattggcct aagtggtcct aaatgctact gctgatgctg tcttagtttg 480
cgactgttgt ttgcacccca ccttttccca aaggtaatct gtagacttgc atggattggg 540
ttaaggtggt taacctgcag ctttgctgtt caaagcttgg cttcccacta ccagtttgcc 600
aacttaatga gtacttcaac ttgagtcaaa ttagtattgt tccaaatatc ctaatagtat 660
cctctatgtg tgactctagg tcttacaaaa tcaaggtgtc ctttctcatt gagacttcct 720

tattaataaa atatttcttc tattaaattc aacctggcac caagcatagt aggtaatagg 780
cacacacaat gactgtttat tgaatgaatg aataaaatga ttatgttagg gcattctgag 840
caattcatcc taagcagcta attttctcct acttctttta ttatagtgtg tgtttgtgtg 900
tgtgtgtgtg tgtgtctgaa atgtcccatc ctacaggttc attaatattt aatagaaatg 960
aaagaagaaa aatacctatt aagtgttttg atttcatcct tttcattgaa ttgaaaaagt 1020
atatcattta ttcctgaaga gaaatctaga ttttgctcta tattaaacat ttgacattta 1080
ttggtcctta atgctaatat agataccagc ctgctggttg tcacattcta tctgtttata 1140
cgaaggttgt agacacacag cgtatgtaca tatgcctagt tgctctcatt ccttttgttt 1200
cacatctcaa gcctaaccca gactgaaaag gttttgaagg ctgagattat tcatcacccc 1260
atcattatag aaagcagggc tggcccaagg ttctcacagt gggagcaagg tggattttaa 1320
ctctgatcag tgttgtagct caaatataaa aagaactgca gcacaaaagt cacaaggata 1380
aatgatcccc tcgttcttct cccataaaaa taagcagcca attgaaggtg gaagtcagta 1440
cagtgcggca ttcccagagg cgacagaacc taagattcca tttctaaaga cactgctcaa 1500
caagaagacc acctgggatg tcttacataa aaccattggc ctggcagctt ttggctgagt 1560
tctctattct ggttcaagcc agcatcacag cctatctgtg gttttaacaa ctgatggaat 1620
ttgtattttg agaaccctca tccgttagca tgaagcaaac tcaaagcatt gttgctcatc 1680
agttgtcatc tgtttgagaa agattttgat ttgtttactt gtagtgaagc ttgaccatac 1740
ttctccaggg gctttttaaa aagatgaatg tgtcagcttg tagatttgtc cccatgaatg 1800
aaaccacaag caaattctct tctctcttcc agcctccctt cctccctctt gtttcttcag 1860
tggccatctg tgcattatgt tcccattgcc aggccctctt caagcagctt atctatgagt 1920
gaattcagaa acttcaaatt ataaaggaca cccagataat tggcctgttc tccaaagtat 1980
ctgtcccctg tgctgctgcc agattccttc ttaatgaata catccagtga cagtgggatt 2040
cttgagcttg tccgtatctg tgagaaaatg agctctcctg ctttgtaaca gcttgtggct 2100
cagggaaaaa aatgacagcc attgcacaag tttcctttga atgtagtttt ctttcccata 2160
aatgatactt tgagaataca gttaaggggt tattagtttt ctatttcatg cctggcctgt 2220
gtgtgagaat aacacaagct gtcactgcaa atcagtagct aaaaatgctt tgtctggtta 2280
atgtgaacat ttaatatttg gctcaattaa aaattaaccg atgaaagtac atgtcattgg 2340
aatttgaaaa taccttttgt acggaatact taaagggcat cacccatgac taaaccagtg 2400
cttttaaaat atggagaata tggggaaatt taatatgagt tgggatactt gactcttttt 2460
taaaacctct ctacctgttt ggcacaacag ggtattgata aagagtgggc tcattgttat 2520
ggcaaaggat tcacttgcat ctctgtgttt ttaagtgggt aattgttttt ttgcactcag 2580
tcacatgatt aaagcagaca gaacaagaga tcagttattc atttatacca tacttttaaa 2640
aaaatattga gccaggccct ggggaagtgg gaagtgagag ccagagcggc gtggctgata 2700
gtctagggca gtgctatcca atcttttggc ttccctgggc cacattggaa gaagaagaat 2760

tgtcttgggc cacacgtaaa atacgctaac acgaatgata gctgatgagc t 2811

<210> 28
<211> 394
<212> RNA
<213> Homo sapiens

<400> 28

tttttggag acggagtcgt ccccgtcacc caagctggaa cgcatggtgt ggatctcggc 60
tcactgcaaa ctctgcctcc caggtttaag tgattctcct gccccagcct cccaagtagc 120
tgggattaca gaagcgcgcc actacaccca gctaattttt gtatttttag tagagacagg 180
gtttcagtat gttggtcagg ctggtctcga actcttgacc tagtgatcca cccgcctcgg 240
cctcccaaag tgctgggatt acaggcgtgc gactgcgtcc ggcctgattc acatatattt 300
taagagacta aacataggaa agctaggaga tcttgtgtgg tggcaggttt cttctgccac 360
tcaggggtag gacactgggg cagggggagt ggcc 394

<210> 29
<211> 497
<212> RNA
<213> Homo sapiens

<400> 29

gcacgagaag agtctcattc caggaaccct ttgtagttag ttggctggca tgtttacttg 60
ctgctgtagc cagccaagat gagtgcacct aggccctcaa aaaggatttt tttttacctc 120
aatctagcct gaccctcata tctgtggttg cctctgagac gaacatccat gctagtataa 180
aaatagttag gaatgccctt ggcagaactg aagctcttat taaatggtgg acagagctct 240
tgccagcttt gatccagccc ctcagtctcg gagttatggg gcagggttgg ggggcagtgc 300
tacactgtaa agaatttcca ggctgggcgc ggtggctcat gcctgtaatc ccagcacttt 360
gggaggccga ggagggcgga tcacaaggtc aagagattga gatcatcctg gccaacatgg 420
tgaaactccg tctctactaa aaatacaaaa attagctggg catggtggca cgtgcctgta 480
gtcccagcta cttggga 497

<210> 30
<211> 206
<212> RNA
<213> Homo sapiens

<400> 30

tgagacagtg tcttactcag ttgggactac aagtgtgtgc caccatgccc ggctatctta 60
tctacctatc gacctgagac agggtctccc cttctgttgc ctgggctgga gtgcaccggt 120
gtgatctcgg ctccctatag cctccacctc ttgggcccaa gtgatcctcc aacctcagtc 180
tcacgagtag ctgggattac agctgc 206

<210> 31
<211> 376
<212> RNA
<213> Homo sapiens

<400> 31

ttttttttt taagtgtcag tgttcataaa ggccctttt ctttttcaag gatgggtata 60
aagtgttact cggccgaacg cggtggctca cacctgtaat tccaacactt tgggattaca 120
ggcgtgagcg accgcgccca gccgaacttc tgcctcttaa atccagggtt ctccctgtca 180
gtacagtgag gtggtaacta gcaaaagcta tgagatatga ctgcctgggt acatatccca 240
gctctttcac ttatctttgt ggctttacgc aaattactta acctctttat gattgtttct 300
tcatttgtaa aaggaagata ataacagtgc ctatatatag ggtttttatg aagaataaat 360
gagatagtat atataa 376

<210> 32
<211> 337
<212> RNA
<213> Homo sapiens

<400> 32

ttttttttt ttttattttt agacaaagtc ttgctctatt gcccaggctg tagtgcagtg 60
gcacaatcat agctcactat aaccctcgac ctcccgggct caagcaatcc tcccacctca 120
gcctcccgaa tagctgggac tacaggcatg caccaccaag cctggctaat ttgctatttt 180
tgtttttcat agagacagag tctggccatg ttgcttaggc aggtttcgaa ttccttgcct 240
cagcctctca aggaatttgc attgtttta atgaaaaaac acacatatgg tgaacagtaa 300
aagtgggaga attgaacagc cctaaaatca agtagtc 337

<210> 33
<211> 381
<212> RNA
<213> Homo sapiens

<400> 33

aagatggagt cttactctgt cgcccagact ggagtggtgc gatctcggct cactgcaacc 60
tccaactcct gggttcaagc aattctcctg cctcagcttt ccaagtagct gggactacag 120
gtgtgcgccg ccacacccag ctaattttg tatttttag tagagacagg gcttcactat 180
atgttggcaa gactggtctc gaacccctga cctcaggtaa tctgcctgcc ttggcttccc 240
taagtgctgg gattacagtt gtgagccacc acgcccagcc agcactacct tttctattgt 300
gcatcctaat ggtctgtagt atagacatat ttatagggga aagaaaggaa tagatgtggg 360
caaaaagaag ctaaaaaaca t 381

<210> 34
<211> 494
<212> RNA
<213> Homo sapiens

<400> 34

ttatttggct aaattattga tcctacttca gagggaaagt gtaccaggca gttttggtgg 60
gtggtgctga agtctgggga gtgagtttag tcttcagact attcttggcg acatcaccag 120
tgttgcaagc accaccattc ccagttaggc actttttgtc cctggtaaga cttgaccttt 180
atctggaaca ctccttttgt ccctagagtg gggacctaag gctcagcaaa agggcagaat 240
caggaaagcc tttatggtgt ggctaaagga gtggccagag ccttgggact cctttgctgc 300
cttctccctg gttccagttg tctttagatt ttcacggctc ttactgctgt tacttaacag 360
tattttccag ccaggcatgg tggttcacgc ctctggtccc tgcactttgg gaggccgagg 420
caggcggatc acctgggatt gggagttcgg gaccagcctg tccaacatgg cgaacctcgt 480
ctcttctgag agta 494

<210> 35
<211> 521
<212> RNA
<213> Homo sapiens

<400> 35

tttttttttt tttttacttg ataatagatt aagatttatt tattcagtaa gcgataacaa 60

ttttgaattt ttatgcctaa tggtattaac ttaaaataat aatacaagca atattgagaa 120

atctataaga aataacagac aaatctataa tcatagaagg aaatttcagc cactgctaga 180

taaggtagac acaaaagtca gtaaggttgc aaaaggtgag cagaatgatt aaaaatttaa 240

caagttggca gggcacagtg gctcatgcat gtaatcccag cactttagga ggccgaggca 300

ggctgatcac gaggtcagga gttcaagacc agcctggcca acatggtaaa accccatctc 360

tactaaaaat acaaaaatta tccgggtgta gtggtgcatg cctgtaatcc cagctactcg 420

ggaggctgag gcaggagaat tgcttgaatc caggagggag agattgtggt gagccaagat 480

tgcctcactg cactccagcc tggggaacag agaaaggccc t 521

<210> 36
<211> 351
<212> RNA
<213> Homo sapiens

<400> 36

tttggtctct ctttttatat ttattaactt ttgtagtaaa taagtataat tttataaatt 60

gtctgaacag aatgggcatg gtggctcatg cctgtaatgc caacacatcc agccttgtat 120

gtgttcttga ccggcatata ttgttagtgt gcatgtattt taactgatat aaatgataag 180

ttccctattg ctatgtgaac atataatcca ttactttgaa tcactacaca ttttcacatt 240

ttacttacct tccccataca gacttccaat tcctgccccc aacaattaat attgtgacca 300

gcatccatac tgtgcctctt gcggtcctct gtgagagctt cctgagagac g 351

<210> 37
<211> 451
<212> RNA
<213> Homo sapiens

<400> 37

tttttgagac acagtcttgc tctgtcaccc gggctggaat acagtggtac aatcttggcc 60

tccacctccc aagttcaagc aattctcctg cctcagcctc ccaagtagct aggattacag 120

gcacccacca ccacgcccgg ttaacttttg gttttaagac ggtgtcttgc tctgttgccc 180

aggctagggt gcaatggtgc catcttggct caccgcaacc tccacctcat gggttcaagc 240

aattctcctg cctcagcctc ccgagtagct gggattacaa gcgcacccca ccacacccgg 300

ctaatttttg tatttttagt agagacggag tttcaccatg ttggccaggc tggtctggaa 360

ctcctgacct caagtgatcc gcccgcctcg gcctcccaag gtgctaggat tacaggcgtg 420

agccaccgct cccagccgca ccgttttttt c 451

<210> 38
<211> 674

<212> RNA
<213> Homo sapiens

<400> 38

```
ttatttggtg ttaaacaggt ttaatgacgg tcatggcaac tttttggcac aatgaaaaat     60
atcgcccatg atcaacgtgt tctgttctgg ggaagggggc aaaggcaggg tgaatcactt    120
tcttaaaaag tatagctcaa gttgggagtg cagagggaat ggggagaaaa cccttccgct    180
gcctgtgtcg aagtgcagga gcccccaccc ccatactcac ctgagtccag cccctctggg    240
gaaagaaggg gtgcatgaac tccccttagt ccacaggcgc ctccctgtgg cccaaggccc    300
tcttcacact ccatcttgta gccccagcag gagctatttt ccgaaaagtg ctgggattac    360
aggcgtgagc cactgtgccc agctgagatc tgatggtttt aaaaagagga gctcccctgc    420
atgagatctc actttttgcc tgctaccatt tatgtaagat gtgacttgct ccttcttgcc    480
ttccatcatg actgtgaagc ttcccccacc atatggaatt gtaagttcaa ttaaacttct    540
tttctttgga anttcnaaag ccctcccttt acacttgcaa agggtcccaa aatacttcct    600
tgaggggggg gccccgtacc ccaattcgcc ctttggtgga gtcgttttaa caattccctg    660
gcccgccgtt ttaa                                                      674
```

<210> 39
<211> 330
<212> RNA
<213> Homo sapiens

<400> 39

```
tttttttaga aagaagtggg gtctcacatg ctgtccaggc tagtctcaaa ctcctgggct     60
caagccatcc tctcacctcg gcctcccaaa gtgctgggat tcaggcatga gccaccactc    120
ccggccctca attaataact tgacttaaga taatctagtt catattaact taatttcata    180
gcatacaaaa actatgcttc atttcttcct tccattattc tatcatgaat atggcacctt    240
tttgtgttat aagcccattg acacagttta taattattgc ttatgcaggt gggtgtcttt    300
taaatcagag ataagagaat aaaatatcta                                    330
```

<210> 40
<211> 446
<212> RNA
<213> Homo sapiens

<400> 40

```
ccagtttgta tttatttatt tatttattta tttagagaca gagtctcgct ctgtcgccta      60
gggggtgca gtggcgcaat ctcagctcac tgcaacctcc acctcccggg ttcaagcgat      120
tctcctgcct cagcctcctg agtagctggg attacaggcg tgtgccacca tgcccagcta      180
atttttgta tttttagtag agacagggtt tcaccgtgtt agccagggtg gtcttgatct      240
cctgacctca tgatccgtcc gcctcagcct cccagagtgc tgggattaca ggcatgagcc      300
actgcgcctg gcccaattta tttttttttg tagtttcatt ctcctcacat ccaaacagct      360
acagctttcc ctccttttgt ggggtcccca aaccaagtct cttttcagga gagcagacat      420
gtgcctccac acagttctga agttcn                                           446
```

<210> 41
<211> 406
<212> RNA
<213> Homo sapiens

<400> 41

```
tttttctga gacggagtct tgctctgtcc cccaggccgg agtgcagtgg cgccatctca      60
tctcactgca agctccgcct cccgggttca cgcccttctc ctgcctcagc ctcccgagtt      120
gctgggacta caggcgcccg ccaccacgcc cggctaattt ttgtattttt agtagagaag      180
gggtttcacc gtgttagcca taatggtctc gatctcctga cctcatgatc cacccgtctc      240
agcctcccaa agtgctagga ttacaggcgt gagccagcgc gcccggccta ccctccctat      300
tttcaaaaac attgtggcaa tggacaaaat tcacatgtac aaccgatcat tacaatcaga      360
cgctctgtga tacgtgtacc aacgacaagg gctgaaataa tgactg                     406
```

<210> 42
<211> 320
<212> RNA
<213> Homo sapiens

<400> 42

```
cacacccagc taacttttg tattttttgt agacagggtt tcaccttatt tctcaggctg      60
gtcttcaact tctgggctca agcaatccac ccgcctcagt cacccaaaat gctaggatta      120
caggcgtgag ccattgcgcc cagcctcaaa actcttctac ctaaaatcac cttcagagcc      180
atgctagaaa attagtatca ttcctttaca atcggaatcc aacttggcca ctaaaatgtt      240
tccttagact tggtcctaaa tgatttttgg attgtttcaa aacctgaaaa acaccttcac      300
aggataaaga taaaagaatg                                                  320
```

<210> 43
<211> 448
<212> RNA
<213> Homo sapiens

<400> 43

tttcttttag acagggtctc actctgttgc ccagactgga atgcagtggt gcagtcttgg 60
ctcactgcag cctcaacgtc ttgggctcaa gcgatcctcc catctcagcc tttcaagtac 120
ttgggactac aggcatgctc caccacatcc agctaatttt tgtattntgc gtaaagatgg 180
nngttttgcc atgctgcttc tcgaactcct ggagggggnc aagtaattct gtccacctca 240
acctacaaaa gtgccggaac tataagcatg agccactgac ccagcttgaa atggtaatnt 300
aataaaatat atcatttatt tttcaaagac tagatctacc catganccac agatctgaat 360
attttaaatt gtcttccctg gtacatcatt gccattacct nnnaatggta cactctacan 420
tatgctannn nnnggtgcat anaangaa 448

<210> 44
<211> 270
<212> RNA
<213> Homo sapiens

<400> 44

ttttttttt tttttgctca cagaatgtat acgtttattt tttaacggag ttaattcatg 60
gccgggtgtt gtggctccca cctgtaatcc cagcactttg ggaggctgag gcgggtggat 120
cacctgaggt caggagttca agatcagcct ggccaaaatg gtgaaacctc atctctacta 180
aaaatacaaa aattagccag gtgtgatggc atgtacctat aatcccagct actcaggagg 240
ctgagacagg agaatcgctt gaatctggga 270

<210> 45
<211> 386
<212> RNA
<213> Homo sapiens

<400> 45

taaaataata gaggcatagt ctctctatgt caggctggtc tcaaaactcc tggcctcaag 60
caatcctccc acctcagcct cccaaagtgc tgggattaca ggcatgagcc actgtgccta 120
gccaacatgg gacatttcta actcgagggt attgtcaggc catgtaggaa agggagcaga 180
gattgccctt gaggagatgc tcccaggtgg cagatttgtc ctacttgata gattccaaaa 240
tggaaaacgg atttttctgc tgcctctggg gacactgaaa aaagaacctc cacatgagtt 300
cagaggcagc accggcagct taggggaagt catggcttcc actgcgtgtc taggaagcgc 360
tctttcagga tgctctgagg ctgcca 386

<210> 46
<211> 413
<212> RNA
<213> Homo sapiens

<400> 46

tggtgagaca gagatttact cttgttgccc aggctggagt gcaatggcat gatctcagct 60
caccgcatcc tccacatcct ccgcctccca ggttcaagtg attctcctgc ctcagcctcc 120
tgagtatctg ggattacagg catgtgccac cacgcccggc taattttgta ctttttagt 180
agagacgggg tttcatagtg ttgcctaggc tgatctcaaa ctcctgacct caggtgatct 240
gcccgcctct gcctcccaaa gtactgggat tacaggcgtg agccactgcg cccggcctac 300

cagaactaat ttttaatcaa atttcataaa taaatctagc caatcttagc tggttcatta 360
aggaccagca aaatcatctg ttgggacttg ttagtggagc tctcctagat agt 413

<210> 47
<211> 438
<212> RNA
<213> Homo sapiens

<400> 47

ttcttttctc cttttttttt tttcttttt tgagtcagag tctgtcgccc agcctggagg 60
gcagtggtgg gatcttggct cactgcaatc tctgccttcc aggctcaagc aattctcctg 120
cctcagcctc ctgagtagct gggactacag gcctgcacta ccacacctgg ctaacttttg 180
tatttttagg agacagggtt tcaccatgtt ggccaggctg gtctcgaact cctggcttca 240
agtgattcgc ctgcctccca aagtgatggg attacaggcg tgagccactg tgcccggcca 300
gggttttttt ttcctgaagg gctgatcatg gctttgttcc actcactgtg cccttcttcc 360
tctgcttgga actggacaga agttccaata agctactgtc ttctattaag taaggaccag 420
acatgaaaaa ctttatgg 438

<210> 48
<211> 651
<212> RNA
<213> Homo sapiens

<400> 48

```
tccatttgaa agacactcat ttatttgtta ataacacaag ccaaacaaaa acatatctgg   60
ggatgaatct gcgaaaccta ctagggttaa aattttactt ctcttaattg tttggcttcc  120
aaaacatatt tggcttccaa aacagattca aattcaaaaa atatttacgg ccagctgtgg  180
tggctcatgc ctgtaatccc agcactttgg gaggccaagg tgggcggatc acgaggtcag  240
gagatggaga ccattctagc caacatggtg aaaccccgtc tctactaaaa atgcaaaaat  300
tatctgggta tggtggtacg tgcctggagt cccagctact tcggaggctg aggctggaga  360
atcactttca cctggaaggg cgaggttgca gtgagctgag atttgccact gcactccaac  420
ttggtgacag agtgagactc tgtctcanaa aaatggaata attaaataaa aaataattgt  480
tcagagtgcc actagggaga ggtatattca ttagaatgga caatgccttt taatggtatg  540
gttgccggtg gctggctcac gcctgatccc acaactttgg agggcgaggn gggcgaacaa  600
gaggtcaggt cgaaccagcc tgacccaaat gtgaaacctg cttactaaaa a            651
```

<210> 49
<211> 428
<212> RNA
<213> Homo sapiens

<400> 49

```
ccactgttgc tgagacattt ttattggcat aggttatatg tttgtgtgtg tgtgtgtgtg   60
tgtccctaaa caatatttag caagttgact gtttttaaac tttatatcaa tggtgtatat  120
taaatatgat cgtctacagt ttgcttttac agctcaatag tttaaaaaca aaacaaaaca  180

aaaagctgca gtaatccccc tgccgttatt catgaggatt acatttcacg acccccagtg  240
gatgtctaaa actagattag tactaaatcc tgtatacatt ttcctataca tatgtaccta  300
tgatggttta atttataatg tttgcacggg agattaaaaa caataactaa taataaaata  360
gaataactag agcaggccag gcaaggtgac tcacgcctgt aatcccagct ctttgggagg  420
ctgaggtg                                                           428
```

<210> 50
<211> 436
<212> RNA
<213> Homo sapiens

<400> 50

tttttttta aaggatgaga aaaaattggt acacacgaac aatgctcaca aaacggctgg 60
gagaaaggca aaatctaagc atattataag ggtgggattc agaatacagg agggcagagg 120
gggctgccac tgtgatgggt gggaatgaag aaagggaact gctactgctc tgaaggagaa 180
gggaaagccc gctgtcgggc agtgtgtgtg cagagacagg aaactggctg aagcatccac 240
tgtgaagaat ggaagactgg gactacattt cccaaattct acttgtgtat tataaactgc 300
tacccatgaa gatggttcta tttgaaagta atatttaggc cgggcgtaat ctcagcactt 360
tgggattaaa cgcgtgagcc accacacccg gcccaagtct taaaaagaaa aaacaaaacg 420
acagggatat attatt 436

<210> 51
<211> 475
<212> RNA
<213> Homo sapiens

<400> 51

tatgagatgg gggtcttact atgttgccca ggatggattt gaaatcctgg gcttaaggga 60
tcctcctgct caggctctgg actagctggg attacaggtg tgtgccacca caccttgctt 120
tcccactaat tctgttcctg ctagtttctt cccttacaag taaggtgggt catatttacc 180
tgtgagaaac tcagaaatac tcacttttcc aggacagctg gggtgaagag aatatgtagt 240
ggccactgta ctttgtagga aagacctagg gctgcccagc cagatgcagg ggcttcccgg 300
ggagaagttt cccgagaagg cccttctctt gcctgagtag catctttgtg ctcctttccg 360
tgatactcga ttgtcaagtg caacagaggg agaaggttgt catcatctag caataccttg 420
tgtgctgact gttgaaaggc cacattcaca tcatgctcag ttactgcagt gggtg 475

<210> 52
<211> 439
<212> RNA
<213> Homo sapiens

<400> 52

tggagagttg gatctcgtat cctgcctaga ctggtcttga acacctgggc taagcgatcc 60
tccacttcag cctccccaag ttcttggact acaggcgtca gccaccatgc ccagctccta 120
gtgtcctttt tagggtctta agcaccacaa agggaatctt gattaactag tgacaatcac 180
aacaagtcca cagccttgct cctagcctgc ctccatacag acagcaatta aataccacct 240
gtgtaaactg caggagagta gttcaaattt ggctgagtaa ctttttcctg gcatgaaaga 300
accggctcta atgactagtt cattccagat ttcactggac attagatcta gtgctttgtt 360
ttgtttgcaa catttcctat ttgcccacac ataaatggac tttggggtct aaggccccac 420
tgctcttcaa atggacatg 439

<210> 53
<211> 519
<212> RNA
<213> Homo sapiens

<400> 53

tctttcctca aatctttatt gtcagtctat cacctatata tctatagtta gggaagcttt 60
catatagagc aagggtgcac tccagatata tgattcatct actaattaat aatgaataac 120
ttgcaatgtg ccaggtgctc ttttaaaagc atttagatgt tttaacttat ttaaatctgt 180
aaacatttct tttaaaagta tgttatcagt aatgaaaatg gcctacatcc tactctataa 240
aggccagtag tttacttctt ggaatatcat cttggtcagt catgatctga ggagaatata 300
cacctgtttc aacagtgatt atcattgtat aaaatttttg aaacaccttt tggaattact 360
aaagggttgt gacacatctc tatgtacatt ctcagtaatg aaaattttta acttcaggga 420
gaattaaatt ttggaaagaa taaaaaatat ctaggccagg catggtggct ctaaaggtaa 480
ttntaaaagt cctcaaaatg ttttaattgt agcattgcg 519

<210> 54
<211> 319
<212> RNA
<213> Homo sapiens

<400> 54

tttttaatg tgacccattt atttatttat ttatttatga tggagtctca aaaaaaaaa 60
aaagaaagaa aaacaattct tgtaatccca gcactttggg aggcatatca cttgaggtca 120
ggagttggag acgagcctga ccaacatgaa accctatctc taaaaaagaa aaagacctct 180
ttgcaaacaa ccttggtgca aaagtttact actaccattt cattctcaac attaaggacc 240
tagtgtgctt ggtgggtgga caagaaaaca aatctaggaa agggaaagct tttctacaca 300
aagagtagta gcacctcaa 319

<210> 55
<211> 352
<212> RNA
<213> Homo sapiens

<400> 55

ttttttttt tttttttaat ttttttttat ttatttattt tgagacagag tctcattctg 60
tcccccaggc tggagtgcag tggtacgatc ttggctcact gcagcctccg cctcctggat 120
tcaagcgatt ctcctgcctc agcctgccga gtggctggga ttacaggtgt gcaccaccat 180

gcccggctaa tcttttgtat ttttagtaga tatggggttt caccatgttg gccaggttgg 240
tctcaagctc ctgacctcaa ggatccgccc accttggctt cccaaagtgg ctgggttaca 300
ggcgtgagcc accatgccca gccagaatgc aaccatatgt ttaaagataa ta 352

<210> 56
<211> 232
<212> RNA
<213> Homo sapiens

<400> 56

ttgagacgaa gtgtcgctct tgttgcccag cctggagtgc aatagcgcaa tctccaccca 60
ctgcatcctc cacctcctgg gctcaagtga ttctcccgcc tgagcctccc gagtagctag 120
gactacaggc gcccaccacc gggcccagct aatttttttgt atttttagta gagatggggt 180
ttcaccatgt tggccaggct ggtctggaac ttctgacctc aggtgatcca cc 232

<210> 57
<211> 446
<212> RNA
<213> Homo sapiens

<400> 57

tgagacggag tcttagttgt ccaggctgga gtgcagtggt acgatctcag ctcactgcaa 60
ccactgactc ccaggttcaa gcaattcttc tgtgtcagcc tcctgaggag ttggggctgc 120
aggcaagtgc caccacgcct ggctaacttt tgtattttta gtagagacgg ggtttcacca 180
tatcgctcag gctggtctca aacttctgac ctcatgacct gcccgcctct acctcccaaa 240
gtgttgggat tacaggcgtg agctaccacg cctggccaga actatcattt gattcagaaa 300
tctcatcatt gggtatctac ccaaagaaaa atagtttatt atatgaaaat gatacgtata 360
cttgcacatt tattgcagca tgctcacaac agcaaactgt atatatcaga aaagcttaat 420
attcaaaata tatagaaaat tcaaag 446

<210> 58
<211> 510
<212> RNA
<213> Homo sapiens

<400> 58

aattagctgg gcatggtggt gcacacctat agtcctaact acttaggagg ctgaggtggg 60
acgactgctt gagccgagga gtttgaaggc aatagagaga gactctgttt caaagaaata 120
aaatgtaaag acaaatttct ccttcctctt caaatatgag aatcatcata gccctcccta 180
actcctatat tttttagatt aatcaaactc agatttctca aacattctag aacacaactt 240
gatcttgcct cccaagatta acccttccag aactttttaa ctttgttaaa gtgcctgtct 300
ttccatcttt ttaaaataga gcttatcaaa gaatttctgt gaaagtttcc ctttgcttcc 360
tcaccggaat gatctgtgat cacattagga ttccatcttt gaaaactact atctaagcca 420
tctttccatt ttaagatttc tgaatacaaa aaaaaaatcc ctttttctta atttctctaa 480

aattcactga cttaatgggt cttattcttt 510

<210> 59
<211> 245
<212> RNA
<213> Homo sapiens

<400> 59

ttctttaaga gatggggcct ctctatgttg ctcaggctgg tcatgaattc cagccctcaa 60
atgatcctcc caccttggct tctccaagtg ctgggattac aggtgtgact caccatgctc 120
ggccagatca tcacttttct gtcacttaaa tctcttgata aaggtgcttg atctcaaatt 180
ttctcttctt taccttagct cctataccac taaagtcttc tttgaaaaaa aaaaaaatca 240
ctttt 245

<210> 60
<211> 479
<212> RNA
<213> Homo sapiens

<400> 60

tttttttgag atggggtctc gctctgtcgc ccaggctgga gtgcgtgcag tggcacaatc 60
tcggctcacg gcaaactctg cctcccagat accacacaag gacttctccg agccagcttt 120
ctgagggtta actgagggct gaggggttca agaaggagga cacgggcaca gggactcacg 180
ggcagtgaga ggcagtgggg ccaatggcgt gaggagcacc agagagcagg agggaacggg 240
cccggggcgt gaatccggcc ccatgagtgc tcttcggccg cccaaaaccg gtcccatggg 300
taacagcgtg gccttcggca agtgactaaa gggcttcctg cctcagcttc cccacctgta 360
aacagaggat aacaatggca tgtactggat ctggcataaa gtaaatgttc aatagatagc 420
tagaaaagaa tgttttaaaa cctcagagat acattaggcg aaaataaaag ctgggctac 479

<210> 61
<211> 480
<212> RNA
<213> Homo sapiens

<400> 61

tgagacagag tctcaccctg tcacccaggc tggatggagt gcagtggtgt gatctcggct 60
cactgcaagc tccgcctcct gggttcacac ttctcctgcc tcagcctcct gagtagctgg 120
gactacaggc gcccgccacc acgcccagct aatttttttt gtagttttag tagagtcggg 180
gtttcaccgt gttaaccagg atggtctcga tctcctgccc ttgtgatccg cccgcctcgg 240
cctcccaaag tgctgggatt acaggcgtga gctaccacgc ccggccgtct tgtgtcttct 300
ttactgtgac tgggtcgttt ttaagaaagg ttatcagctt tgtgtttggt ttcccacagt 360
tgataaaaat ctatcaaaaa cattataatt tgcaaggaaa aaggtttttc aatggctgca 420
ggaaccagaa agaaatagca tttcttatct gtataaacac aaacatttaa agctagtcac 480

<210> 62
<211> 179
<212> RNA
<213> Homo sapiens

<400> 62

ttttttttta atttcagaca gaatctcact cggtcgccca ggctggagtg caatggtgcg 60
atctcggctc actgcaacct ctgcctcctg gattcaggca attctcctgg ctcagcctcc 120
tgagtagctg ggattacagg cacccaccac catgcccagc tattntctgt atttttagt 179

<210> 63
<211> 307
<212> RNA
<213> Homo sapiens

<400> 63

gtatttttag tagagacggg gtttcaccat gttggttagg ctggtctcga acccctcacc 60
ttgtgatcca cccacctcgg cctcccaaag tgctgggatt acaggcatga gccaccgcac 120
ccggccctaa acatttgtta gacaatactt ccgaatgttt tgtctatgta atttagttca 180
caaatcattc agctcataaa tcaacttgtc tagactcatg ccctgggttc acagaattag 240
aaataatact attttacatt agggactact aagaacaacc aggatgatga taatagtcat 300
cactaaa 307

<210> 64
<211> 275
<212> RNA
<213> Homo sapiens

<400> 64

tgtattttca gtagagacaa ggtttcagtc ttgaattcct aacctccggt gatccacctg 60
cctcagcctc ccaaagttct gggattacag gcatgagaaa ccatgcccag ccgatttctc 120
tcattttttt tttttttttt ttttaagaga caaggtgctc gctgtgttgc ccaggnctgg 180
tctcaaactc ctgggctcaa gcaatcctcc tgccttggcc tcctgagtca aaaagtgcat 240
ctcanatagt tttaaaatgg atgtgcaata tttag 275

<210> 65
<211> 306
<212> RNA
<213> Homo sapiens

<400> 65

tttttttttt ttgagacagt ctcactttgt cgccaggctg gagtgtggag tgcagtggca 60
caatcttggc tcactgcaac ctctgcctcc tgggttcaag cgattctcct gcttcagcct 120
cctaagtagc tgggattaca ggcacacccc accacacccg gctaattttt atatttttag 180
tagaaagggg gtttacccat gttggccagc tgggtcttga actcctacct ntntggatcc 240
acccgcctcg gcttccaaaa gggggaagtc actgcaccca gccttgctgg atttttctaa 300
aacctt 306

<210> 66
<211> 444
<212> RNA
<213> Homo sapiens

<400> 66

tttttttttt tttttttttt ttttgagatg gaattttgtt cttgttgtcc aggctggagt 60
gcaatggggt gatctcggct caccgaaacc tccagcctgg gtgacagagt gacaccctat 120
ctcaaaaaaa aaaaattctt cattgctcat atacgtcaga ttattacaat tttggtatgc 180
ttaaaaatca cagagagcca aataaggtgg ccaagagaga agaaaatgat aagttgtcca 240
cacaagcgct ctgatccaag ttaaaaacaa gcaaagtaag gtatgggagt acaaaatcac 300
aaaaatattt tagagcattt taaaaagggg gctattataa ttatcttctt ttaattatta 360
attttaatat cttaacatgc caccaaatta aattcttcct gaatagagaa agataagctt 420
ttaaaaattc tgcatcatta ctgg 444

<210> 67
<211> 311
<212> RNA
<213> Homo sapiens

<400> 67

tttttttttt tttttttttt tttttttttt atttagagat ggggttttgc tctgttgccc 60
aggntggagt gcagtggcat gatcatagct cacagcagcc tctaactcat gggctcaagc 120
aactcttaca cttcagcctc caaagtagct gggactacag gcatgagaaa ccacacttgg 180
ctaacacaca cacacacaca cacacacaca cacacataat tgctatcatc tctatcaaat 240
atacacatat atttgatata tatgtatatt tgtgtgtgtg tgtgtgtgtg tatatatata 300
tatatatatg t 311

<210> 68
<211> 441
<212> RNA
<213> Homo sapiens

<400> 68

tttttttttt tttgagacag ggtctcactc tgtcacccag gctagagtac agtggcacaa 60
tctcggctta ctgcaacctc tgcctcccag gttcaagcga ttctcctgcc tcagcctccc 120
gagtaactag gaccacaggc acacaccacc atgcccggct aatttttgca tttttagtag 180
agacagggct tcaccatgtt ggccagggct ggtctcaatt tcttgacctc atgatccacc 240
agcatcggcc tcatgatgtg ctggggatta cagggcatga gncaacgcac tcgggcctag 300
tattcaattt tacagggcag ggccacctct tacctatttt cacaggaaaa ccagtnttta 360
cacagganca gtnaggaacc actggaattc agtnggtctt ttcngggggg ttntaggttc 420
acantggatt taaantacag g 441

<210> 69
<211> 435
<212> RNA
<213> Homo sapiens

<400> 69

tgttgttgaa tattccttaa tggagtcgat gaatttgcag agacccctcc aagattcttt 60
tgtttgattc ctttcatgac catcacccta gaccttcaag tttgctgact agatttgggg 120
gttgtgtgtt tagaaaggat aacaagcttg tcatgggcta gaacctgtgg tcttcataaa 180
tagcttagta ggatatatgg ctttttctat gaaaggtgga gaacatgcat taaaaatggg 240
caaattctgg cctggggcat ggtggcttat gcctgttaat cccagcactt ggggaggctg 300
aagcgggcag gtcgtctgag ggtcagggag ttttgagacc agcctggccc aaaataatgn 360
aatcctgtct cntgctaaaa ctaccaaaan tagcntgggc ntggtagcac acccntagtc 420
ccngctactt gggga 435

<210> 70
<211> 348
<212> RNA
<213> Homo sapiens

<400> 70

```
tttttttttt tttttttttt tttttttttt aaagagatgg agtcttgcca tcttacgcag 60
gatggtctca aactcctggg ctcaagcgag tctcctgcct tggtgtttca aagtgctggg 120
attacaggtg tgagccactg tgcccagcca acttctcatt ttaaaagaat ttcagattta 180
aaaaaattgc aaaaatactg cgagagaatc cncatatact tttcacccag atccaccaaa 240
tgttaacatc ttaaataacc atattatgnt gatcaaaacc agaaatacta ttaactactc 300
tacagacttg actcaaaatt caccaactgt ctgcctaatt ttagtcca 348
```

<210> 71
<211> 304
<212> RNA
<213> Homo sapiens

<400> 71

```
tgtagagaca ggcgcttact atgttgccca ggctcggttt taaactccaa gcctcaagtg 60
atcctcctgc cttggattcc aaagtgctgg gattatagtt gtgagccact gcgcccaaca 120
ttcccatgac ttttttgtga aggaggcatt caccaagctt ttcctaatct ttaccataag 180
ccaggctctg cggtaaacac cccacaataa atgtttatca gaggacttag cagggaagta 240
cattaaatgt taacgcctta atctgatact gaaaataaaa gataatttca acttggtttt 300
tnaa 304
```

<210> 72
<211> 192
<212> RNA
<213> Homo sapiens

<400> 72

```
gggcgtctcc ctatgttacc caggctggtc ttgaagtcct gggctcaagc aatgctcctg 60
cctcagcctc ccaaagtact gggattatgg gcatgagcac tgccctgcac ccagtcagaa 120
atgcttctct tgaataagca gttattagag gaattaaaca ttcaagaacc ctaacatgcc 180
cccaaacatc gt 192
```

<210> 73
<211> 487
<212> RNA
<213> Homo sapiens

<400> 73

ttttttttt tntctatttt tagcagagac ggggtttcac catgttggtc aggctggtct 60
agagctcctg acctcaggcg atccacccgc ctcagcctcc caaaatgctg gtataacagg 120
catgagccac agcgtctggc cagaatcata tcttaatagc aatcccataa tgtagtttta 180
ccagaaatac catagtcaat tttacagggt gggttcagtt tttcttaaat tacttacccc 240
taagattaaa gaatatttta aaatattgtt ataagngaca taactaaact attaggtttn 300
tgcaaaagta attgtagttt ttgccattaa aaggcaatta taaaggaaaa cggggatatt 360
aataggngtt acttctaggc ttgnaagggn taacattctt ttttggctac ttaaaagtaa 420
tgggcaaaaa ctggcaattg tttttggcac caacctatta gggcaagaga acccnatggg 480
ctttttg 487

<210> 74
<211> 446
<212> RNA
<213> Homo sapiens

<400> 74

ttttttttt aatagagatg ggggatctca tcgtcaccca ggttggaatg cagtgatacc 60
atcacagctc gctgcagcct ccacctcctg ggatcaaccc ctacctcatt ctcctgactg 120
ggactacagg cactcaccac cacactgggc taattaaaaa aaaaaattct tttttgtagg 180
gaagtggtct tgctatgtca cccaggttga tctagaactc ctgacctcaa gtcacccgtc 240
cgcattatcc tcccaaagtg ctgaggatta cagacgtgag gccactgcac ttgggcctat 300
ttaggggctt ctaattcact ttccttttcc ttcttgtcta aattcttgtg tttttagaat 360
ctggcatttt attttaaggt natcttcaan tcctttttggg aagtagtgag gggagtaaat 420
gcttaacctg tgtaggaaac cntttt 446

<210> 75
<211> 6213
<212> RNA
<213> Homo sapiens

<400> 75

cagtctttga ttggttgctg agaggcgggg ctactcgact gctctggagg tagcggccgc 60
ggtgaggaga gccatgggac gggcagtcaa ggttttacag ctctttaaaa cactgcacag 120
gaccagacaa caagtttttta aaaatgatgc cagagcatta gaagcagcca gaataaagat 180
aaatgaagaa ttcaaaaata ataaaagtga aacttcttct aagaaaatag aagagctaat 240
gaaaataggt tctgatgttg aattattact cagaacatct gttatacaag gtattcacac 300
agaccacaat acactgaaac tggtccctag gaaagacctt cttgtagaaa atgtgccata 360

ttgtgatgca ccaactcaga agcaatgagt tttctagaat acaacaagtc tttgtacttt 420
ttaactttaa aatctacaac tctggcaaaa gtcctggaaa tgcagacatt ttccctgaac 480
tggcatattg aaaatgaatg aattacagaa tagcttcata tttaaatttc atgttaaaag 540
gtcattactg agaactaaag aacataatta agtatttcta aaggaaatta gataagaaaa 600
catttcattt tcattgaaaa tcaaatttca taaagcaaag taaatgctta gggagatata 660
ttcaatcttt gaccttgatg agtatttgat cttaccatag ctatttgaga atgtggtgct 720
tttacaaatt ggtgagtttt cctgccatgt gaaatgcaat tattacattt aaattgttag 780
attaaaatga tatttagtcc tgaaaaatat taaattggtc aaaaaaatca cagtgtatgc 840
cagctctcta cagaaagtgg cctttgtttt ctaaagcact gggattattt ctgtagctaa 900
tatataattg tacagtttct ttttagagat agagagtatc tctgtgttct tatgaagaca 960
tttttatca gttttctgaa aatagatgaa taaaatatta tagtcaccta gggtcactat 1020
ggaataaaga aatcctagtt taaagaggaa atagtggccc ttgatcaaac tatttaatat 1080
ggccttagta gaattagctg tatttagaca aagttagact ttagtgtgaa atgtaatcgg 1140
tggctacatt ctcatcgttt taattaatga aacttaaatg gcttctcttc ttccacatgt 1200
cctgtccttg acaagatggg cagtatcaca aaaggtcctg gcattctacc atctaacact 1260
aggaactgta aaatactgtt taatattctt cttgtttctc ttttatctgt gtatctttgc 1320
cattctattt tctcagtgaa tagtatgttt tctcccattc actgataaat tctctcattt 1380
gatgatgata cagggttttt aatttttgca agattctcaa tgcaagcatt gttatgtatc 1440
tagaaattat acctagagaa aaatgaaagt cgtttcaaat ttgaaatttg cccttttaag 1500
agaatgctga atgtcatcgc agtatataat cactatataa atgtgctgac ttacagttat 1560
tttagtgtct atatgacata ttttgaggaa agttggctga cgttatttaa atttaatata 1620
tattctatat tttagtgtta ttgaatattt tatcactgag ctttttttctt taacctgaat 1680
tccctgttcc atttttcatt catattaatt taaataactc cagatttctt tcttatagtc 1740
attattagta gcagatgaga ttaataattc acatgtttat taaagatagt ggcttagaaa 1800
ttttaagata tattgatata ggcccgggcg ctgtggctca cacctgtaat cccgcacttt 1860
gggaggctga ggcgggcaga tcacaaggtc aggagttcga gaccagcctg gccaatgtgg 1920
tgaaacccca tctctactaa aaacacaaaa attagccagg tatggtggcg ggcgcctgta 1980
gtcccagcta ctcgggaggc tgaggcagga aaatcacttg aacccgggag gtggaggttg 2040
cagtgaactg agattgtgcc actgcactcc agcctggggg acagagtgag actctgtctc 2100
aaaaaaaaaa aagaaaaaaa aaggaaaaag gaaaaaaaaa agatatattg atacagatag 2160
gtagatatga tattgtactt tcatgccata agactacaca ataaagttcc tgaaagttcc 2220
tggctgggcg cagtggctca cgcctgtaat cccagcactt tgggaggccg aggcaggcag 2280
atcacctgag gtcaggagtt ctagaccagc ctgaccaaca tggggaaacc ctgtctctac 2340
taaaaaaaat acagaattag ccaggtgtgg tggcacatgt ctgtaatccc agctactcgg 2400

gagactgagg caggagaatt gcttgaaccc aggagacgga ggttgcagtg agccgagatc 2460
gcaccattgc actccagcct aggcaacaag agtgaaactc cgtctcaaaa ataaataaat 2520
aaataaagtt cctgtgaagt atataaacat gtcaacaaca ggcttgactg tcacaaaatt 2580
ctgaaagatg tcgcactcta ttcttatata gcatatgcta atttatttat ttattttttg 2640
agattgagtt ctgctgtgtc acccaggttg gagtgcagtg gcatggtcat ggtccactaa 2700
agccttgacc cctggggctc agcagttatg ccaactaagc ctcccaaata gctgagacta 2760
gaggtatgcg ccaccacacc tagctatttt ttttattttt agtaaggaca aggtctcatt 2820
atgttggcca ggctggtctc aaattcctga gctcagttga tcctcccacc tcagcctccc 2880
aaagtgctgg gattacaggt gtaagccact gcaccctgcc tattcttata atcatatatt 2940
tatatttcaa atggatttta actggttatt taatagttta attagataaa gtaattcatg 3000
gctgggtgtg gtggctcacg cctgtaatcc cagcactttg gcaggctgag gcaggtggat 3060
tacctgaggt cggaagttcg agaccagccc aaccaacgtg gagaaacccc atctctatta 3120
aaaatgcaaa attagcagga catggtgata cacacctgta atcccagcta gtcaggaggc 3180
tgaggcagga gaattacttg agccagggaa gcagaggttg tggtgagcta agattgtgcc 3240
actgcactcc agcctgagag aacaagactc cgtctcaaaa aaagaaaaaa agaaaacttt 3300
tttacacatg ggtatctcac catgttgccc aggctggagt gcagtagcta ttcataggca 3360
cagtcatagc acactgcagc ctagaatttc tgacctcaag caatcatcct gcctcagcct 3420
cctaagtagc taggactaca ggtgcatacc accataacca gctttaatta aatgtttttt 3480
atttggttat ttttttaag ttttctgtat tcacacaagg ggttgcccaa atataatttt 3540
gctttgacta ttgagatcta gtgaaagtgg ggtatatgaa ttctaattgc aaatatccag 3600
gctcagaggc ccagcaggac tttctaacac aatcttttag cggaagttag aaatggtata 3660
tagcaggaga gtcagatttg agaagcatat gtagattcga agctggggga atatggcagg 3720
tagtttgtac aacatctaat tcagaacatt aaaattaaga ttttagtcaa actgtgttta 3780
agttagttct tattttcctg tagatgcatc tcacagcatc agtacaatac caaaaaagca 3840
cacaagaata agaatatgtg gaatttctat acctattgac aaagcacata atttaaccat 3900
aaacacaaag ccataggtca acaaagaaat gaagattcca gttctgaagg tgagttttct 3960
gaagccaaag tggatacatg caaaattaat atagttttac tgtatatcag ttgtcaccaa 4020
tcagaaatgg aaaacagatc ctatttataa ttgcaaacaa aactgtaaaa tagacttttt 4080
aaagtctggg aatagacttc taaaataagc tataacactt aaaaaggaga gatatactat 4140
gttcctagat aggacaattg aaaattctgg agatgacagt ttttcaaaaa tctattgagg 4200
ccaggtgcag tggcccatgc ctgtagttcc agtactttgg gaggcctagg tgggtggatc 4260
acctgaggtt gggagtttga gaccagcctg accaacatgg agaaaccccg tctctactaa 4320
aaatacaaaa ttagccaggc gtggtggtgc atgcctgtaa tcccagctac tcgggaggct 4380
gaggcgtgag aatcgcttga acccggtagg cagatgttgc agtgagccga gatcgcacca 4440

ttgcactcca gcctaggcaa caagagcgaa actccatctc aaaaatagaa aaaacattta 4500
tcgaaatccc aacaagttga caaatatatc cacataaaaa tataaaactt ctgtattctg 4560
tgaaagctac tataaataaa gtttagagaa agttatttgc cacctatgtc atgattgaaa 4620
tagttaattg atcctgtgaa tcagttagca aaacataact caatggaaag ataggcaaat 4680
gatacaaata agaaattcac aaaagaagaa atactaagtc tctagtgatg agagaaatgt 4740
aaattaaaat gaaacatgtt tgttcatcaa gttgtcacaa gttagacaat catatccaat 4800
atttttaaag gttgtaagac tataaggaaa tagccactgt catatcattt ttaaaggaat 4860
ataaattata gggccttttg tttctttggg tttttttttt ttagagacaa gatctctccg 4920
tgttgtctag gctggactca aacttctgga ctcaagcaat cctcgcaaca tcattaatag 4980
ctgagagtag agacttgagc caccacacct gactataggg ccttttttgaa aggaaaattg 5040
acatcatcaa aattttaaat atattcagtc tatttctcaa aaactcaaag aatactaata 5100
aatgtgtact caggtatatg tacagaaatt gctgtaacat tataatttta aacaatttaa 5160
aacagactga gtttccaaag ttagggtaca atgaaagaaa aggtggctta tttatactct 5220
ggaatatttt ccaagagttg aaaaggatga ggatacacac acacacacac acacacacac 5280
acacacacac acacacacac agtttgggta tccctaatcc agaaattcaa atgctccaaa 5340
gtccaaaact ttctgaccca ccaacatgac tgatgctcaa aggaaatggc cactggaaga 5400
tttcagattt tcagatttgg agtgctcaac cagtaagtat ataatgcaaa taatccaaaa 5460
tacaaaaaaa aaaaaaaaga aatctgaaac acttctgatc ccaagcattt cagaaaacgg 5520
atgttcattt gtgtgtgtgt gtgtgtgtaa gcaggtgttg ctagaaattc acttatatac 5580
aagaaaactt tttgtgtaca tatttgcata tatatgtaca aatgggtaga aacgatacat 5640
gattaatctt aatcgggaag gaaaagagat ttagggaagg aagcagtaag tgagaacttt 5700
tattctattt actcctgcac gtttaaatat tgtttacagt gagtatatca acatgtaagt 5760
gttaaaagac aataagctac tagtgatttt taatataaaa ttaactataa aatattttaa 5820
atattagcaa ataatatagc acactcatga acctaattcc cacatttgat agttgttaca 5880
ttttgccatg tttgtttaaa ggtctaagtc ataaaatctt ataaagctaa accccaccct 5940
tctctttctc ctctctctcc aggataatta ctgttttata gtttgtggat atcattccct 6000
tacttgtgtt tatactttta ccaagtgtgt atgtattcaa aaaacagttg ttttgtgatt 6060
ttaaaatgta aatgaatggc gttatgctcc atgtattctg caacttttca tcatacatta 6120
ggttttggcg atttagccat aatttggcat gaattcaggt cttttaagtt ttattccatt 6180
gtaagaataa acaagtttgt tcattcatgt ctc 6213

<210> 76
<211> 354
<212> RNA
<213> Homo sapiens

<400> 76

gtaaaaggca aaaatttgag acttataagc tatatggtag cttatttttg ggtggggaag 60
aaatgagaaa agaatataac atctcttact ggcatgacac attttgataa aaaatcttat 120
tgtcctttcc tactaggaat gatccactgt aagggcaaaa ataatataca aggcaaagtt 180
tttntttggg aggacagagt ctcactctgt cacccgggct gggagtgcag tgggtacgat 240
tcttgggctc actggcaacc tctccctccc ggggttcaag gtgattcttc gtgcctcagc 300
ctcttgagta gctgggggtt tacagggcgc gtgccactgc gtnccggcta nttt 354

<210> 77
<211> 399
<212> RNA
<213> Homo sapiens

<400> 77

gcgtgtgtgt aaccttgaac tcctaggctc aagtgatcct cccaccttag cctctcaagt 60
agctgggtct acaggtgtgt accaccatgt ctggctaatt tattaatttt ttttgtagag 120
acagggtctc actatgttgc ccaggctggt cttgaattcc tgggcttcaa gtgancctaa 180
tgcctcagcc tcctaaagct ctgggactac aggcatgagc tatcatgccc agccagtact 240
aaataatttt taacaaaaga ntaaatcatt attttttata taaggtttct gtaagggggg 300
ctacaggatt tattatactt ttctgacatc caaagntttc aaatttggtt atatttttcc 360
ngatatatgg agggcccaaa atactttttt aataaccttt 399

<210> 78
<211> 510
<212> RNA
<213> Homo sapiens

<400> 78

ttttgtagat aatggggtct taccatattg cccatgctgg tgtcaaactc ctgggctcaa 60
gcaatcctcc cacctcagcg tcccgagtag ctgggaccac aggcacccac caccatgcca 120
cactaaaatt tttttttggg gggggagggt agagaagggg tcttaccatg ttgcccaggc 180
tggtgtcaaa ctcctgggct caagcgatcc tcccacctca gcctcccgac atgtaaacgg 240
tggctacatt tccgcacaat ccccgcggtn tccctcattc tgttttacaa ctactcccac 300
ataaagtaac gtaggaaaga cggagccccg ttattccctt aggaagggta ggactgggag 360
ntttgcaggg aagctntagg ggattaaaca ttcagagggc caacttgagg attaaaacgg 420
aaacacccgg ggtgattttt aaggttaatt caagaggccc cttttcacgt gggggtgatt 480
ttttaaactt antcaggggn cttttttca 510

<210> 79
<211> 392
<212> RNA
<213> Homo sapiens

<400> 79

ttcagagata gggtctagct ctgtcactta ggctggagtg cagtagatga tttatagctc 60
actgcaacct tgaactcctg acctcgtgat ccgcccacct tggcctccca aagtggtggg 120

attacaggcg tgcnccgttg cctggccatg ccagctaatt taaatttttt tttttgtaga 180
ggaaggagtc atgctacatt ccccaggctg gtcttaagct cctggcctca agtcggcctg 240
ggcttccaaa ttctgggatt atgggtttta cctgggccag agaagatata tttgaatcaa 300
acttaggggg acaaggattt ctgtacatca gtgttgtcct tgaggaaact gaaatgcagc 360
tttggggaaa gatnttttca gagcagagag aa 392

<210> 80
<211> 498
<212> RNA
<213> Homo sapiens

<400> 80

tttttaagta gagatggggt tttgccatgt tgncagggtg gtctcaaact catagcctca 60
tgtaatccac ctgcctcgac ttccaaaagt gctgggatta caggtgtgag ccactgtgac 120
cagcctgact tcaaatcctg tgttgaatag aagtagtgag atcgggcatc cttctcttat 180
tcctgatctt ggaggcaaag atttcagtct ttcacctaaa atgactgaaa gactttcagc 240
catgggcttt gcatgactgg cctttatttt gttgctgtac attccttctt ttcctggntt 300
tgggagtgtt ttaccagggg aaagggtntt caaggctggg ggcaccgtgg gcctcaagcc 360
ttgcaaattg cccagcactt ttggggaggg ccaagggtgg ggcgctccgt gcccaatttc 420
ttgggncctc gagggccaaa atttccccaa taagtgaagg ccgtatttta aaattccgna 480
aatcaangtc aaaaggct 498

<210> 81
<211> 325
<212> RNA
<213> Homo sapiens

<400> 81

cccnnctggt ttcaaactcc tgacctcaga tgatccaccc acctcagcct cccaaagtgc 60
tgggattaca ggcgtgaggc accacaccca gcccagatga gcttcttttc ttgtttattg 120
ccaaataaga gtcctttgaa ttatacatca tgttgttttg agccattcac atgctgatga 180
acatttgagt tgtttttcac tttttgacta ttattgatgc tgctgtgaac gttcacctgc 240
gtgtgcttgt gtggggcatt ctgaggacca gancacgngt aagcaaaagt gangctacat 300
ttngttggga natgatgctg gtatc 325

<210> 82

<211> 431
<212> RNA
<213> Homo sapiens

<400> 82

```
cggagtcccn tcgttgttgc ccaggntgga gtgcaatggc ngntctttgg ctcaccacaa     60
cctccgcctc ccgggttcaa gagattctcg tctcaaactt ccgagtagct gggattacag    120
gcatgcacca ccacacccgg ctaattttgt atttttagtg gagacagggt ttctccatgt    180

tggtcaggct ggtcttgaac tcccgacctc aggtgatccg cctgcctcgg cctcccaaag    240
tgctggggat tacaggcgtg cgacccacgn cccagccacc tnttaaattt cttaatcacg    300
gattgttttc agctcaggac atacacaagg gcaagtagga attactaata aaatcacttt    360
taccctcaac cattcanggt ctctaaggng catgcanagg ggttacatgn cgggggnaag    420
ggaaggcact t                                                         431
```

<210> 83
<211> 2350
<212> RNA
<213> Homo sapiens

<400> 83

```
atatgccttt ttaaaaaaat tatcttttcc attggtgact atgaggttga gagatgattc      60
tcctacattt ctggctgctc ctcttcaagt accttccctg gctcctctgg atttttttgt     120
tttgttttgt tttgttttgt tttgtttttg agacaaagtc ttgctttgtt gcccaggctg     180
gagtgcagtg gcaggatctt ggctcaccag ctcactgcag cctccacctc ccgggttcga     240
gggattctgg tgcctcagcc tccagagtag ctgggactac aggcccggct agtttttgta     300
cttttggtag agatgggggt ttcaccaggc tggtcttgaa ctcctgcctc gggtgatctg     360
cccgcctcgg cctcccaaag tgctgggatt ctaggcatga gccaccgcgc ctggcctggc     420
tcctcttctt cttccactca gatatgcctg accctgtcaa cactttggtt gaggtcttct     480
ttcttctttc ttttttgctc cgcacattta gcttatgact tcaaccatca tttctcagag     540
catgggtctg gctcaacctc tctcctgaat ttcagaccta caagtctagc tacttggtgg     600
agacctcccc agaatgacct gctgcttccc aaaagcagac tctccaaatt acagtcagta     660
tctccccccgg aagcattccc ccaggcattt ctctttctgc cttcaattcc ccattctcct     720
acattgcctt gccagaagcc tgctggtcag cttggatttc tttttgtcct ttttttttcta     780
tattttgctg gtgcctagtc atgtagttgc tgcctctaca ctttctcttc tttaaaaaaa     840
attattaaag caccacgtgc ttgttgtaaa catttccaga aaatacagaa gtgctcaaag     900
tgaaaaaatg gaaatgcctt gtcccttcct cattccctgc cctaacctca cgccccagat     960
tcagctatgt aatagtctgt catgccaagt cttatttcca gctcctcttt tccatcccca    1020
ctgccatcat ctgaactaaa cggattgttt tccatctggt ctccttggct tttcctttca    1080
gtgcagctca acagacatta atcaagtgcc ttccacacac caaagtccta ccctagatcc    1140
tagaggttca gagacaagta agatagttaa agagatccac attccagagc tgtttaactt    1200
tgggcaagtt acttaatctc tctgaccctt acttccttat ctgtaaaatg atgctaatcc    1260
cagcaccttt ttcatgggtt tggacgagca ttaatgagat gatccatgta aaactctttg    1320
tactaactac ctggtacact gtatctgctc cataaatgtc agtgacaaca atgataataa    1380
tgacaatgtt tggaggagtt tatagcttaa tggagagact taaagcataa gaattatcta    1440
ggcgaagaat gatgagaaaa tatttttgga aaaggaaaac aaacagttct actaaaatta    1500
aaaggctgat gtagaggctt gggaaactgg gaggtaagag ctcggactgt gtcctctaag    1560
```

acagtaattc ccgaagtgtg agcaaaagtc cacctgcatc agtcttactt ggggtgattg 1620
ctcaaaatga ggatttaatg gctgcacctc cgagcaagtt ggtaatttac atatcggaat 1680
gctatccatc aaggaaaatg ggcagactac agttacatgc atcaacacag acaagcttca 1740
aacaatattg agtgtaaaaa gcaagacata gaaatatata tttagtaaga gtaaaaatac 1800
agtaaaggta aaaaagaggc aaaactaaac aatatattgc ttaagcaata aggatacaca 1860
aactaatgaa aatcaaagga tttactaata caaacttcag tatagtaatt aattggaatg 1920
ggagagaaag atgcaaagtt tctatttctt tttttgtttt gttttgagac agtgtctcat 1980
tctgttgcca aggcaggagt gcggtggcag gatctcagat cactgcaggc tcagcctcct 2040
gggttcaggt ggttcttctg cctcggcctc ccgagtggct gggattgcag gcatgcacca 2100
ccacgcccgg ctgatttttg taattttggt agagatggag tttcaccgtg ttggccaggc 2160
tggtctcgaa ctcctggtct taagtaatcc gcccacctct gccatcaaag tttctgtttc 2220
ttaagttggc tgccgagtac acaggttttc tttgtaaaat aattatttaa attgttaata 2280
tgcattactt atatgctttt catttacaat gtatttcaca agaaaaataa aacaaagcaa 2340
ataagaaaac 2350

<210> 84
<211> 184
<212> RNA
<213> Homo sapiens

<400> 84

gttgcagaga tggtgaggat gtcttgcttt gttacccagg ttggtcttga atttgtggct 60
ttaagtgatc ctcccacctt ggcctcccaa agtgctcggg ttacaggcgt acaacagtgc 120
ctggcctgta ttttattgta attccttttt ccattctcat ctcaatgcat ttccaaatta 180
gaga 184

<210> 85
<211> 410
<212> RNA
<213> Homo sapiens

<400> 85

aaaatatcag ctttattacc aaggatgact gtgctgcagg aggcggctca gtgtgaagaa 60
ctacgggttt cctgatgttg aagctcagaa ttagccacac tgaccttctc agtcatgcat 120
gatgccagga aaatcacagg cttccattct acagtgaagg gcttggagga gcaggcaata 180
atctgtagtc cccagctact tggaggcagt ggacgggaag atggcttgag cccacggagt 240
tccaagttgt agtgcactat catcatgcca ctgcatctgc actccagcct gggtgacaga 300
atgaaactct gtgtctccat tccccgtggt ttgcttggtt tggtttgatt tgggtctggt 360
cttactactg ctgcctctcc gtttgactgg caaagtgtgg actgggcact 410

<210> 86
<211> 16459
<212> RNA
<213> Homo sapiens

<400> 86

gtgcaatggt gcaatctcag ctcgctgtaa cctccgcctc ctgggttcaa gcaattctcc 60
tgcctcagcc tcctgagtag ctgggattac aggtgcctgc caccatgcct ggctaatttt 120
ttgtattttt agtagacaca gggtttcagc ttgttggcca ggctggtctc gaacccctga 180
tctcaggtga tccacctgcc tcggccaccc aaagtgctgg gattacaggc atgagccacc 240
gtgcccggat gaaaagtgct tttaaaaaag catacccggt ctctactaaa aatacaaaaa 300
aaaaaattag ccagacatgg tggcaggcgc ctgtagtccc agctactcgg gaggctgagg 360
caggataatg acgtgaaccc gggaggtgga gcttgcagtg agccgagatt gcgccactgc 420
actccagcct gggcgacaga gcgagactct gtctcaaaaa ataaataaat aaataaaata 480
aaaataaata aaaaagcata aaataattca atttttttga aggactttta gaaactgttt 540
aattttagaa actatctgat tgtgatacat gctaacacac tcatatactc cctcctcccc 600
acaacacaca cacagcctct ctttgtcgct cataaagtct tcggaagctt tctcagtgct 660
tttaggagta tgacagaagt ccttacatgg ccaacaggaa cctgcatggt cttttcacca 720
cttactgtgt cttcctcatc tttctgttgt gctcccccctt gtgctctcct ccagccctgc 780
tgtcattcct ccacatggaa gtcttttttt tttttttttt tttttgaga cagagtctcg 840
ctctgtcgcc aggctggagt gcagtggcgc aatctcggct tactgcaacc tccgcctcct 900
gggttcaagc gattctcctg cctcagcccc cccaagtagc tgggactata ggagcacacc 960
accacgtcca gctaattttt gtatttttag tagagacagg gtttcaccat gttggccaga 1020
tctgctgacc tggtcgtgat ctcctgacct tgtgatccac ccacgttggt ctcccaaagt 1080
gctgggatta caggtgtgag ccaccgcacc cggccaggaa gtcttttttg actcctgcca 1140
tgttctcctg gcacttcttc cttatacaga gatcacacat gcacattgta catttgctca 1200
gtgagtgtag ggactgctgc tctggttttc ttgtttgttt gttgcttatt tctgtatcac 1260
caggatctaa cacaatgctt ggttagctgt acccgagtat ttactgagtg catgaattcc 1320
atccattgta ttttctgtag ctacctgatc tttatttgaa cctttcaaga tatctcattc 1380
cattttggtg ttcttattat aatagaaatt agagaaaata tttgcaacca aaatgagaaa 1440
aaggtaatat taatatacaa aaagctcata taagttactg aagaaaatgt ctaaagccct 1500
aataaatagg caaagaatgt aaatagctaa gtcacaaaag aaatcttaga atgcttaaaa 1560
gatccaggtg caatggttca tgcctgtaat cccaacactt taggaggcca aggcagtagg 1620
atcacttgaa gccaggagtt acaagcttag caacaaagca agacctcatc tctacaaaaa 1680
acaaaaaaat aaaaaaacta gccaggcgta gtggcactca cctgtagtcc cagctattct 1740
agagccaagg gagggaggat tgccttgagc ccagggattt gacgctatgg tgagctatga 1800
tcgtgtcact gcactcagcc tgggcaataa agagacacac tgactcttaa aaaaaatggc 1860
caaaagagat ctgagaataa ttatctttac taggcatcaa ataagtgcaa atcaaagcaa 1920

actgccacct attaattgag caaaacattt aattgataat ctatttttca gaatgtattg 1980
gtctagttag aatatcaatt cttacctttc tgacagatga ctagtccttt gtaaataccc 2040
agtcacctct tttcagttaa agttgctgtc tccaaggagt ttgcaatcta attggggagg 2100
taaaatctca actcaagaaa tgagaagtca gcatgaaaac ccattgatgt cgtattgctt 2160
ttgctgctct gatgtggtgg ctcacacctg taatcccagc actttgggag gctggggtga 2220
gaggatcact tgaacccagg agttcaagag cagcctgggt aacatggcca aaccctgtgt 2280
caaaaaaagt ttttaaaaat tagcccggcg tggtggcaca tgcctgtagt cccagctact 2340
caggaggctg aggtgagagg atggctggag gctggcaagt agaggctgta atgaactgag 2400
atggtgccac cagaaggacg gagtttccct taaccaagat aatatgtata gtggctagtc 2460
tggcacatgg cacttactgg gtattccata aagagtagtt tatttcccca aaatgtagag 2520
taagagtgaa agactttgat ccaatgtact tctgtccacc tacacaagca aatagaatgt 2580
ttcaccagaa taattagaca aaaaatttta tatgtaattg gcacattgga atccttgtaa 2640
attactcctt ctgttggcca agagatttac tcctttggtg gaacttgtgt ttttccatat 2700
gacaataata tagtaatggc aagtatatca ataataataa aacttttttt aaaaagtaaa 2760
gggaaaatct taccaaatta atgtttcatt ttaaggaaaa tatgactcta tgcccatttt 2820
tttccttcca ggatgttgcc ttatggctgt ttagcaacag gagatcgctc tggcctcatt 2880
gaagttgtga gcacctctga aacaattgct gacattcagc tgaacagtag caatgtggct 2940
gctgcagcag ccttcaacaa agatgccctt ctgaactggc ttaaagaata caactctggg 3000
ttagtttatt ctgtttaatt atcatttttc tgtacaaaca gccaaacaaa tactgtatgc 3060
tcccaataga agtcagcagt gtgttagagg aaatattagt gtttttttatc tattgcttca 3120
tttcttgtta gaacaaaatg acacatagcc cttcgtaaag tcttgtaaat ggtgaatgtt 3180
gaattctact ttatctaaat caaattttgg agccccgcag taaagttaca atctatgaat 3240
ttaagtattt aaagataaca tactgaagcc tttgttcaag tgcatcagct tctctaatta 3300
tgtgaatata tgaacttaag tgagttttta atgagttggt agattgtgat ttctccaaac 3360
taaaaaatgc aatgtttgga attatggcta tggtgttaga aaagcactaa tatataggaa 3420
ataaaagaac ttcacagtgt gagggggaaa tggtctgcaa gtatttttgg ctaaagactt 3480
cagagtcaga cacattttat cgagaacttg taatatgcaa atcagtttcc aaattttgat 3540
cttaaggcct tgtctccagg gaatctctat tacttacttc taattgaaat cagtgactta 3600
aatgtttgaa actgcagtgc ttaactctta aacatgaaat tgtagtcagt ctttggtcaa 3660
aactaactaa aatgttccca accccctagca tgatctagca aagccatggt ctcttctaag 3720
tactgtgaac atgagtctac tcacagcccc accgaaacac agctcccagg acgtttgaat 3780
atctaaggcc cagttattta atgtctttga aggcagctct ctcagcccag cccctgtgaa 3840
gaccacccac actccccttg gctgatccac atgttctctc atacggtttt ggcagctctg 3900
tgttctcctc acaattaaaa aggaaacaga ggtatggttt gggtctcact ctacacgctt 3960

ggaggctgaa aacctttttt gcttctgttc ttttctcttg ttcagggatg acctggaccg 4020
agccattgag gaatttacac tgtcctgtgc tggctactgt gtagcttctt atgtccttgg 4080
gattggtgac agacatagtg acaacatcat ggtcaaaaaa actggccagg tgagctgctc 4140
ctcaggatct gccaagggcc ttagtaatgc tatttcttat gtatagcata atctcttgtg 4200
caactcagcc agattctttt gtgattctta gtgtcatatc tttgtcttta cttcaatttc 4260
tcactacctc tcgtttcata tatagtctac tacatgtatt catttgtttg cttgcttgat 4320
ggtaagcatt tatttgttta aaaaattact aaaggctgtg tgtggtggct cacgcctgta 4380
atcccagcac tttgggatcc cgagccggcc ggattacctg aggtcaggag tttgagacca 4440
gcctggtcaa catggcgaaa ccccgtctct actaaaaata taaaaattag ccaggcatgg 4500
tggcaggcgc ctgtaatccc agctacttgg gagactgagg caggagaatc acttgaacct 4560
gggaggcgga ggttgcagtg agccgagatc gcctcactgt gctccagcct gggcaacaag 4620
agtgaaactc catctcaaaa aaaaattatt gaaaaaattt ttgtagttaa agtggcctgt 4680
tcttcaatat aagaaatagt atttgggata catttgtacc taacagaagg agcggataat 4740
gtactggatg tattaaattt aaagattacc aatgctattc atatcctttg cccacttttt 4800
gatggggttg tttgtttttt tcttgtaaat ttgtttgagt tcattgtaga ttctggacat 4860
tagccatttg tcagatgagt aggttgcgaa aattttctcc cattttgtag gttgcctgtt 4920
cactctgatg gtagtttctt ttgctgtgca gaagctcttt agtttaatta gatcccattt 4980
gtcaattttg gcttttgttg ccattgcttt tggtgtttta gacatgaagt ccttgcccat 5040
gcctatgtcc tgaatggtaa tgcctaagtt ttcttctagg gtttttatgg ttttaggtct 5100
aacgtttaag tctttaatcc aaaagaagac atttatgcag ccaacagaca catgaaaaaa 5160
tgctcatcat cactggccat cagagaaatg caaatcaaaa ccacagtggg ataccatctc 5220
acaccagtta gaatggcaat cattaaaaag tcaggaaaca acaggtgctg gagaggatgt 5280
ggagaaatag gaacactttt acattgttgg tgggactgta aactagttca accattgtgg 5340
aagtcagtgt ggcgattcct cagggatcta gaactagaaa taccatttga cccagccatc 5400
ccattactgg gtatataccc aaaggactat aaatcatgct gctataaaga cacatgcaca 5460
cgtatgttta ttgcggcact attcacaata gcaaagactt ggaaccaacc caaatgtcca 5520
acaatgatag actggattaa gaaaatgtgg cacatatata ccatggaata ctatgcagcc 5580
ataaaaaagg atgagttcat gtcctttgta gggacatgga tgaaattgga aatcatcatt 5640
ctcagtaaac tatcacaagg acaaaaaacc aaacaccgca tgttctcact catagatggg 5700
aattgaacaa tgagaacaca tggacacagg aaggggaaca tcacactctg gggactgttg 5760
tggggtgggg ggaggggga gggatagcat taggagatat acctaatgct aaaggacgag 5820
ttaatgggta cagcacacca gcatggcaca tgtatacata tgtaactaac cggcacattg 5880
tgcacatgta ccctaaaact taaaataaaa aaaaaaaaaa agattaccaa tgctaaaaaa 5940
aaaaagttgg gatgaacccc acttgagtta ttttctcttt tagaacatca tacctaatta 6000

tatatgggag aagggagaac agtcgtgtga gtaatagcat tctggggtac ctagggaatc 6060
tagaccatgt tgtttataaa gtacttaagt tttcaaatga aaatttcatt tttcagagtg 6120
acatatttgt aaacactttt tatgttaagc aaaaacagat gagatatctt agataatttt 6180
tcagtttagc ccccctagga attcccacat tagaggcata ctagaatgaa aatctctctg 6240
ggtcactgtg tgaactttgg ttctatgagg gacccacagt tttgtatctc cttggaaatc 6300
tggattagtt ctggcttggg ctttgagagt tgatgtggaa tgaatttgta atgcactata 6360
atacatgaat gcaccatgta cgattgagga atctcgtgtc catacttaaa aggagtccct 6420
tggacttccg tccaatcaca ttaaacactt agatttatct ccattttctt cttttacacc 6480
tctaaaacaa taataaggaa ttaagaaata tataaactca agaagtcaaa gatgatagga 6540
aaataggcca cagtgggtga aacctattac cagacttcgg ggtgatagaa agtgagatag 6600
agaagtggca gtgacttagc agaactgaga aaatagaaag tcagtacttg taaaggggta 6660
cgcaagtccc ataaaagctc tggaatcaga ggcaccatgt atactgttca aggagggata 6720
cagaaagagg ctgaaacaga actagttggc agcttatata tggaaccagt taggcactca 6780
agtccccttc ccttatgcca agaaggagac agatttattc tctgaagaaa ctggttctga 6840
ctcaagcaca cttatttcta cagagactac aagaaagggt cccatattaa aaatggagat 6900
ggagtgaaag tcggcgtagt aaatggtaca atctgcagcc acttccactt gctttattct 6960
aagaacagtg gcagccagat gtatagttga ccctcatcaa aagactaggt gattctattc 7020
tagaaatctg attggttcca ttgaaaagtc ctgcagatct gacagttgag atttctccct 7080
agtttctata caaggaagtc caccaatcaa caagcaagcc taccatgtac atagaatttt 7140
cattcagctt tttagtgatt cattgttaaa tatgaatggt cagctaagga ttaatatact 7200
tttaaaaaat gacaaaaatt gaatatgttt atcatgtacg acatattgtt ttgaagtatg 7260
tcttcattgt ggacttgata aattgagctg acctgtgtgt tacctcacat acttatcttt 7320
ttttgtgatg ataacattta aaacctagtc tttcagcagt tttcaggaat acaatatatt 7380
gttgttaact atagttgcca tgttgtaaga cagatctgtt gaacttattc ctcctaagtg 7440
aaattttgta ttatttgacc agtatctccc cagcacctgc agccaccatt ctactctcta 7500
ctggattagc agacatttaa gggaaacctc taccatgaaa gatagaaacc aaataggcaa 7560
aatagggaag aaaagaaaag aactctggga aaacagggta actgcagtaa acagagggtg 7620
attttaaaaa tcaaagcaaa aaaatatatt gtatctttaa agtaataata ggattctgtt 7680
ttttacaatc agcacataga ggtcttgaaa attagaaata ggaacaaaca aaaagttaaa 7740
atagaaatag tgtaaatatt ttgtggcagg catagttgta agtgctttat atataatcta 7800
atgccttcag gcctgatgac aaccttatag agtggcggca atagccccat tttacaagtg 7860
aggatattaa ggcaacagag agtagacata tcaggatttt aaccctggga gtttggctca 7920
tattcttttt ttttaatcca ccatgcctta atatctccaa aaaatatgat aaatagagag 7980
aagatccaga aacaatagag actcagtcca gggaggtata ctctcagcct aatggatatt 8040

ccagaaagaa ggagaatggc gtgaacctgg gaggtggagc ttgcagtaag ctgaaatcac 8100
gccactgcac tctagcctgg gcgacagagc gagactccgt ctcaaaaaaa aaaaaaaaaa 8160
aaaaaaagaa gaggaaagaa gggcacttac taagcaaata atatactaag acatttccca 8220
gagccaatgt tcatgaatat ccagtcaaaa tagcacaaaa attttctgag aacggaagaa 8280
aaacaccctt actaagtacc tgcactatga aatttcagtg catcagacgt aaagagaaag 8340
tcttaaaact ttcacagaga cacagcaggc cacataccag agtttagaag tcaaaatgac 8400
ttggctcctc aatagctaca ttggcaatta taagacagtg cacgttagcc ttcataattt 8460
tgagggaaaa tgatttctaa cctaaaattc gataaccaaa cttttttttt tttttctgag 8520
acggagactt gctctgtcgc ccaggctgga gtgcagtggc gtgatcccgg ctcactgcag 8580
cctccgcctc ctgggttcaa gtgattctcc tgcctcagcc tccccaggca gctgggggac 8640
cacaggcatg cgccaccacg cccagtcaag tataaagggt agaatgaaga cattttccca 8700
acttgcaggg tcacaaaact tgtcactcct ctgcatcttt tctcagaggc tgccagaata 8760
aggacatcta ccaaaacaag tgcctaaacc aataattatc aagtggggtg atttttcact 8820
caggggacat ttgttaatat atgaaaacag ttttagttgt cataactggg gggtggggta 8880
gtccagtgga tagaggccag ggatgctgct aaacacccat acaggacaga accccatatc 8940
aaagaattat atggcctatg tcagtgtgtg ccagtattga gaaaccctgg tctcaaccaa 9000
aagagaatgt gatgtggaca caaaacgggc tccaaatggg agaagaggaa agggaaggcc 9060
caggatgatg gctctgcagc aacaccggag aacaaccagc ccagctagaa accagtagat 9120
tacctgactg tctggaaaac agttttgaaa atgatttgta gatttgttgt tgtttgattt 9180
gtagattttt aaggagagtt tgggaagaat taatgctaag gtcatagaac actaagctaa 9240
atgaatgaat gaatacatgc gtacagtttt ctaaaggaaa aaaaggtgaa catgtgaaaa 9300
ataaaaacac tgaatattga tgtaaccaga aattatgaca taatgtgcca cagtgtgtag 9360
cattatgtta gcataaaagt actaaatctt tatcttccat aataagaata gtaatataca 9420
attagggagc aaaaataaat ataaacatat tatttagaaa aatggaggca gacaccagga 9480
aaaacaccta gtgagaagag taagaagtta cctctaaaga gtagcactca atgtggggag 9540
ctagtagggc aaggatgtac tttttgtgg ttgttgttgt tgttgagaca gggtcttact 9600
ctgtcaccca ggcaagagcg tagtggcatc atcatggccc attgcagcct cgacctccta 9660
ggctcaagca attcccccac ctcacccccc ctgagtagct gggaccacag gtgtgtgcca 9720
ctatgcctga ctaatttttt ccttcatttg tagagatggg gtctcgctat gttgcctagg 9780
ctggccttga actcctggtt tcaagtgatc ctcctgcctc agcttcccaa agtgctggga 9840
ttgcagacat gagccaccac aaccaacctg tactcttttc ttgttatgtt ttatagaact 9900
atttgacttt ttaaaaatca gacattttaa ttctttggat gtatttttct ttctaaagcg 9960
tctccttttc cactagatat ctacagttta atatcagggt tatttattat tgattgtaaa 10020
agtcttaaga gtgttagata tggtctcttc tcacctggct cagtgggcta taaacagagg 10080

gagaagggct cagatgtgga tgggtatagt tcctgggggt ctaggactat ggaggttttg 10140
cctttatatt tggaacccac cagaaaaatg aaggaaatta aatcccattt gttttccagc 10200
tcttccacat tgactttgga catattcttg gaaatttcaa atctaagttt ggcattaaaa 10260
gggagcgagt gccttttatt cttacctatg atttcatcca tgtcattcaa caaggaaaaa 10320
caggaaatac agaaaagttt ggccggtgag tactgccctt gtgccaaggc tgaacacttc 10380
taacattttc ttatctgacc aggtggacca gcatttctta gctgagatat atttggatct 10440
gggagatatt cagtctgatt ataggaagct tttgggggaa tttgcctgtc agattattgt 10500
gctggttcag aaattcccag ataggagaaa cagaatgcta gaaatttaaa atagttatta 10560
tattttattc caataatata ctacctttta cctgtttcag aacattctct gaaactatta 10620
agacagttga taggaagatg ctgaaaagac atctgctgtc attgtgtatg gagtacagta 10680
agggaactag aattcagggc aaattttta atctctgatc tattactggc tacctacatg 10740
ttcccaagca taatacttgt ctttttgcct ttgtatcatc ttaaaatggg gacaaaaata 10800
tgaaattaag ggctactatg atggttagag acatatcatg taaccaacac atacttaaca 10860
aatcattgtc actatccttt atagttctgt ttgggtttct gcagaatata cctcagtggg 10920
tctagttttc tctttggcaa aataagggga tacgattgga tggtcctcag cctggcgcgg 10980
taggtcatgc ctgtaatctc agcactttgg gaggccaagg cacacagatc actggaggtc 11040
ggagtttgag accagcctga ccaacatggt gaaaccccgt ctctactaaa aatacaaaaa 11100
gtagccaggt gtggtggcac acgcctgtaa tctcagctac tcaggaggct gaggcagaat 11160
tgcttgaacc tggaaggcag aagttgcagt gagccaagat cacgccactg cactccagcc 11220
tgggagacag agcaaggttc catctcaaaa aaaaaacatt ggatgatctt cttgagggac 11280
catcttgaag aggagagaga gggaaggcag gatgacagga aggagcaaaa gcactgacac 11340
tgattgaggg gacttttaaa aaattagttt ctggaatcag accacaaata tagaacccag 11400
aagtatgttc agaaatcctt gtggattata attataactg atttaataat cagttcttgt 11460
gtctcataga attaaaaagt ctagattatt attaaaaatg taatagcctt tcgtcataga 11520
atttctattt agttttgttt ttgaaaaata tatctgtgat gttagagaga ttgattgttt 11580
tatgtagatt ttagtcctgg gacaattttc gcagaagtaa aaatcagaaa tgaaccttta 11640
gttcagtagg aattttctta ttctaataga aagtctagct gtgttttctt aatttcctgt 11700
atgaaatgtg ctctctcccc tctaacactg tgctcatgtg gtttgctgca tcacccaaag 11760
gttccgccag tgttgtgagg atgcatatct gattttacga cggcatggga atctcttcat 11820
cactctcttt gcgctgatgt tgactgcagg gcttcctgaa ctcacatcag tcaaagatat 11880
acagtatctt aaggtataaa accacttttc cttctctctt ggactttgtg ggcattgagc 11940
tcagttttag ctgcctgttt tattcaagtg gctgaaggaa gtagaacaaa gtcatttcct 12000
ctaagatggt tcttagccag gagggaaaga atctgggaag tacataaagg aggaattttg 12060
tagagtagct tgtaacccag aagattttcc catcagtaga aggggcgtaa agaagactgg 12120

tattgggctg tagccttctt actcacatta ctgaaagacc gcagatcagg agcgggttgc 12180
caccttgatt ttccacagtc tgcctttttc ttgccaggat cttagtaggt cttgagtcat 12240
cttactggat ttgggtggta gtggcaagca gctgtgcatc ctgcagtaat tataaattat 12300
tttcatcttc aaaaaccttt tggaagaagt catattatct ccctatcttg gggttctgcc 12360
tcctgcagat gatatttaaa taagaacatg aagcatgctg cctgatggtg gctggggagg 12420
cacaaccaat ccagcctcct gcagactttg atatttgcac atctctacta aagttatata 12480
aaatcatatt ttccccttc cattttagga ctctcttgca ttagggaaga gtgaagaaga 12540
agcactcaaa cagtttaagc aaaaatttga tgaggcgctc agggaaagct ggactactaa 12600
agtgaactgg atggcccaca cagttcggaa agactacaga tcttaacgat cagccttcgc 12660
tcctaatgta tttgttggtt tcatttcatt ttcattttgc acttgcacta aattgaacat 12720
gaccctgtta gagatgttat aaagggaatg aaatcctgga actcagagtt aaattaagaa 12780
caaggcatcc cacagaacct aatctgaaca atccccgatg attccctctg ctttttgaat 12840
gcttccaaga cttatcatga aaactgtcaa tggataatca tttcctgctg actttgcacg 12900
ccaaggaatg ctactaggga ttgtttccgt ttttgtttgt tttttctaat atttggtact 12960
tcccagaatg gtgtaaatac ttcttttcaa tgttgtgacc aagtattgtc actcagccaa 13020
caacttttcc acacctgggg gttggtggct gttcttactg tccaaatgaa gctaaaaaga 13080
aaggcatctt tcttcccttt taaaattgtg taaactgcaa attataatat aatttgaatt 13140
tatgattatt ttccagaaga aatcttgtaa acctgtggat actcattaat tcttttgtta 13200
atatttattt ccatgatagc atcattccag ccagacttgc tgaaaatcta ctggtgaggc 13260
aaatataata tatataaata tgctacatat atatttataa aatttctagt gggagttcta 13320
tataaatgtt tctttggtat tcttcagcct gtgatttaaa gttttacaaa aagcagagct 13380
ttttcctaag ttacttttca gttaggtaac tgtgtgatcc agttcttcca gctgcttcta 13440
taatgaggca catattaata cagtttttat atggtatcta tgaaagagtt cacttcatag 13500
agaataatac ttgagcaaat gtatccaaga aagcaagcaa atgaaaagaa acctatttat 13560
ggaataaact ccagatctga aattcagtat tttagaaaaa tgccagctct tcttactgta 13620
tttattaaaa cttgtaataa tgtgattttt ttcaaggata ttagttcaaa ttgaaatggt 13680
ttcacgccac acggaaatct ttaagttatt tgttgaggta ccatatattt agggtgctag 13740
gggcaagtaa tgttaatatg tgcaatagga actactggtt tgaatgtgta aatgggtgat 13800
ctctctgagt cctggcaaca tccagcaaaa ctactgctta ttctccaaag aatattggga 13860
gctctcaatc ctcggtgata tgggaaagag aactgagtat ttgccctatg actgagcttt 13920
ctataggaat tttattaaag aatgtttaat tttgttgtcc ttcttaatgt tctcagtcaa 13980
ataaatgagt gagctggttt cggctgctct tggaatgggt gagcctcttc tttatgggta 14040
gactgggcct ttggaacttg gcactggaac tccaagaaat ggccaagtca gtagacaaac 14100
caacctcagg aataggctaa ggcttattat ggcctcttcc ctgacttctc cccttgtttc 14160

ccagcctcat caggcatggt ataggaggcc ccctggactt tggtgggagc ctgaggtaag 14220
gagccatgca tatgggaggt gtcctgaagt ctgggtagtt acttggcact gagccaaggc 14280
cagactctgc tgctttggag ctcttgttca tggggcagat gctggagcag tccagttcct 14340
tggaaataac tcagctgagg atgggagttg gcccctgaat tcctcatttc cagggctggt 14400
gtagactcac tgagacttcc aggaatagaa ctatggaagg acaggtttgt tcagagatct 14460
ttgtctagta gccacccacc atttcatgaa ccaggccgca ggtcagtggt ttggagaatg 14520
gtgaacactg ccaggaagaa atggatacca ttctttccag aggggtctcc tcagccaaaa 14580
ggagggcctt gataaataca tgccaaatca gtgaagttca agtcaactgt ttttcccata 14640
tgggcaccaa attgtatctt tcctgttttc tttgaagggt taagtaacgt gaccatagtc 14700
acagagtagt tgatggagcc agtattcaaa cccagaaagt aagaagccta ttttaattat 14760
ctgtgctctt tactcacaat gcctcagtat acatttcaga tttattgggt tccacaaata 14820
gaaacctatg gaaattttga atcaaattgc attaagctat agacaaggtt gagacaaatt 14880
gacatctcca gaatattgag ttttccaaaa tatgtaagtg gagtacccat ttatttagat 14940
tatctttcat ttatatccgc aatgatttat agtattctgt gtttacatat tatgcatcgt 15000
ttgttagatt cctgggtaag tgactttatt gtaagtttca ttgttgttaa tctatagcaa 15060
tcattctcca agtgtggtcc cctgatgagg agcatcagca tcaccagaga gaactggtta 15120
aaaatgcaaa ttcttaattt ttaatttttg tgagtacata gtagatatat atatgggata 15180
catggaatat tttgatacag gcatataaca tgtaattatc gcatcagggt aattggggta 15240
tccattacct caagcattta tcctttgtat tagaaacaat ccagttatac tcttttagtt 15300
attttaagat ctataattaa attatcgact atagttactc tgttgtgcta tcaaatacta 15360
gatcttattc attcttacta tttttttgt acccatagaa atgcagattc ttggtggggc 15420
ctggcagctc acacctgtaa tcccagcatt ttgggagggc gaggccgggg aatcacctga 15480
ggtcaggagt tcaagattag cctggccaac atggtgaaac ctgtatctac taaaaacaca 15540
aaaattagct gggcatggtg gctggctcct gtaatcccag ctactcgaaa ggctgaggta 15600
ggagaatcac ttgaacccag gaggcggagg ttgcagtagc cgagatcaca ccactgcact 15660
ccagcctggg taacagagtg agactccatc tcaaaaaaag aaaaaaaaaa gaaatgcaca 15720
tccttgagcc ctgccctgga gctactgatt tagaaatggg ggtggagccc caaacctgta 15780
tttaatttaa tttatttatt tatttatttt ttgagatgga gtctcgcttt gttgcccagg 15840
ctggagtgca gtggtgtgat ctcgattcac ttcaacctcc acctcccagg ttcaagcaat 15900
tatgtctcaa cctcccgagt ttagctggga ctacaggtat gcaccaccat gtccagctaa 15960
tttttgtatt tttaataaag acagggtttc accatattgg tcaggctggt ctcgaactcc 16020
tgacctcagg tgatccacct acctcaacct cccaaagtgc tgggattata ggcatgagcc 16080
accgcaccca gccaaacctg tattttcata aagttccaga gcaaaggtcc acaaatacat 16140
ttgatgtttg tatttagcag acttataaac tttctaatta atcctaacaa tttatttgta 16200

tgaatttttt tttttttttc ttgagacagg gtctcactat gtcacccagg ctggagtgca 16260
gtggcgtact ctcggctcac tgcaacctct gtctcctggg cttaggtggt cctccgacct 16320
cagcctcctg aatagctggg gccacaggca tgcaccacca cacgcagcta atttttgttt 16380
gtttgtttgt ttgttttaag agacggaggt ttaccatgtt gcccaggttg gcctcaaact 16440
tctggactca agcagtctg 16459

<210> 87
<211> 1145
<212> RNA
<213> Homo sapiens

<400> 87

attctctccc cagcttgctg agccctttgc tcccctggcg actgcctgga cagtcagcaa 60
ggaattgtct cccagtgcat tttgccctcc tggctgccaa ctctggctgc taaagcggct 120
gccacctgct gcagtctaca cagcttcggg aagaggaaag gaacctcaga ccttccagat 180
cgcttcctct cgcaacaaac tatttgtcgc aggaataaag atggctgctg aaccagtaga 240
agacaattgc atcaactttg tggcaatgaa atttattgac aatacgcttt actttatagc 300
tgaagatgat gaaaacctgg aatcagatta ctttggcaag cttgaatcta aattatcagt 360
cataagaaat ttgaatgacc aagttctctt cattgaccaa ggaaatcggc ctctatttga 420
agatatgact gattctgact gtagagataa tgcaccccgg accatattta ttataagtat 480
gtataaagat agccagccta gaggtatggc tgtaactatc tctgtgaagt gtgagaaaat 540
ttcaactctc tcctgtgaga acaaaattat ttcctttaag gaaatgaatc ctcctgataa 600
catcaaggat acaaaaagtg acatcatatt ctttcagaga agtgtcccag gacatgataa 660
taagatgcaa tttgaatctt catcatacga aggatacttt ctagcttgtg aaaaagagag 720
agaccttttt aaactcattt tgaaaaaaga ggatgaattg ggggatagat ctataatgtt 780
cactgttcaa aacgaagact agctattaaa atttcatgcc gggcgcagtg gctcacgcct 840
gtaatcccag ccctttggga ggctgaggcg ggcagatcac cagaggtcag gtgttcaaga 900
ccagcctgac caacatggtg aaacctcatc tctactaaaa atacaaaaaa ttagctgagt 960
gtagtgacgc atgccctcaa tcccagctac tcaagaggct gaggcaggag aatcacttgc 1020
actccggagg tagaggttgt ggtgagccga gattgcacca ttgcgctcta gcctgggcaa 1080
caacagcaaa actccatctc aaaaaataaa ataaataaat aaacaaataa aaaattcata 1140
atgtg 1145

<210> 88
<211> 732
<212> RNA
<213> Homo sapiens

<400> 88

```
tttttttttt ttttttttgg acacagggtc ttgctgttgc ccaggctgga gtgcagtggc    60
atgaccatag ctcactgcag ccttgacttc cttaactcaa gcaatcctct tgcctcagcc   120
tcctgtagca ctgtaggcac acacaactat gcctggctaa ttttaacatt tttctttcac   180
cttcttgacc cttatcttct atacccggct aatttttttgt agagacagtg tcttgctatg   240
ttgtccaagc tggtcttgaa ttcctcgcct caagcaatcc ttccacctca gcttcctgag   300
tgttaggatt acaggcatga gccactgcac ctggcctcca acaggtaatt ttagaacatt   360
tttccctcta cactaattac cctcctataa cctccatttg ttatcactta ctttctgatg   420
ttgtattcat agagcatgaa tatcttagaa agatggcacc atccttctat taataagacc   480
agcagaatag ctcagtttaa agttcctcta aacccaagaa aatatcaaac aaaaatgtct   540
tttttagat aaatttgaag tcagaagata ttttgatatg agtctagtca tctcttggta    600
tccatggggg attggttcct gaaacccttg gataccaaaa tccacagaag gatgctcaag   660
tctctgtaaa atagcatagt atttatatat agcctatgca catttcccca tacactttag   720
attactctag at                                                       732
```

<210> 89
<211> 2914
<212> RNA
<213> Homo sapiens

<400> 89

```
gtgctctgag tttttggttt ctgtttcacc ttgtgtctga gctggtctga aggctggttg     60
ttcagactga gcttcctgcc tgcctgtacc ccgccaacag cttcagaaga aggtgactgg    120
tggctgcctg aggaatacca gtgggcaaga gaattagcat ttctggagca tctgctgtct    180
gagcagcccc tgggtgcgtc cactttctgg gcacgtgagg ttgggccttg gccgcctgag    240
cccttgagtt ggtcacttga accttgggaa tattgagatt atattctcct gccttttaaa    300
aagatggact tcagcagaaa tctttatgat attggggaac aactggacag tgaagatctg    360
gcctccctca agttcctgag cctggactac attccgcaaa ggaagcaaga acccatcaag    420
gatgccttga tgttattcca gagactccag gaaaagagaa tgttggagga aagcaatctg    480
tccttcctga aggagctgct cttccgaatt aatagactgg atttgctgat tacctaccta    540
aacactagaa aggaggagat ggaaagggaa cttcagacac caggcagggc tcaaatttct    600
gcctacaggt tccacttctg ccgcatgagc tgggctgaag caaacagcca gtgccagaca    660
cagtctgtac ctttctggcg gagggtcgat catctattaa taagggtcat gctctatcag    720
atttcagaag aagtgagcag atcagaattg aggtctttta agtttctttt gcaagaggaa    780
atctccaaat gcaaactgga tgatgacatg aacctgctgg atattttcat agagatggag    840
aagagggtca tcctgggaga aggaaagttg gacatcctga aaagagtctg tgcccaaatc    900
aacaagagcc tgctgaagat aatcaacgac tatgaagaat tcagcaaagg ggaggagttg    960
tgtggggtaa tgacaatctc ggactctcca agagaacagg atagtgaatc acagactttg   1020
gacaaagttt accaaatgaa aagcaaacct cggggatact gtctgatcat caacaatcac   1080
aattttgcaa aagcacggga gaaagtgccc aaacttcaca gcattaggga caggaatgga   1140
acacacttgg atgcaggggc tttgaccacg acctttgaag agcttcattt tgagatcaag   1200
```

ccccacgatg actgcacagt agagcaaatc tatgagattt tgaaaatcta ccaactcatg 1260
gaccacagta acatggactg cttcatctgc tgtatcctct cccatggaga caagggcatc 1320
atctatggca ctgatggaca ggaggccccc atctatgagc tgacatctca gttcactggt 1380
ttgaagtgcc cttcccttgc tggaaaaccc aaagtgtttt ttattcaggc ttgtcagggg 1440
gataactacc agaaaggtat acctgttgag actgattcag aggagcaacc ctatttagaa 1500
atggatttat catcacctca aacgagatat atcccggatg aggctgactt tctgctgggg 1560
atggccactg tgaataactg tgtttcctac cgaaaccctg cagagggaac ctggtacatc 1620
cagtcacttt gccagagcct gagagagcga tgtcctcgag gcgatgatat tctcaccatc 1680
ctgactgaag tgaactatga agtaagcaac aaggatgaca agaaaaacat ggggaaacag 1740
atgcctcagc ctactttcac actaagaaaa aaacttgtct tcccttctga ttgatggtgc 1800
tattttgttt gttttgtttt gttttgtttt tttgagacag aatctcgctc tgtcgcccag 1860
gctggagtgc agtggcgtga tctcggctca ccgcaagctc cgcctcccgg gttcaggcca 1920
ttctcctgcc tcagcctccc gagtagctgg gactacaggg gcccgccacc acacctggct 1980
aatttttttaa aaatattttt agtagagaca gggtttcact gtgttagcca gggtggtctt 2040
gatctcctga cctcgtgatc cacccacctc ggcctcccaa agtgctggga ttacaggcgt 2100
gagccaccgc gcctggccga tggtactatt tagatataac actatgttta tttactaatt 2160
ttctagattt tctactttat taattgtttt gcactttttt ataagagcta aagttaaata 2220
ggatattaac aacaataaca ctgtctcctt tctcttatgc ttaaggcttt gggaatgttt 2280
ttagctggtg gcaataaata ccagacacgt acaaaatcca gctatgaata tagagggctt 2340
atgattcaga ttgttatcta tcaactataa gcccactgtt aatattctat taactttaat 2400
tctctttcaa agctaaattc cacactacca cattaaaaaa attagaaagt agccacgtat 2460
ggtggctcat gtctataatc ccagcacttt gggaggttga ggtgggagga ttgcttgaac 2520
ccaagaggtc aaggctgcag tgagccatgt tcacaccgct gcactcaagc ttgggtgaca 2580
gaacaagacc ccgtctcaaa aaaaattttt tttttaataa aacaaaattt gtttgaaatc 2640
ttttaaaaat tcaaatgatt tttacaagtt ttaaataagc tctccccaaa cttgctttat 2700
gccttcttat tgcttttatg atatatatat gcttggctaa ctatatttgc tttttgctaa 2760
caatgctctg gggtcttttt atgcatttgc atttgctctt tcatctctgc ttggattatt 2820
ttaaatcatt aggaattaag ttatctttaa aatttaagta tctttttttca aaaacatttt 2880
ttaatagaat aaaatataat ttgatcttat taaa 2914

<210> 90
<211> 2153
<212> RNA
<213> Homo sapiens

<400> 90

aagactgcga gctccccgca ccccctcgca ctccctctgg ccggcccagg gcgccttcag 60

cccaacctcc ccagccccac gggcgccacg gaacccgctc gatctcgccg ccaactggta 120
gacatggaga cccctgcctg gccccgggtc ccgcgccccg agaccgccgt cgctcggacg 180
ctcctgctcg gctgggtctt cgcccaggtg gccggcgctt caggcactac aaatactgtg 240
gcagcatata atttaacttg gaaatcaact aatttcaaga caattttgga gtgggaaccc 300
aaacccgtca atcaagtcta cactgttcaa ataagcacta agtcaggaga ttggaaaagc 360
aaatgctttt acacaacaga cacagagtgt gacctcaccg acgagattgt gaaggatgtg 420
aagcagacgt acttggcacg ggtcttctcc tacccggcag ggaatgtgga gagcaccggt 480
tctgctgggg agcctctgta tgagaactcc ccagagttca caccttacct ggagacaaac 540
ctcggacagc caacaattca gagttttgaa caggtgggaa caaaagtgaa tgtgaccgta 600
gaagatgaac ggactttagt cagaaggaac aacactttcc taagcctccg ggatgttttt 660
ggcaaggact taatttatac actttattat tggaaatctt caagttcagg aaagaaaaca 720
gccaaaacaa acactaatga gtttttgatt gatgtggata aaggagaaaa ctactgtttc 780
agtgttcaag cagtgattcc ctcccgaaca gttaaccgga agagtacaga cagcccggta 840
gagtgtatgg gccaggagaa aggggaattc agagaaatat tctacatcat tggagctgtg 900
gtatttgtgg tcatcatcct tgtcatcatc ctggctatat ctctacacaa gtgtagaaag 960
gcaggagtgg ggcagagctg gaaggagaac tccccactga atgtttcata aaggaagcac 1020
tgttggagct actgcaaatg ctatattgca ctgtgaccga gaacttttaa gaggatagaa 1080
tacatggaaa cgcaaatgag tatttcggag catgaagacc ctggagttca aaaaactctt 1140
gatatgacct gttattacca ttagcattct ggttttgaca tcagcattag tcactttgaa 1200
atgtaacgaa tggtactaca accaattcca agttttaatt tttaacacca tggcaccttt 1260
tgcacataac atgctttaga ttatatattc cgcacttaag gattaaccag gtcgtccaag 1320
caaaaacaaa tgggaaaatg tcttaaaaaa tcctgggtgg acttttgaaa agcttttttt 1380
tttttttttt tttgagacgg agtcttgctc tgttgcccag gctggagtgc agtagcacga 1440
tctcggctca cttgcaccct ccgtctctcg ggttcaagca attgtctgcc tcagcctccc 1500
gagtagctgg gattacaggt gcgcactacc acgccaagct aatttttgta tttttagta 1560
gagatggggt ttcaccatct tggccaggct ggtcttgaat tcctgacctc agtgatccac 1620
ccaccttggc ctcccaaaga tgctagtatt atgggcgtga accaccatgc ccagccgaaa 1680
agcttttgag gggctgactt caatccatgt aggaaagtaa aatggaagga aattgggtgc 1740
atttctagga cttttctaac atatgtctat aatatagtgt ttaggttctt tttttttca 1800
ggaatacatt tggaaattca aaacaattgg gcaaactttg tattaatgtg ttaagtgcag 1860
gagacattgg tattctgggc agcttcctaa tatgctttac aatctgcact ttaactgact 1920
taagtggcat taaacatttg agagctaact atatttttat aagactacta tacaaactac 1980
agagtttatg atttaaggta cttaaagctt ctatggttga cattgtatat ataatttttt 2040
aaaaaggttt ttctatatgg ggattttcta tttatgtagg taatattgtt ctatttgtat 2100

atattgagat aatttattta atatacttta aataaaggtg actgggaatt gtt 2153

<210> 91
<211> 5133
<212> RNA
<213> Homo sapiens

<400> 91

```
cctctttcac cctgtctagg ttgccagcaa atcccacggg cctcctgacg ctgcccctgg    60
ggccacaggt ccctcgagtg ctggaaggat gaaggattcc tgcatcactg tgatggccat   120
ggcgctgctg tctgggttct tttcttcgc gccggcctcg agctacaacc tggacgtgcg    180
gggcgcgcgg agcttctccc caccgcgcgc cgggaggcac tttggatacc gcgtcctgca   240
ggtcggaaac ggggtcatcg tgggagctcc aggggagggg aacagcacag gaagcctcta   300
tcagtgccag tcgggcacag gacactgcct gccagtcacc ctgagaggtt ccaactatac   360
ctccaagtac ttgggaatga ccttggcaac agaccccaca gatggaagca ttttggcctg   420
tgaccctggg ctgtctcgaa cgtgtgacca gaacacctat ctgagtggcc tgtgttacct   480
cttccgccag aatctgcagg gtcccatgct gcaggggcgc cctggttttc aggaatgtat   540
caagggcaac gtagacctgg tatttctgtt tgatggttcg atgagcttgc agccagatga   600
atttcagaaa attctggact tcatgaagga tgtgatgaag aaactcagca acacttcgta   660
ccagtttgct gctgttcagt tttccacaag ctacaaaaca gaatttgatt tctcagatta   720
tgttaaatgg aaggaccctg atgctctgct gaagcatgta aagcacatgt tgctgttgac   780
caataccttt ggtgccatca attatgtcgc gacagaggtg ttccgggagg agctgggggc   840
ccggccagat gccaccaaag tgcttatcat catcacggat ggggaggcca ctgacagtgg   900
caacatcgat gcggccaaag acatcatccg ctacatcatc gggattggaa agcattttca   960
gaccaaggag agtcaggaga ccctccacaa atttgcatca aaacccgcga gcgagtttgt  1020
gaaaattctg gacacatttg agaagctgaa agatctattc actgagctgc agaagaagat  1080
ctatgtcatt gagggcacaa gcaaacagga cctgacttcc ttcaacatgg agctgtcctc  1140
cagcggcatc agtgctgacc tcagcagggg ccatgcagtc gtgggggcag taggagccaa  1200
ggactgggct gggggctttc ttgacctgaa ggcagacctg caggatgaca catttattgg  1260
gaatgaacca ttgacaccag aagtgagagc aggctatttg ggttacaccg tgacctggct  1320
gccctcccgg caaaagactt cgttgctggc ctcgggagcc cctcgatacc agcacatggg  1380
ccgagtgctg ctgttccaag agccacaggg cggaggacac tggagccagg tccagacaat  1440
ccatgggacc cagattggct cttatttcgg tggggagctg tgtggcgtcg acgtggacca  1500
agatggggag acagagctgc tgctgattgg tgccccactg ttctatgggg agcagagagg  1560
aggccgggtg tttatctacc agagaagaca gttggggttt gaagaagtct cagagctgca  1620
gggggacccc ggctacccac tcgggcggtt tggagaagcc atcactgctc tgacagacat  1680
caacggcgat gggctggtag acgtggctgt gggggcccct ctggaggagc agggggctgt  1740
gtacatcttc aatgggaggc acgggggct tagtccccag ccaagtcagc ggatagaagg    1800
```

gacccaagtg ctctcaggaa ttcagtggtt tggacgctcc atccatgggg tgaaggacct 1860
tgaaggggat ggcttggcag atgtggctgt gggggctgag agccagatga tcgtgctgag 1920
ctcccggccc gtggtggata tggtcaccct gatgtccttc tctccagctg agatcccagt 1980
gcatgaagtg gagtgctcct attcaaccag taacaagatg aaagaaggag ttaatatcac 2040
aatctgtttc cagatcaagt ctctctaccc ccagttccaa ggccgcctgg ttgccaatct 2100
cacttacact ctgcagctgg atggccaccg gaccagaaga cgggggttgt tcccaggagg 2160
gagacatgaa ctcagaagga atatagctgt caccaccagc atgtcatgca ctgacttctc 2220
atttcatttc ccggtatgtg ttcaagacct catctccccc atcaatgttt ccctgaattt 2280
ctctctttgg gaggaggaag ggacaccgag ggaccaaagg gcgcagggca aggacatacc 2340
gcccatcctg agaccctccc tgcactcgga aacctgggag atccctttttg agaagaactg 2400
tggggaggac aagaagtgtg aggcaaactt gagagtgtcc ttctctcctg caagatccag 2460
agccctgcgt ctaactgctt ttgccagcct ctctgtggag ctgagcctga gtaacttgga 2520
agaagatgct tactgggtcc agctggacct gcacttcccc ccgggactct ccttccgcaa 2580
ggtggagatg ctgaagcccc atagccagat acctgtgagc tgcgaggagc ttcctgaaga 2640
gtccaggctt ctgtccaggg cattatcttg caatgtgagc tctcccatct tcaaagcagg 2700
ccactcggtt gctctgcaga tgatgtttaa tacactggta aacagctcct gggggactc 2760
ggttgaattg cacgccaatg tgacctgtaa caatgaggac tcagacctcc tggaggacaa 2820
ctcagccact accatcatcc ccatcctgta ccccatcaac atcctcatcc aggaccaaga 2880
agactccaca ctctatgtca gtttcacccc caaaggcccc aagatccacc aagtcaagca 2940
catgtaccag gtgaggatcc agccttccat ccacgaccac aacataccca ccctggaggc 3000
tgtggttggg gtgccacagc ctcccagcga ggggcccatc acacaccagt ggagcgtgca 3060
gatggagcct cccgtgccct gccactatga ggatctggag aggctcccgg atgcagctga 3120
gccttgtctc cccggagccc tgttccgctg ccctgttgtc ttcaggcagg agatcctcgt 3180
ccaagtgatc gggactctgg agctggtggg agagatcgag gcctcttcca tgttcagcct 3240
ctgcagctcc ctctccatct ccttcaacag cagcaagcat ttccacctct atggcagcaa 3300
cgcctccctg gcccaggttg tcatgaaggt tgacgtggtg tatgagaagc agatgctcta 3360
cctctacgtg ctgagcggca tcggggggct gctgctgctg ctgctcattt tcatagtgct 3420
gtacaaggtt ggtttcttca aacggaacct gaaggagaag atggaggctg gcagaggtgt 3480
cccgaatgga atccctgcag aagactctga gcagctggca tctgggcaag aggctgggga 3540
tcccggctgc ctgaagcccc tccatgagaa ggactctgag agtggtggtg gcaaggactg 3600
agtccaggcc tgtgaggtgc agagtgccca gaactggact caggatgccc agggccactc 3660
tgcctctgcc tgcattctgc cgtgtgccct cgggcgagtc actgcctctc cctggccctc 3720
agtttcccta tctcgaacat ggaactcatt cctgaatgtc tcctttgcag gctcataggg 3780
aagacctgct gagggaccag ccaagagggc tgcaaaagtg agggcttgtc attaccagac 3840

ggttcaccag cctctcttgg ttccttcctt ggaagagaat gtctgatcta aatgtggaga 3900
aactgtagtc tcaggaccta gggatgttct ggccctcacc cctgccctgg gatgtccaca 3960
gatgcctcca ccccccagaa cctgtccttg cacactcccc tgcactggag tccagtctct 4020
tctgctggca gaaagcaaat gtgacctgtg tcactacgtg actgtggcac acgccttgtt 4080
cttggccaaa gaccaaattc cttggcatgc cttccagcac cctgcaaaat gagaccctcg 4140
tggccttccc cagcctcttc tagagccgtg atgcctccct gttgaagctc tggtgacacc 4200
agcctttctc ccaggccagg ctccttcctg tcttcctgca ttcacccaga cagctccctc 4260
tgcctgaacc ttccatctcg cccacccctc cttccttgac cagcagatcc cagctcacgt 4320
cacacacttg gttgggtcct cacatctttc acacttccac caccctgcac tactccctca 4380
aagcacacgt catgtttctt catccggcag cctggatgtt ttttccctgt ttaatgattg 4440
acgtacttag cagctatctc tcagtgaact gtgagggtaa aggctatact tgtcttgttc 4500
accttgggat gacgccgcat gatatgtcag ggcgtgggac atctagtagg tgcttgacat 4560
aatttcactg aattaatgac agagccagtg ggaagataca gaaaaagagg gccggggctg 4620
ggcgcggtgg ttcacgcctg taatcccagc actttgggag gccaaggagg gtggatcacc 4680
tgaggtcagg agttagaggc cagcctggcg aaaccccatc tctactaaaa atacaaaatc 4740
caggcgtggt ggcacacacc tgtagtccca gctactcagg aggttgaggt aggagaattg 4800
cttgaacctg ggaggtggag gttgcagtga gccaagattg cgccattgca ctccagcctg 4860
ggcaacacag cgagactccg tctcaaggaa aaaataaaaa taaaaagcgg gcacgggccc 4920
ggacatcccc acccttggag gctgtcttct caggctctgc cctgccctag ctccacaccc 4980
tctcccagga cccatcacgc ctgtgcagtg gcccccacag aaagactgag ctcaaggtgg 5040
gaaccacgtc tgctaacttg gagccccagt gccaagcaca gtgcctgcat gtatttatcc 5100
aataaatgtg aaattctgtc caaaaaaaaa aaa 5133

<210> 92
<211> 2357
<212> RNA
<213> Homo sapiens

<400> 92

cgagcttggc tgcttctggg gcctgtgtgg ccctgtgtgt cggaaagatg gagcaagaag 60
ccgagcccga ggggcggccg cgacccctct gaccgagatc ctgctgcttt cgcagccagg 120
agcaccgtcc ctccccggat tagtgcgtac gagcgcccag tgccctggcc cggagagtgg 180
aatgatcccc gaggcccagg gcgtcgtgct tccgcgcgcc ccgtgaagga aactggggag 240
tcttgaggga cccccgactc caagcgcgaa aaccccggat ggtgaggagc aggcaaatgt 300
gcaataccaa catgtctgta cctactgatg gtgctgtaac cacctcacag attccagctt 360
cggaacaaga gaccctggtt agaccaaagc cattgctttt gaagttatta aagtctgttg 420
gtgcacaaaa agacacttat actatgaaag aggttctttt ttatcttggc cagtatatta 480

tgactaaacg attatatgat gagaagcaac aacatattgt atattgttca aatgatcttc 540
taggagattt gtttggcgtg ccaagcttct ctgtgaaaga gcacaggaaa atatatacca 600
tgatctacag gaacttggta gtagtcaatc agcaggaatc atcggactca ggtacatctg 660
tgagtgagaa caggtgtcac cttgaaggtg ggagtgatca aaaggacctt gtacaagagc 720
ttcaggaaga gaaaccttca tcttcacatt tggtttctag accatctacc tcatctagaa 780
ggagagcaat tagtgagaca gaagaaaatt cagatgaatt atctggtgaa cgacaaagaa 840
aacgccacaa atctgatagt atttcccttt cctttgatga aagcctggct ctgtgtgtaa 900
taagggagat atgttgtgaa agaagcagta gcagtgaatc tacagggacg ccatcgaatc 960
cggatcttga tgctggtgta agtgaacatt caggtgattg gttggatcag gattcagttt 1020
cagatcagtt tagtgtagaa tttgaagttg aatctctcga ctcagaagat tatagcctta 1080
gtgaagaagg acaagaactc tcagatgaag atgatgaggt atatcaagtt actgtgtatc 1140
aggcagggga gagtgataca gattcatttg aagaagatcc tgaaatttcc ttagctgact 1200
attggaaatg cacttcatgc aatgaaatga atcccccct tccatcacat tgcaacagat 1260
gttgggccct tcgtgagaat tggcttcctg aagataaagg gaaagataaa ggggaaatct 1320
ctgagaaagc caaactggaa aactcaacac aagctgaaga gggctttgat gttcctgatt 1380
gtaaaaaaac tatagtgaat gattccagag agtcatgtgt tgaggaaaat gatgataaaa 1440
ttacacaagc ttcacaatca caagaaagtg aagactattc tcagccatca acttctagta 1500
gcattattta tagcagccaa gaagatgtga aagagtttga aagggaagaa acccaagaca 1560
aagaagagag tgtggaatct agtttgcccc ttaatgccat tgaaccttgt gtgatttgtc 1620
aaggtcgacc taaaaatggt tgcattgtcc atggcaaaac aggacatctt atggcctgct 1680
ttacatgtgc aaagaagcta aagaaaagga ataagccctg cccagtatgt agacaaccaa 1740
ttcaaatgat tgtgctaact tatttcccct agttgacctg tctataagag aattatatat 1800
ttctaactat ataaccctag gaatttagac aacctgaaat ttattcacat atatcaaagt 1860
gagaaaatgc ctcaattcac atagatttct tctctttagt ataattgacc tactttggta 1920
gtggaatagt gaatacttac tataatttga cttgaatatg tagctcatcc tttacaccaa 1980
ctcctaattt taaataattt ctactctgtc ttaaatgaga agtacttggt tttttttttt 2040
cttaaatatg tatatgacat ttaaatgtaa cttattattt tttttgagac cgagtcttgc 2100
tctgttaccc aggctggagt gcagtggcgt gatcttggct cactgcaagc tctgcctccc 2160
gggttcgcac cattctcctg cctcagcctc ccaattagct tggcctacag tcatctgcca 2220
ccacacctgg ctaatttttt gtacttttag tagagacagg gtttcaccgt gttagccagg 2280
atggtctcga tctcctgacc tcgtgatccg cccacctcgg cctcccaaag tgctgggatt 2340
acaggcatga gccaccg 2357

<210> 93
<211> 4034
<212> RNA
<213> Homo sapiens

<400> 93

agggagaggc agagaggcag gcagcctgct gggctcttcc tgctgttgaa aacttacccg 60
gcccttacag aggaaatctt cctcctctct tctgccctga atgttttccc aaacatgaag 120
gtgataagct tattcatttt ggtgggattt ataggagagt tccaaagttt ttcaagtgcc 180
tcctctccag tcaactgcca gtgggacttc tatgcccctt ggtcagaatg caatggctgt 240
accaagactc agactcgcag gcggtcagtt gctgtgtatg ggcagtatgg aggccagcct 300
tgtgttggaa atgcttttga aacacagtcc tgtgaaccta caagaggatg tccaacagag 360
gagggatgtg gagagcgttt caggtgcttt tcaggtcagt gcatcagcaa atcattggtt 420
tgcaatgggg attctgactg tgatgaagac agtgctgatg aagacagatg tgaggactca 480
gaaaggagac cttcctgtga tatcgataaa cctcctccta acatagaact tactggaaat 540
ggttacaatg aactcactgg ccagtttagg aacagagtca tcaataccaa aagttttggt 600
ggtcaatgta gaaaggtgtt tagtggggat ggaaaagatt tctacaggct gagtggaaat 660
gtcctgtcct atacattcca ggtgaaaata aataatgatt ttaattatga attttacaat 720
agtacttggt cttatgtaaa acatacgtcg acagaacaca catcatctag tcggaagcgc 780
tccttttta gatcttcatc atcttcttca cgcagttata cttcacatac caatgaaatc 840
cataaaggaa agagttacca actgctggtt gttgagaaca ctgttgaagt ggctcagttc 900
attaataaca atccagaatt tttacaactt gctgagccat tctggaagga gctttcccac 960
ctcccctctc tgtatgacta cagtgcctac cgaagattaa tcgaccagta cgggacacat 1020
tatctgcaat ctgggtcgtt aggaggagaa tacagagttc tattttatgt ggactcagaa 1080
aaattaaaac aaaatgattt taattcagtc gaagaaaaga aatgtaaatc ctcaggttgg 1140
cattttgtcg ttaaattttc aagtcatgga tgcaaggaac tggaaaacgc tttaaaagct 1200
gcttcaggaa cccagaacaa tgtattgcga ggagaaccgt tcatcagagg gggaggtgca 1260
ggcttcatat ctggccttag ttacctagag ctggacaatc ctgctggaaa caaaaggcga 1320
tattctgcct gggcagaatc tgtgactaat cttcctcaag tcataaaaca aaagctgaca 1380
cctttatatg agctggtaaa ggaagtacct tgtgcctctg tgaaaaaact atacctgaaa 1440
tgggctcttg aagagtatct ggatgaattt gacccctgtc attgccggcc ttgtcaaaat 1500
ggtggtttgg ctactgttga ggggacccat tgtctgtgcc attgcaaacc gtacacattt 1560
ggtgcggcgt gtgagcaagg agtcctcgta gggaatcaag caggaggggt tgatggaggt 1620
tggagttgct ggtcctcttg gagcccctgt gtccaaggga agaaaacaag aagccgtgaa 1680
tgcaataacc cacctcccag tgggggtggg agatcctgcg ttggagaaac gacagaaagc 1740
acacaatgcg aagatgagga gctggagcac ttgaggttgc ttgaaccaca ttgctttcct 1800
ttgtctttgg ttccaacaga attctgtcca tcacctcctg ccttgaaaga tggatttgtt 1860
caagatgaag gtacaatgtt tcctgtgggg aaaaatgtag tgtacacttg caatgaagga 1920
tactctctta ttggaaaccc agtggccaga tgtggagaag atttacggtg gcttgttggg 1980

gaaatgcatt gtcagaaaat tgcctgtgtt ctacctgtac tgatggatgg catacagagt 2040
caccccccaaa aacctttcta cacagttggt gagaaggtga ctgtttcctg ttcaggtggc 2100
atgtccttag aaggtccttc agcatttctc tgtggctcca gccttaagtg gagtcctgag 2160
atgaagaatg cccgctgtgt acaaaaagaa aatccgttaa cacaggcagt gcctaaatgt 2220
cagcgctggg agaaactgca gaattcaaga tgtgtttgta aaatgcccta cgaatgtgga 2280
ccttccttgg atgtatgtgc tcaagatgag agaagcaaaa ggatactgcc tctgacagtt 2340
tgcaagatgc atgttctcca ctgtcagggt agaaattaca cccttactgg tagggacagc 2400
tgtactctgc ctgcctcagc tgagaaagct tgtggtgcct gcccactgtg gggaaaatgt 2460
gatgctgaga gcagcaaatg tgtctgccga gaagcatcgg agtgcgagga agaagggttt 2520
agcatttgtg tggaagtgaa cggcaaggag cagacgatgt ctgagtgtga ggcgggcgct 2580
ctgagatgca gagggcagag catctctgtc accagcataa ggccttgtgc tgcggaaacc 2640
cagtaggctc ctggaggccc tggtcagctt gcttggaatc cagcaggcag ctggggctga 2700
gtgaaaacat ctgcacaact gggcactgga cagcttttcc ttcttctcca gtgtctacct 2760
tcctcctcaa ctcccagcca tctgtataaa cacaatcctt tgttctccca aatctgaatc 2820
gaattactct tttgcctcct ttttaatgtc agtaaggata tgagcctttg cacaggctgg 2880
ctgcgtgttc ttgaaatagg tgttaccttc tctgggcctt ggttttttaa aatctgtaaa 2940
attagaggat tgcactagag aaacttgaat gctccattca ggcctatcat tttattaagt 3000
atgattgaca cagcccatgg gccagaacac actctacaaa atgactagga taacagaaag 3060
aacgtgatct cctgattaga gagggtggtt ttcctcaatg gaaccaaata taaagaggac 3120
ttgaacaaaa atgacagata caaactattt ctatcctgag tagtaatctc acacttcatc 3180
ctatagagtc aaccaccaca gataggaatt ccttattctt tttttaattt ttttaagaca 3240
gagtctcact ttgttgccca ggctggagcg cagtggggtg atctcatctc cctgcaacct 3300
ccgcctcctg ggttcaagcg attcttgtgc ctcagcttcc caagcagctg ggattacagg 3360
tgcccgccac cacgcccagc taatttttgc atttttagta gagatggggt ttcaccatgt 3420
tggccacgct cgtctccaac tcctgacctc aggtaatccg cctgccttgg cctcccaaag 3480
tgctgggatt acagacatga accaccacgc ctggctggaa tacttactct tgtcgggaga 3540
ttgaaccact aaaatgttag agcagaattc attatgctgt ggtcacaggg gtgtcttgtc 3600
tgagaacaaa tacaattcag tcttctcttt ggggttttag tatgtgtcaa acataggact 3660
ggaagtttgc ccctgttctt ttttcttttg aaagaacatc agttcatgcc tgaggcatga 3720
gtgactgtgc atttgagaat agttttccct attctgtgga tacagtccca gagttttcag 3780
ggagtacaca ggtagattag tttgaagcat tgacctttta tttattcctt atttctcttt 3840
catcaaaaca aaacagcagc tgtgggagga gaaatgagag ggcttaaatg aaatttaaaa 3900
taagctatat tatacaaata ctatctctgt attgttctga ccctggtaaa tatatttcaa 3960
aacttcagat gacaaggatt agaacactca ttaaagatgc tattcttcag aaaaaaaaaa 4020

aaaaaaaaaa aaaa 4034

<210> 94
<211> 2964
<212> RNA
<213> Homo sapiens

<400> 94

agtcggcggc ggctgctgct gcctgtggcc cgggcggctg ggagaagcgg agtgttggtg 60
agtgacgcgg cggaggtgta gtttgacgcg gtgtgttacg tgggggagag aataaaactc 120
cagcgagatc cgggccgtga acgaaagcag tgacggagga gcttgtacca ccggtaacta 180
aatgaccatg gaatctggag ccgagaacca gcagagtgga gatgcagctg taacagaagc 240
tgaaaaccaa caaatgacag ttcaagccca gccacagatt gccacattag cccaggtatc 300
tatgccagca gctcatgcaa catcatctgc tcccaccgta actctagtac agctgcccaa 360
tgggcagaca gttcaagtcc atggagtcat tcaggcggcc cagccatcag ttattcagtc 420
tccacaagtc caaacagttc agatttcaac tattgcagaa agtgaagatt cacaggagtc 480
agtggatagt gtaactgatt cccaaaagcg aagggaaatt ctttcaagga ggccttccta 540
caggaaaatt ttgaatgact tatcttctga tgcaccagga gtgccaagga ttgaagaaga 600
gaagtctgaa gaggagactt cagcacctgc catcaccact gtaacggtgc caactccaat 660
ttaccaaact agcagtggac agtatattgc cattacccag ggaggagcaa tacagctggc 720
taacaatggt accgatgggg tacagggcct gcaaacatta accatgacca atgcagcagc 780
cactcagccg ggtactacca ttctacagta tgcacagacc actgatggac agcagatctt 840
agtgcccagc aaccaagttg ttgttcaagc tgcctctgga gacgtacaaa cataccagat 900
tcgcacagca cccactagca ctattgcccc tggagttgtt atggcatcct ccccagcact 960
tcctacacag cctgctgaag aagcagcacg aaagagagag gtccgtctaa tgaagaacag 1020
ggaagcagct cgagagtgtc gtagaaagaa gaaagaatat gtgaaatgtt tagaaaacag 1080
agtggcagtg cttgaaaatc aaaacaagac attgattgag gagctaaaag cacttaagga 1140
cctttactgc cacaaatcag attaatttgg gatttaaatt ttcacctgtt aaggtggaaa 1200
atggactggc ttggccacaa cctgaaagac aaaataaaca ttttattttc taaacatttc 1260
tttttttcta tgcgcaaaac tgcctgaaag caactacaga atttcattca tttgtgcttt 1320
tgcattaaac tgtgaatgtt ccaacacctg cctccacttc tcccctcaag aaattttcaa 1380
cgccaggaat catgaagaga cttctgcttt tcaaccccca ccctcctcaa gaagtaataa 1440
tttgtttact tgtaaattga tgggagaaat gaggaaaaga aaatcttttt aaaaatgatt 1500
tcaaggtttg tgctgagctc cttgattgcc ttagggacag aattacccca gcctcttgag 1560
ctgaagtaat gtgtgggccg catgcataaa gtaagtaagg tgcaatgaag aagtgttgat 1620
tgccaaattg acatgttgtc acattctcat tgtgaattat gtaaagttgt taagagacat 1680
accctctaaa aaagaacttt agcatggtat tgaaggaatt agaaatgaat ttggagtgct 1740

ttttatgtat gttgtcttct tcaatactga aaatttgtcc ttggttctta aaagcattct 1800
gtactaatac agctcttcca tagggcagtt gttgcttctt aattcagttc tgtatgtgtt 1860
caacattttt gaatacatta aaagaagtaa ccaactgaac gacaaagcat ggtatttgaa 1920
ttttaaatta aagcaaagta aataaaagta caaagcatat tttagttagt actaaattct 1980
tagtaaaatg ctgatcagta aaccaatccc ttgagttata taacaagatt tttaaataaa 2040
tgttattgtc ctcaccttca aaaatattta tattgtcact catttacgta aaaagatatt 2100
tctaatttac tgttgcccat tgcacttaca taccaccacc aagaaagcct tcaagatgtc 2160
aaataaagca aagtgatata tatttgttta tgaaatgtta catgtagaaa aatactgatt 2220
ttaaatattt tccatattaa caatttaaca gagaatctct agtgaatttt ttaaatgaaa 2280
gaagttgtaa ggatataaaa agtacagtgt tagatgtgca caaggaaagt tattttcaga 2340
catatttgaa tgactgctgt actgcaatat ttggattgtc attcttacaa aacatttttt 2400
tgttctcttg taaaaagagt agttattagt tctgctttag ctttccaata tgctgtatag 2460
cctttgtcat tttataattt taattcctga ttaaaacagt ctgtatttgt gtatatcata 2520
cattgttttc aataccactt ttaattgtta ctcattttat tcactaagct cgataaatct 2580
aacagttact cttaaaaaaa aaaaaaagac taaggtggat tttaaaaatt ggaaactgac 2640
ataatgttag gttataattt ctcatttgga gccgggcgca gtggctcacg cctgtaatcc 2700
cagcactttg ggaggccaag gtgggtggat cacctgtggt caagagttca agaccagcct 2760
ggccatcatg gtgaaacccc atctctacta aaaatacaaa aattagccag gcgtggtggc 2820
tggcgcctgt aatcccagct actcaggagg ttgaggcagc agaattgctt gaacccagga 2880
ggcagagggt tgcagtgagc cgagatagca ccattgcact ccagcctggg cgactccatc 2940
tcaaaaaata aaaaaaaaaa aaaa 2964

<210> 95
<211> 1977
<212> RNA
<213> Homo sapiens

<400> 95

```
gttttggcag gagcgggaga attctgcgga gcctgcggga cggcggcggt ggcgccgtag    60
gcagccggga cagtgttgta cagtgttttg ggcatgcacg tgatactcac acagtggctt   120
ctgctcacca acagatgaag acagatgcac caacgaggct gatgggaacc atcctgtaga   180
ggtccatctg cgttcagacc cagacgatgc cagagctatg actgggcctg caggtgtggc   240
gccgagggga gatcagccat ggagcagcca caggaggaag cccctgaggt ccgggaagag   300
gaggagaaag aggaagtggc agaggcagaa ggagccccag agctcaatgg gggaccacag   360
catgcacttc cttccagcag ctacacagac ctctcccgga gctcctcgcc accctcactg   420
ctggaccaac tgcagatggg ctgtgacggg gcctcatgcg gcagcctcaa catggagtgc   480
cgggtgtgcg gggacaaggc atcgggcttc cactacggtg ttcatgcatg tgaggggtgc   540
aagggcttct tccgtcgtac gatccgcatg aagctggagt acgagaagtg tgagcgcagc   600

tgcaagattc agaagaagaa ccgcaacaag tgccagtact gccgcttcca gaagtgcctg   660
gcactgggca tgtcacacaa cgctatccgt tttggtcgga tgccggaggc tgagaagagg   720
aagctggtgg cagggctgac tgcaaatgag gggagccagt acaacccaca ggtggccgac   780
ctgaaggcct tctccaagca catctacaat gcctacctga aaaacttcaa catgaccaaa   840
aagaaggccc gcagcatcct caccggcaaa gccagccaca cggcgccctt tgtgatccac   900
gacatcgaga cattgtggca ggcagagaag gggctggtgt ggaagcagtt ggtgaatggc   960
ctgcctccct acaaggagat cagcgtgcac gtcttctacc gctgccagtg caccacagtg  1020
gagaccgtgc gggagctcac tgagttcgcc aagagcatcc ccagcttcag cagcctcttc  1080
ctcaacgacc aggttaccct tctcaagtat ggcgtgcacg aggccatctt cgccatgctg  1140
gcctctatcg tcaacaagga cgggctgctg gtagccaacg gcagtggctt tgtcacccgt  1200
gagttcctgc gcagcctccg caaacccttc agtgatatca ttgagcctaa gtttgaattt  1260
gctgtcaagt tcaacgccct ggaacttgat gacagtgacc tggccctatt cattgcggcc  1320
atcattctgt gtggaggtga gtgagagtgg ggcaggtggg ctggcctggc acacccagtc  1380
gtcctggggg ttggccctca ctgcagggca ctgtgcctga gctctgacag tgtggggaag  1440
tgtccctgtg atcttggcag tggaacatgc aaggcactga ctgagcatgc aggatcagct  1500
ccatctcatt atgtacgtag atagaggtgg agacaggaaa aagactaagc cagacgtggt  1560
ggctcacacc tgtaatccca gcactttggc aggccgaggc gggtggatca cttgaggtca  1620
ggagttcgaa accagcctgg ccaacatggt gaaaccccgt ctctactaaa aatacaaaaa  1680
attagccaga tgtggtggca cgcgcctgta atcccagcta cttgggaggc tgagccagga  1740
gaatcgcttg aacccgagag gtggaggttg cagtgagcca aaatcccacc actgcactcc  1800
agcctgggtg acagagtgag accctgtctc aaaaaaaagg aaaaggacta acaggcagta  1860
tgctgtcatg ttaatgtggg gtggaaaaat tgtctgcatt ttttctgcat ttttaaaatt  1920
ccaacacaat aaatacaata ataactatgc taaaaaaaaa aaaaaaaaaa aaaaaaa     1977
```

<210> 96

<211> 2594
<212> RNA
<213> Homo sapiens

<400> 96

```
gcttcgggtg ccatggggac tcctcccggc ctgcagaccg actgcgaggc gctgctcagc      60
cgcttccagg agacggacag tgtacgcttc gaggacttca cggagctctg gagaaacatg     120
aagttcggga ctatcttctg tggcagaatg agaaatttag aaaagaacat gtttacaaaa     180
gaagctttag ctttggcttg gcgatatttt ttacctccat acaccttcca gatcagagtt     240
ggtgctttgt atctgctata tggattatat aatacccaac tgtgtcaacc aaaacaaaag     300
atcagagttg ccctgaagga ttgggatgaa gttttaaaat ttcagcaaga tttagtaaat     360
gcacagcatt ttgatgcagc ttatattttt aggaagctac gactagacag agcatttcac     420
```

```
tttacagcaa tgcccaaatt gctgtcatat aggatgaaga aaaaaattca ccgagctgaa    480
gttacagaag aatttaagga cccaagtgat cgtgtgatga aacttatcac ttctgatgta    540
ttagaggaaa tgctgaatgt tcatgatcat tatcagaaca tgaaacatgt aatttcagtt    600
gataagtcca agccagataa agccctcagc ttgataaagg atgatttttt tgacaatatt    660
aagaacatag ttttggagca tcagcagtgg cacaaagaca gaaagaatcc atccttaaag    720
tcaaaaacta atgatggaga agaaaaaatg gaaggaaatt cacaagaaac ggagagatgt    780
gaaagggcag aatcattagc gaaaataaaa tcaaaggcct tttcagttgt catacaggca    840
tccaaatcaa gaaggcatcg tcaagtcaaa ctcgactctt ctgactctga ttctgcatct    900
ggtcaagggc aagtcaaagc aactaggaaa aaagagaaga aagaaagatt gaaaccagca    960
ggaaggaaga tgtctctcag aaacaaaggc aatgtgcaga atatacacaa ggaagataaa   1020
cctttaagtc tgagtatgcc tgtaattaca gaagaagaag agaatgaaag tttgagtgga   1080
acagagttca ctgcatccaa gaagaggaga aaacactgaa caaagagcct ggtgtagttt   1140
ttaattttga gttttctgac agaagaaaag attgatattt tgtgtattga acaggaagac   1200
tgccagtatt aaaaaaatcc ttctgggaat ctgtaggtta tttcttggaa attgcaatac   1260
gtagttctag aataaaagta caaaaaatta gaataagaat tctttaacat tttctttaat   1320
gatttgcata aatggagata aaacttgtat ttagtatgta atagaaaaaa ttctgttatt   1380
cgcagattgt tactatttcc tataaggttt tgtgatacta tactgtccta atacagtctg   1440
gtaatactat tctattttat ttaaaatatt ttttattgaa atattaatgt ttattacatg   1500
caaataacta ttttgtatct acagtcggat aatggatttt ttattttgta tatttattct   1560
attttgtata ttgttaagtg caataaagtt tttgccttgc tttatttttt aatacataaa   1620
acttacattc tcataacgtg attgataact taggaagttc acaatgtatt ttctacttct   1680
gcaattaaat attctttagt gcttgtttat tattactaaa tactaattaa gtactaacaa   1740
gtacttaaat actaatgtat taagtattta agtactttct aataaaatct ttaacaataa   1800
taatgtaaat ttcagaatgt gtctctggta cagaatagtt gatattaaca gaaaaaaaaa   1860
aatctgtagc ttcatgaata tgccactctg ttaatttctt gttccagaca ttttaataga   1920
gattgcttga gccatgttgt ttgaattgct gccaatagca gaccatatcc ctatcatgtt   1980
gttggctcaa ctgttttttt tttttcccta atagagatgg agtatcgcta tgttgctcag   2040
gctggtcttg aactcctggg ctcaagctat cctcctgcct cagcctccca aagtactggg   2100
attataggtg tgagctactg tacccagcct taacctgttt cacagttgat tatacttcat   2160
gctgttttcc agcatggtat tattaaggga tttaaagttt gggttgcatg cctgtaatcc   2220
cagcattttg ggaggccgag gtgggcggat cacgaggtca ggagatcgag accatcgtga   2280
ctaacacagt gaaaccccgt ctctaataaa aatacgaaaa attagccagg cgtggtggcg   2340
ggcgcctgta atcccagcta ctcgggaagc tgaggcagga gaatggtgtg aacccagtga   2400
gccgagatcg tgccactgca ctccagcctg ggcaacagag tgagacttcg tctcaaaaaa   2460
```

aaaaaaaaaa gtttgggttg aagatcaaat tcgtgatatc tctatatcta atctttaaaa 2520
atcagaatgc taatgctgac gcaaataaaa ttttcattta ttagcaaaaa aaaaaaaaaa 2580
aaaaaaaaaa aaaa 2594

<210> 97
<211> 273
<212> RNA
<213> Homo sapiens

<400> 97

tttttttttt tttttttttt gggacggagt tcgctctgtc gcccaggctg gagcgcactg 60
gtgcaatctc agcttgctac accctctacc tcccgggtgt caccatgttg gccaggctgg 120
ttttgaactt ctgactcaag tgatctgcac acctcagcct ttaaagtgct aggattacaa 180
gcatgagcca ccacacctgc tccttctatt tcattttaac ataaataagt aatagtagct 240
aagacttact aagcactatg tattagacag ttt 273

<210> 98
<211> 5059
<212> DNA
<213> Homo sapiens

<400> 98

```
ctggttctca acttcttttg aaataatgtt catagagaag gagggctgtc tgagattcga     60
gggaaacaag ctctcaggac ttccggtcgc catgatggct gtgggcggta aacgcggtta    120
gtgcaagcat ctgggccatc ttcaatggta aaaaagatac agtaaagaca taaataccac    180
atttgacaaa tggaaaaaaa ggagtgtcca gaaaagagta gcagcagtga ggaagagctg    240
ccgagacggg tatacaggga gctaccctgt gtttctgaga ccctttgtga catctcacat    300
tttttccaag aagatgatga gacagaggca gagccattat tgttccgtgc tgttcctgag    360
tgtcaactat ctgggggga cattcccagg agacatttgc tcagaagaga atcaaatagt    420
ttcctcttat gcttctaaag tctgttttga gatcgaagaa gattataaaa atcgtcagtt    480
tctggggcct gaaggaaatg tggatgttga gttgattgat aagagcacaa acagatacag    540
cgtttggttc cccactgctg gctggtatct gtggtcagcc acaggcctcg gcttcctggt    600
aagggatgag gtcacagtga cgattgcgtt tggttcctgg agtcagcacc tggccctgga    660
cctgcagcac catgaacagt ggctggtggg cggccccttg tttgatgtca ctgcagagcc    720
agaggaggct gtcgccgaaa tccacctccc ccacttcatc tccctccaag gtgaggtgga    780
cgtctcctgg tttctcgttg cccattttaa gaatgaaggg atggtcctgg agcatccagc    840
ccgggtggag cctttctatg ctgtcctgga aagccccagc ttctctctga tgggcatcct    900
gctgcggatc gccagtggga ctcgcctctc catccccatc acttccaaca cattgatcta    960
ttatcacccc caccccgaag atattaagtt ccacttgtac cttgtcccca gcgacgcctt   1020
gctaacaaag gcgatagatg atgaggaaga tcgcttccat ggtgtgcgcc tgcagacttc   1080
gcccccaatg gaacccctga actttggttc cagttatatt gtgtctaatt ctgctaacct   1140
```

gaaagtaatg cccaaggagt tgaaattgtc ctacaggagc cctggagaaa ttcagcactt 1200
ctcaaaattc tatgctgggc agatgaagga acccattcaa cttgagatta ctgaaaaaag 1260
acatgggact ttggtgtggg atactgaggt gaagccagtg gatctccagc ttgtagctgc 1320
atcagcccct cctcctttct caggtgcagc ctttgtgaag gagaaccacc ggcaactcca 1380
agccaggatg ggggacctga aaggggtgct cgatgatctc caggacaatg aggttcttac 1440
tgagaatgag aaggagctgg tggagcagga aaagacacgg cagagcaaga atgaggcctt 1500
gctgagcatg gtggagaaga aaggggacct ggccctggac gtgctcttca gaagcattag 1560
tgaaagggac ccttacctcg tgtcctatct tagacagcag aatttgtaaa atgagtcagt 1620
taggtagtct ggaagagaga atccagcgtt ctcattggaa atggataaac agaaatgtga 1680
tcattgattt cagtgttcaa gacagaagaa gactgggtaa catctatcac acaggctttc 1740
aggacagact tgtaacctgg catgtaccta ttgactgtat cctcatgcat tttcctcaag 1800
aatgtctgaa gaaggtagta atattccttt taaatttttt ccaaccattg cttgatatat 1860
cactatttta tccattgaca tgattcttga agacccagga taaaggacat ccggataggt 1920
gtgtttatga aggatggggc ctggaaaggc aacttttcct gattaatgtg aaaaataatt 1980
cctatggaca ctccgtttga agtatcacct tctcataact aaaagcagaa aagctaacaa 2040
aagcttctca gctgaggaca ctcaaggcat acatgatgac agtctttttt tttttgtat 2100
gttaggactt taacacttta tctatggcta ctgttattag aacaatgtaa atgtatttgc 2160
tgaaagagag cacaaaaatg ggagaaaatg caaacatgag cagaaaatat tttcccactg 2220
gtgtgtagcc tgctacaagg agttgttggg ttaaatgttc atggtcaact ccaaggaata 2280
ctgagatgaa atgtggtaaa tcaactccac agaaccacca aaaagaaaat gagggtaatt 2340
cagcttattc tgagacagac attcctggca atgtaccata caaaaaataa gccaactctg 2400
acatttggat tctaccatag actctgtcat tttgtagcca tttcagctgt cttttgatta 2460
atgttttcgt ggcacacata tttccatcct tttatgttta atctgtttaa aacaagttcc 2520
tagtagacac catctggttg agtcagtttt ttttatggtg tattttgaac ccattctgat 2580
agtctctttt aactggaaga tttcaattac ttacgttaat gtaattatta atatgttagg 2640
atttatcctc agtcagccag tttgttatgt cttttctatt ctactgttat cacatttgta 2700
ccacttaaag tggaatctag gcactttatc accatttaga tcctattacc ttttctcatc 2760
taggatatag ttatcttcta cataatcttt ctgtatctta aaacccatca ataaattatt 2820
atatattttc tacttttaat cactcagaag atttaaaaaa ctcatgagaa gagtaatctg 2880
ttatgttttt ccagatattt accatttctg ttgctcttcc ttcattattt tccaaatttc 2940
gttctgcaaa tttccacttc ttctgataga cgtttttag ttcttttaga gtggttctga 3000
taggtacaga ttctcttatt ttttgcttcc tctgaggaca tctttttctc accttcattc 3060
tcagtgatgt tttttgcttg tagtattttt agttgacatt gttttctgtt cagcagtttc 3120
cttttagctt ccgtatttcc tgatgagaaa tctgcagtca ttcaaattgt tgtttccctg 3180

tatgtagtgt gtcatttttc tgtcagattt caaggtattt atctttagtt tttagccatt 3240
tcattatgtt ggggatgagt ttccttgttt tattcccttt ggaatttgct ccaattcata 3300
aatttgcagt tttatgtctt ttaccaaact tagaggtttt cagcctaatt tctaaaaata 3360
ctttttatta gcctgatttt catctttata ggaaatagtt taagtgatga caagttccaa 3420
tagcttatat gcccagaagg ccttcaaaat aagaattttg aaagaataca gaaaacaaac 3480
ttttatatcc ttctcatgtc ttctactgta aaattcatat gctttgctac tctaaaccta 3540
gtttgaaatc aacagtcttg agaatagatg aaaattttga tgaatagtgg aattctttta 3600
aatggaaacc tcttacatgt gattttcctt gccatctaga aataaaccat agtatttatg 3660
ttgaatcaat caatattata ttttgttttt ttcctcctct tctgagactc ttattgtgga 3720
aatgttagac ttttatgttt tcctaaatgt ccctgatatt ctacttattt agaacatctt 3780
ttcatttttt ccattattct gattgggtaa ttttaatttg tctattttca aatttgctgg 3840
agtgttcacc tgttgttgtc tgtgtcgtcc cactgagtgc attcaccacc ttttaaattt 3900
tggtcactgt atgtatcagt tctaaaattt ccattttgtt ctctatattt taaatttctt 3960
ggcttatatt ctattttcct gcaaatgtgt cagcatttgc ttgtttgagc tttttttttt 4020
tcaagacagg gtctcaactc tgttacccag gctggagtgc agtggtgcga tctcagctca 4080
ctgcaacctc tgcctcctgg ttcaagcgat tattgtgcct cagcctcctg agtagctggg 4140
attacaggca tgcaccacca cagcccagct aatttttgt attttagta gagacagagt 4200
tttgctatgt tggccaggct ggttttgaac tcctggcctc aagtgatcca cccacctcag 4260
cctcccaaag tgctgggatt acaggccact acacctggca catttgagta ttttttttt 4320
tttttttttt ttgagatgga gtctcgctct gtcatctagg ctggagtgca gtggtgtgat 4380
ctcagctcac tgcagcctct gtctcccggg ctcaagcgat tctcttgcct cagcctcctg 4440
agtagctagg actacaggtg catgccaaca cgcccggcta atttttttaa aaaatatttt 4500
tagtagagac agggtttcac cattttggcc aggatggtct cgatctcctg acctcatgat 4560
ccacccgcct cggccttcca aagtgctggg attacaggca tgagccaccg tgcctggcct 4620
catttgagta tttttataat gtctctttta aagtctttgt cagataattc cactgtacat 4680
gttattcagt gtttggtgtc cactgagttg tcatttgcca gacaagtgga gatttttgca 4740
gctcatcctt gtattctcag tagttccgat atgtaccctc gacatgtgaa tgttatctta 4800
tgagactctg ttttatttgt atccaacaga agatgtttat tatttatttg gctttctgtg 4860
aactgaggtc ttaatatcag ctcattttaa aagtctttgc agtggtattc ggatctatcc 4920
tgtgtgtgcc tatgagattg ggtgcagtgt atcctgttag ctccattctc agggcgtttg 4980
aatgtgaatt aggaccagcg caatgaatgc tcaagttggg gttgggcgtt agaattcata 5040
aaagtcttta tatgctcag 5059

<210> 99
<211> 2962
<212> DNA
<213> Homo sapiens

<400> 99

```
ggatcctttc tggaatggag gtcttatgag ctgctattga acacggcaga gcctgttggt      60
gacctgcaca caggagccct ccagtcagta ctgattgaat tactcaaggc tgcctctctg     120
caaagttgag cactacagga cgtcgggact gggcatttcc ttccaacatg gccgccactg     180
cctctccgca gccactcgcc actgaggatg ccgattctga gaatagcagc ttctattact     240
atgactacct ggatgaagtg gccttcatgc tctgcaggaa ggatgcagtg gtgtcctttg     300
gcaaagtctt cctcccagtc ttctatagcc tgatttttgt gttgggcctc agcgggaacc     360
tccttcttct catggtcttg ctccgttacg tgcctcgcag gcggatggtt gagatctatc     420
tgctgaatct ggccatctcc aaccttctgt ttctggtgac actgcccttc tggggcatct     480
ccgtggcctg gcattgggtc ttcgggagtt tcttgtgcaa gatggtgagc actctttata     540
ctattaactt ttacagtggc atctttttca ttagctgcat gagcctggac aagtacctgg     600
agatcgttca tgctcagccc taccacaggc tgaggacccg ggccaagagc ctgctccttg     660
ctaccatagt atgggctgtg tccctggccg tctccatccc tgatatggtc tttgtacaga     720
cacatgaaaa tcccaagggt gtgtggaact gccacgcaga tttcggcggg catgggacca     780
tttggaagct cttcctccgc ttccagcaga acctcctagg gtttctcctt ccactccttg     840
ccatgatctt cttctactcc cgtattggtt gtgtcttggt gaggctgagg cccgcaggcc     900
agggccgggc tttaaaaata gctgcagcct tggtggtggc cttcttcgtg ctatggttcc     960
catacaatct caccttgttt ctgcatacgc tgttggacct gcaagtattc gggaactgtg    1020
aggtcagcca gcatctagac tacgcactcc aggtaacaga gagcatcgcc ttccttcact    1080
gctgcttttc cccccatcctg tatgccttct ccagtcaccg cttccgccag tacctgaagg    1140
ctttcctggc tgccgtgctt ggatggcacc tggcacctgg cactgcccag gcctcattat    1200
ccagctgttc tgagagcagc atacttactg cccaagagga aatgactggc atgaatgacc    1260
ttggagagag gcagtctgag aactacccta acaaggagga tgtggggaat aaatcagcct    1320
gagtgaccaa attttggtct ggtgggaaca gatgggaacc agctcaattg ggtgtccact    1380
caaagtgctc tctccagggg cctcagtgac tgtgttgcta aacccagtgg tcagttctca    1440
gttctcagcc atcagcagca tttgctcgcc ccgccttctt cctccacttt cttcacttgc    1500
ttccaggata ccacgctttc ttttctgaat tgctacaatc tttcttcctt ccttccttgc    1560
ttccttcctt ccttccttcc ctctctccct ccctccctcc ctcgcttctt cccttcctcc    1620
tttcctccct tcctactttc cttccttcct tctgacaggg tcttgctcta ttgctctgtc    1680
acccaggctg gaatgcagtg gcgagatctc cgctcactgt agcctcctcc ccctgggttg    1740
aagcaattct catgcctcag cctcccaagt agccaggact ataggcacct gccaccatgc    1800
ctggctaatt tttgtatttt ttttctttct ttctttcttt tctttttttt ttttttttga    1860
gacggagtct cactcttgtt gcccaggctg gacaacaatg gcgcgatctc ggctcactgc    1920
aacctccacc tcccggattc aagcgattct cctgcctcag cctcctgagt agctggaact    1980
```

acatgcgcgt gccaccacgc acagctaatt tttataattt tagtagagat ggggtttcac 2040
tgcgttggcc aggatgatct cgatctcttg accttgggat ccacccgcct tggcctccca 2100
aagtgctggg attacaggtg tgagccacca tgcctggccc taatttttgt gtttttatta 2160
gaaacagagt ttcaccatgt tggccaggct ggagaattgc tgtaatagtt ttccaactgg 2220
cccctgtcct tcctctctct tgctctcctc ccatctcatc tgcacctagc agccagagtg 2280
atcctgatac tctcggcctt tacttccgcc tccctcagag cagcagcctg tcaaaacacc 2340
agattacaac aaatttagtt taaaggtctc aattagcgtt attggcaatt ctagaatcag 2400
gcaacagact cattgaatca ggaacagatt cactccataa aatacagaga gtgctgcaat 2460
gagctgggta gaagaggtta gttttataga caggaagggg ctgtcaaagg cagaaagaaa 2520
tgaagaacaa aaaaaaagat tgattttttt ttttttgaga caggatctca ctctgtcatc 2580
caggctgaag tccaatccca caatcatggc tcactgcagc caccacctcc tgagctcaag 2640
tgatcctccc atctaagccc ccaagtagct aggactacag gagcacacca ccacacctgg 2700
ctaatttttg tatttttttgt ggagacaggg tctcagtatg ttacccaggt tggactggaa 2760
acccttggct caagcaattt gcctgcctca gcctcccaaa gtgctgggat tacaggcgtg 2820
agccactgca cagggccaga ttcatcattt caaagttact ttctatatgc ggccggaaca 2880
gggtggttga catcagtttt cttcaggtta ctttttaata atgattaaaa cggggaactt 2940
cattatcaaa aaaaaaaaaa aa 2962

<210> 100
<211> 562
<212> DNA
<213> Homo sapiens

<400> 100

ctggaattga ggctgagcca aagaccccag ggccgtctca gtctcataaa aggggatcag 60
gcaggaggag tttgggagaa acctgagaag ggcctgattt gcagcatcat gatgggcctc 120
tccttggcct ctgctgtgct cctggcctcc ctcctgagtc tccaccttgg aactgccaca 180
cgtgggagtg acatatccaa gacctgctgc ttccaataca gccacaagcc ccttccctgg 240
acctgggtgc gaagctatga attcaccagt aacagctgct cccagcgggc tgtgatattc 300
actaccaaaa gaggcaagaa agtctgtacc catccaagga aaaaatgggt gcaaaaatac 360
atttctttac tgaaaactcc gaaacaattg tgactcagct gaattttcat ccgaggacgc 420
ttggaccccg ctcttggctc tgcagccctc tggggagcct gcggaatctt ttctgaaggc 480
tacatggacc cgctggggag gagagggtgt ttcctcccag agttacttta ataaaggttg 540
ttcatagagt tgacttgttc at 562

<210> 101
<211> 1873
<212> DNA
<213> Homo sapiens

<400> 101

gacgatacgc cgggcgcagg cgcagaagcc gcgcccgtcc gcggcgccgc cagccagggc 60
ggaaacggct gcggcttcgc tagggacgca tgcgcgggtc ccttagtttt cgcgagataa 120
cggtcgaaaa cgcgctcttg tcgatttcct gtagtgaatc aggcaccgga gtgcaggttc 180
gggggtggaa tccttgggcc gctgggcaag cggcgagacc tggccagggc cagcgagccg 240
aggacagagg gcgcacggag ggccgggccg cagccccggc cgcttgcaga ccccgccatg 300
gacccgttcc tggtgctgct gcactcggtg tcgtccagcc tgtcgagcag cgagctgacc 360
gagctcaagt tcctatgcct cgggcgcgtg ggcaagcgca agctggagcg cgtgcagagc 420
ggcctagacc tcttctccat gctgctggag cagaacgacc tggagcccgg gcacaccgag 480
ctcctgcgcg agctgctcgc ctccctgcgg cgccacgacc tgctgcggcg cgtcgacgac 540
ttcgaggcgg gggcggcggc cggggccgcg cctggggaag aagacctgtg tgcagcattt 600
aacgtcatat gtgataatgt ggggaaagat tggagaaggc tggctcgtca gctcaaagtc 660
tcagacacca agatcgacag catcgaggac agataccccc gcaacctgac agagcgtgtg 720
cgggagtcac tgagaatctg gaagaacaca gagaaggaga acgcaacagt ggcccacctg 780
gtgggggctc tcaggtcctg ccagatgaac ctggtggctg acctggtaca agaggttcag 840
caggcccgtg acctccagaa caggagtggg gccatgtccc cgatgtcatg gaactcagac 900
gcatctacct ccgaagcgtc ctgatgggcc gctgctttgc gctggtggac cacaggcatc 960
tacacagcct ggactttggt tctctccagg aaggtagccc agcactgtga agacccagca 1020
ggaagccagg ctgagtgagc cacagaccac ctgcttctga actcaagctg cgtttattaa 1080
tgcctctccc gcaccaggcc gggcttgggc cctgcacaga tatttccatt tcttcctcac 1140
tatgacactg agcaagatct tgtctccact aaatgagctc ctgcgggagt agttggaaag 1200
ttggaaccgt gtccagcaca gaaggaatct gtgcagatga gcagtcacac tgttactcca 1260
cagcggagga gaccagctca gaggcccagg aatcggagcg aagcagagag gtggagaact 1320
gggatttgaa cccccgccat ccttcaccag agcccatgct caaccactgt ggcgttctgc 1380
tgcccctgca gttggcagaa aggatgtttt gtcccatttc cttggaggcc accgggacag 1440
acctggacac tagggtcagg cggggtgcgt ggtggggaga ggcatggctg gggtgggggt 1500
ggggagacct ggttggccgt ggtccagctc ttggcccctg tgtgagttga gtctcctctc 1560
tgagactgct aagtaggggc agtgatggtt gccaggacga attgagataa tatctgtgag 1620
gtgctgatga gtgattgaca cacagcactc tctaaatctt ccttgtgagg attatgggtc 1680
ctgcaattct acagtttctt actgttttgt atcaaaatca ctatctttct gataacagaa 1740
ttgccaaggc agcgggatct cgtatcttta aaaagcagtc ctcttattcc taaggtaatc 1800
ctattaaaac acagctttac aacttccata tcacaaaaaa aaaaaaaaaa aaaaaaaaaa 1860
aaaaaaaaaa aaa 1873

<210> 102
<211> 4082

<212> DNA
<213> Homo sapiens

<400> 102

```
ggcggtcccc tgttctcccc gctcaggtgc ggcgctgtgg caggaagcca ccccctcggt     60
cggccggtgc gcggggctgt tgcgccatcc gctccggctt tcgtaaccgc accctgggac    120
ggcccagaga cgctccagcg cgagttcctc aaatgttttc ctgcgttgcc aggaccgtcc    180
gccgctctga gtcatgtgcg agtgggaagt cgcactgaca ctgagccggg ccagagggag    240
aggagccgag cgcggcgcgg ggccgaggga ctcgcagtgt gtgtagagag ccgggctcct    300
gcggatgggg gctgcccccg gggcctgagc ccgcctgccc gcccaccgcc ccgccccgcc    360
cctgccaccc ctgccgcccg gttcccatta gcctgtccgc ctctgcggga ccatggagtg    420
gtagccgagg aggaagcatg ctggccgtcg gctgcgcgct gctggctgcc ctgctggccg    480
cgccgggagc ggcgctggcc ccaaggcgct gccctgcgca ggaggtggcg agaggcgtgc    540
tgaccagtct gccaggagac agcgtgactc tgacctgccc gggggtagag ccggaagaca    600
atgccactgt tcactgggtg ctcaggaagc cggctgcagg ctcccacccc agcagatggg    660
ctggcatggg aaggaggctg ctgctgaggt cggtgcagct ccacgactct ggaaactatt    720
catgctaccg ggccggccgc ccagctggga ctgtgcactt gctggtggat gttccccccg    780
aggagcccca gctctcctgc ttccggaaga gccccctcag caatgttgtt tgtgagtggg    840
gtcctcggag caccccatcc ctgacgacaa aggctgtgct cttggtgagg aagtttcaga    900
acagtccggc cgaagacttc caggagccgt gccagtattc ccaggagtcc cagaagttct    960
cctgccagtt agcagtcccg gagggagaca gctctttcta catagtgtcc atgtgcgtcg   1020
ccagtagtgt cgggagcaag ttcagcaaaa ctcaaacctt tcagggttgt ggaatcttgc   1080
agcctgatcc gcctgccaac atcacagtca ctgccgtggc cagaaacccc cgctggctca   1140
gtgtcacctg gcaagacccc cactcctgga actcatcttt ctacagacta cggtttgagc   1200
tcagatatcg ggctgaacgg tcaaagacat tcacaacatg gatggtcaag gacctccagc   1260
atcactgtgt catccacgac gcctggagcg gcctgaggca cgtggtgcag cttcgtgccc   1320
aggaggagtt cgggcaaggc gagtggagcg agtggagccc ggaggccatg ggcacgcctt   1380
ggacagaatc caggagtcct ccagctgaga acgaggtgtc caccccccatg caggcactta   1440
ctactaataa agacgatgat aatattctct tcagagattc tgcaaatgcg acaagcctcc   1500
caggttcaag aagacgtgga agctgcgggc tctgaaggaa ggcaagacaa gcatgcatcc   1560
gccgtactct ttggggcagc tggtcccgga gaggcctcga cccaccccag tgcttgttcc   1620
tctcatctcc ccaccggtgt cccccagcag cctggggtct gacaatacct cgagccacaa   1680
ccgaccagat gccagggacc cacggagccc ttatgacatc agcaatacag actacttctt   1740
ccccagatag ctggctgggt ggcaccagca gcctggaccc tgtggatgat aaaacacaaa   1800
cgggctcagc aaaagatgct tctcactgcc atgccagctt atctcagggg tgtgcggcct   1860
ttggcttcac ggaagagcct tgcggaaggt tctacgccag ggaaaaatca gcctgctcca   1920
```

gctgttcagc tggttgaggt ttcaaacctc cctttccaaa tgcccagctt aaaggggcta 1980
gagtgaactt gggccactgt gaagagaacc atatcaagac tctttggaca ctcacacgga 2040
cactcaaaag ctgggcaggt tggtgggggc ctcggtgtgg agaagcggct ggcagcccac 2100
ccctcaacac ctctgcacaa gctgcaccct caggcaggtg ggatggattt ccagccaaag 2160
cctcctccag ccgccatgct cctggcccac tgcatcgttt catcttccaa ctcaaactct 2220
taaaacccaa gtgccttagc aaattctgtt tttctaggcc tggggacggc ttttacttaa 2280
accgccaagg ctgggggaag aagctctctc ctccctttct tccctacagt tgaaaaacag 2340
ctgagggtga gtgggtgaat aatacagtat ctcagggcct ggtcgttttc aacagaatta 2400
taattagttc ctcattagca ttttgctaaa tgtgaatgat gatcctaggc atttgctgaa 2460
tacagaggca actgcattgg ctttgggttg caggacctca ggtgagaagc agaggaagga 2520
gaggagaggg gcacagggtc tctaccatcc cctgtagagt gggagctgag tgggggatca 2580
cagcctctga aaaccaatgt tctctcttct ccacctccca caaaggagag ctagcagcag 2640
ggagggcttc tgccatttct gagatcaaaa cggttttact gcagctttgt ttgttgtcag 2700
ctgaacctgg gtaactaggg aagataatat taaggaagac aatgtgaaaa gaaaaatgag 2760
cctggcaaga atgtgtttaa acttggtttt taaaaaactg ctgactgttt tctcttgaga 2820
gggtggaata tccaatattc gctgtgtcag catagaagta acttacttag gtgtggggga 2880
agcaccataa ctttgtttag cccaaaacca agtcaagtga aaaaggagga agagaaaaaa 2940
tattttcctg ccaggcatgg tggcccacgc acttcgggag gtcgaggcag gaggatcact 3000
tgagtccaga agtttgagat cagcctgggc aatgtgataa aaccccatct ctacaaaaag 3060
cataaaaatt agccaagtgt ggtagagtgt gcctgaagtc ccagatactt ggggggctga 3120
ggtgggagga tctcttgagc ctgggaggtc aaggctgcag tgagccgaga ttgcaccact 3180
gcactccagc ctgggtgaca gagcaagtga gaccctgtct caaaaaaaga aaaagaaaaa 3240
gaaaaaatat tttccctatt agagaagaga ttgtggtttc attctgtatt ttgtttttgt 3300
cttaaaaagt ggaaaaatag cctgcctctt ctctactcta gggaaaaacc agcgtgtgac 3360
tactccccca ggtggttatg gagagggtgt ccggtccctg tcccagtgcc gagaaggaag 3420
cctcccacga ctgcccggca gggtcctaga aattccccac cctgaaagcc ctgagctttc 3480
tgctatcaaa gaggttttaa aaaaatccca tttaaaaaaa atcccttacc tcggtgcctt 3540
cctcttttta tttagttcct tgagttgatt cagctctgca agaattgaag caggactaaa 3600
tgtctagttg taacaccatg attaaccact tcagctgact tttctgtccg agctttgaaa 3660
attcagtggt gttagtggtt acccagttag ctctcaagtt atcagggtat tccagagtgg 3720
ggatatgatt taaatcagcc gtgtaaccat ggacccaata tttaccagac cacaaaactt 3780
ttctaatact ctaccctctt agaaaaacca ccaccatcac cagacaggtg cgaaaggatg 3840
aaagtgacca tgttttgttt acggttttcc aggtttaagc tgttactgtc ttcagtaagc 3900
cgtgattttc attgctgggc ttgtctgtag attttagacc ctattgctgc ttgaggcaac 3960

tcatcttagg ttggcaaaaa ggcaggatgg ccgggcgcgg tggctcacgc ctgtaatcct 4020
agcactttgg gaggccaagg tgggaggatt gcttgagctc aggagtttga gaccaacctg 4080
gg 4082

<210> 103
<211> 2887
<212> DNA
<213> Homo sapiens

<400> 103

ggagctgaga ggaacaggaa gtgtcaggac tttacgaccc gcgcctccag ctgaggtttc 60
tagacgtgac ccagggcaga ctggtagcaa agcccccacg cccagccagg agcaccgccg 120
aggactccag cacaccgagg gacatgctgg gcctgcgccc cccactgctc gccctggtgg 180
ggctgctctc cctcgggtgc gtcctctctc aggagtgcac gaagttcaag gtcagcagct 240
gccgggaatg catcgagtcg gggcccggct gcacctggtg ccagaagctg aacttcacag 300
ggccggggga tcctgactcc attcgctgcg acacccggcc acagctgctc atgaggggct 360
gtgcggctga cgacatcatg gaccccacaa gcctcgctga aacccaggaa gaccacaatg 420
ggggccagaa gcagctgtcc ccacaaaaag tgacgcttta cctgcgacca ggccaggcag 480
cagcgttcaa cgtgaccttc cggcgggcca agggctaccc catcgacctg tactatctga 540
tggacctctc ctactccatg cttgatgacc tcaggaatgt caagaagcta ggtggcgacc 600
tgctccgggc cctcaacgag atcaccgagt ccggccgcat tggcttcggg tccttcgtgg 660
acaagaccgt gctgccgttc gtgaacacgc accctgataa gctgcgaaac ccatgcccca 720
acaaggagaa agagtgccag cccccgtttg ccttcaggca cgtgctgaag ctgaccaaca 780
actccaacca gtttcagacc gaggtcggga agcagctgat ttccggaaac ctggatgcac 840
ccgagggtgg gctggacgcc atgatgcagg tcgccgcctg cccggaggaa atcggctggc 900
gcaacgtcac gcggctgctg gtgtttgcca ctgatgacgg cttccatttc gcgggcgacg 960
ggaagctggg cgccatcctg acccccaacg acggccgctg tcacctggag gacaacttgt 1020
acaagaggag caacgaattc gactacccat cggtgggcca gctggcgcac aagctggctg 1080
aaaacaacat ccagcccatc ttcgcggtga ccagtaggat ggtgaagacc tacgagaaac 1140
tcaccgagat catccccaag tcagccgtgg gggagctgtc tgaggactcc agcaatgtgg 1200
tccaactcat taagaatgct tacaataaac tctcctccag ggtcttcctg gatcacaacg 1260
ccctccccga caccctgaaa gtcacctacg actccttctg cagcaatgga gtgacgcaca 1320
ggaaccagcc cagaggtgac tgtgatggcg tgcagatcaa tgtcccgatc accttccagg 1380
tgaaggtcac ggccacagag tgcatccagg agcagtcgtt tgtcatccgg gcgctgggct 1440
tcacggacat agtgaccgtg caggttcttc cccagtgtga gtgccggtgc cgggaccaga 1500
gcagagaccg cagcctctgc catggcaagg gcttcttgga gtgcggcatc tgcaggtgtg 1560
acactggcta cattgggaaa aactgtgagt gccagacaca gggccggagc agccaggagc 1620
tggaaggaag ctgccggaag gacaacaact ccatcatctg ctcagggctg gggggactgtg 1680

tctgcgggca gtgcctgtgc cacaccagcg acgtccccgg caagctgata tacgggcagt 1740
actgcgagtg tgacaccatc aactgtgagc gctacaacgg ccaggtctgc ggcggcccgg 1800
ggagggggct ctgcttctgc gggaagtgcc gctgccaccc gggctttgag ggctcagcgt 1860
gccagtgcga gaggaccact gagggctgcc tgaacccgcg gcgtgttgag tgtagtggtc 1920
gtggccggtg ccgctgcaac gtatgcgagt gccattcagg ctaccagctg cctctgtgcc 1980
aggagtgccc cggctgcccc tcaccctgtg gcaagtacat ctcctgcgcc gagtgcctga 2040
agttcgaaaa gggccccttt gggaagaact gcagcgcggc gtgtccgggc ctgcagctgt 2100
cgaacaaccc cgtgaagggc aggacctgca aggagaggga ctcagagggc tgctgggtgg 2160
cctacacgct ggagcagcag gacgggatgg accgctacct catctatgtg gatgagagcc 2220
gagagtgtgt ggcaggcccc aacatcgccg ccatcgtcgg gggcaccgtg gcaggcatcg 2280
tgctgatcgg cattctcctg ctggtcatct ggaaggctct gatccacctg agcgacctcc 2340
gggagtacag gcgctttgag aaggagaagc tcaagtccca gtggaacaat gataatcccc 2400
ttttcaagag cgccaccacg acggtcatga accccaagtt tgctgagagt taggagcact 2460
tggtgaagac aaggccgtca ggacccacca tgtctgcccc atcacgcggc cgagacatgg 2520
cttgccacag ctcttgagga tgtcaccaat taaccagaaa tccagttatt ttccgccctc 2580
aaaatgacag ccatggccgg ccgggtgctt ctgggggctc gtcgggggga cagctccact 2640
ctgactggca cagtctttgc atggagactt gaggagggag ggcttgaggt tggtgaggtt 2700
aggtgcgtgt ttcctgtgca agtcaggaca tcagtctgat taaaggtggt gccaatttat 2760
ttacatttaa acttgtcagg gtataaaatg acatcccatt aattatattg ttaatcaatc 2820
acgtgtatag aaaaaaaata aaacttcaat acaggctgtc catggaaaaa aaaaaaaaaa 2880
aaaaaaa 2887

<210> 104
<211> 1902
<212> DNA
<213> Homo sapiens

<400> 104

ctggcgcgcg cggccctgcg ggtgacaggc aggcgggaag gggcggggcc tcgggcgggg 60
ccgccgtggg gaggagggcg gtgggagggg aggagtggag atggcggcgg cggcggctca 120
gggggcgggg ggcggggagc cccgtagaac cgaggggggtc ggcccggggg tcccggggga 180
ggtggagatg gtgaaggggc agccgttcga cgtgggcccg cgctacacgc agttgcagta 240
catcggcgag ggcgcgtacg gcatggtcag ctcggcctat gaccacgtgc gcaagactcg 300
cgtggccatc aagaagatca gccccttcga acatcagacc tactgccagc gcacgctccg 360
ggagatccag atcctgctgc gcttccgcca tgagaatgtc atcggcatcc gagacattct 420
gcgggcgtcc accctggaag ccatgagaga tgtctacatt gtgcaggacc tgatggagac 480
tgacctgtac aagttgctga aaagccagca gctgagcaat gaccatatct gctacttcct 540

ctaccagatc ctgcggggcc tcaagtacat ccactccgcc aacgtgctcc accgagatct 600
aaagccctcc aacctgctca tcaacaccac ctgcgacctt aagatttgtg atttcggcct 660
ggcccggatt gccgatcctg agcatgacca caccggcttc ctgacggagt atgtggctac 720
gcgctggtac cgggccccag agatcatgct gaactccaag ggctatacca agtccatcga 780
catctggtct gtgggctgca ttctggctga gatgctctct aaccggccca tcttccctgg 840
caagcactac ctggatcagc tcaaccacat tctgggcatc ctgggctccc catcccagga 900
ggacctgaat tgtatcatca acatgaaggc ccgaaactac ctacagtctc tgccctccaa 960
gaccaaggtg gcttgggcca agcttttccc caagtcagac tccaaagccc ttgacctgct 1020
ggaccggatg ttaaccttta accccaataa acggatcaca gtggaggaag cgctggctca 1080
cccctacctg gagcagtact atgacccgac ggatgagcca gtggccgagg agcccttcac 1140
cttcgccatg gagctggatg acctacctaa ggagcggctg aaggagctca tcttccagga 1200
gacagcacgc ttccagcccg gagtgctgga ggccccctag cccagacaga catctctgca 1260
ccctggggcc tggacctgcc tcctgcctgc ccctctcccg ccagactgtt agaaaatgga 1320
cactgtgccc agcccggacc ttggcagccc aggccggggt ggagcatggg cctggccacc 1380
tctctccttt gctgaggcct ccagcttcag gcaggccaag gccttctcct ccccacccgc 1440
cctccccacg gggcctcggg acctcaggtg gccccagttc aatctcccgc tgctgctgct 1500
gcgcccttac cttccccagc gtcccagtct ctggcagttc tggaatggaa gggttctggc 1560
tgccccaacc tgctgaaggg cagaggtgga gggtgggggg cgctgagtag ggactcaggg 1620
ccatgcctgc ccccctcatc tcattcaaac cccaccctag tttccctgaa ggaacattcc 1680
ttagtctcaa gggctagcat ccctgaggag ccaggccggg ccgaatcccc tccctgtcaa 1740
agctgtcact tcgcgtgccc tcgctgcttc tgtgtgtggt gagcagaagt ggagctgggg 1800
ggcgtggaga gcccggcgcc cctgccacct ccctgacccg tctaatatat aaatatagag 1860
atgtgtctat ggctgaaaaa aaaaaaaaaa aaaaaaaaaa aa 1902

<210> 105
<211> 2826
<212> DNA
<213> Homo sapiens

<400> 105

tcgagacctc aagggtagag gtgggcaccc ccgcctccgc acttttgctc ggggctccag 60
attgtagggc agggcggcgc ttctcggaaa gcgaaagccg gcggggcggg gcgggtgccg 120
caggagaaag aggaagcgct ggcagacaat gcgacccgac cgcgctgagg ctccaggacc 180
gcccgccatg gctgcaggag gtcccggcgc ggggtctgcg gccccggtct cctccacatc 240
ctcccttccc ctggctgctc tcaacatgcg agtgcggcgc cgcctgtctc tgttcttgaa 300
cgtgcggaca caggtggcgg ccgactggac cgcgctggcg gaggagatgg actttgagta 360
cttggagatc cggcaactgg agacacaagc ggaccccact ggcaggctgc tggacgcctg 420
gcagggacgc cctggcgcct ctgtaggccg actgctcgag ctgcttacca agctgggccg 480

cgacgacgtg ctgctggagc tgggacccag cattgaggag gattgccaaa agtatatctt 540
gaagcagcag caggaggagg ctgagaagcc tttacaggtg gccgctgtag acagcagtgt 600
cccacggaca gcagagctgg cgggcatcac cacacttgat gaccccctgg ggcatatgcc 660
tgagcgtttc gatgccttca tctgctattg ccccagcgac atccagtttg tgcaggagat 720
gatccggcaa ctggaacaga caaactatcg actgaagttg tgtgtgtctg accgcgatgt 780
cctgcctggc acctgtgtct ggtctattgc tagtgagctc atcgaaaaga ggtgccgccg 840
gatggtggtg gttgtctctg atgattacct gcagagcaag gaatgtgact tccagaccaa 900
atttgcactc agcctctctc caggtgccca tcagaagcga ctgatcccca tcaagtacaa 960
ggcaatgaag aaagagttcc ccagcatcct gaggttcatc actgtctgcg actacaccaa 1020
cccctgcacc aaatcttggt tctggactcg ccttgccaag gccttgtccc tgccctgaag 1080
actgttctga ggccctgggt gtgtgtgtat ctgtctgcct gtccatgtac ttctgccctg 1140
cctcctcctt tcgttgtagg aggaatctgt gctctactta cctctcaatt cctggagatg 1200
ccaacttcac agacacgtct gcagcagctg gacatcacat ttcatgtcct gcatggaacc 1260
agtggctgtg agtggcatgt ccacttgctg gattatcagc caggacacta tagaacagga 1320
ccagctgaga ctaagaagga ccagcagagc cagctcagct ctgagccatt cacacatctt 1380
caccctcagt ttcctcactt gaggagtggg atggggagaa cagagagtag ctgtgtttga 1440
atccctgtag gaaatggtga agcatagctc tgggtctcct gggggagacc aggcttggct 1500
gcgggagagc tggctgttgc tggactacat gctggccact gctgtgacca cgacactgct 1560
ggggcagctt cttccacagt gatgcctact gatgcttcag tgcctctgca caccgcccat 1620
tccacttcct ccttccccac agggcaggtg gggaagcagt ttggcccagc ccaaggagac 1680
cccaccttga gccttatttc ctaatgggtc cacctctcat ctgcatcttt cacacctccc 1740
agcttctgcc caaccttcag cagtgacaag tccccaagag actcgcctga gcagcttggg 1800
ctgcttttca tttccacctg tcaggatgcc tgtggtcatg ctctcagctc cacctggcat 1860
gagaagggat cctggcctct ggcatattca tcaagtatga gttctgggga tgagtcactg 1920
taatgatgtg agcagggagc cttcctccct gggccacctg cagagagctt tcccaccaac 1980
tttgtacctt gattgcctta caaagttatt tgtttacaaa cagcgaccat ataaaagcct 2040
cctgccccaa agcttgtggg cacatgggca catacagact cacatacaga cacacacata 2100
tatgtacaga catgtactct cacacacaca ggcaccagca tacacacgtt tttctaggta 2160
cagctcccag gaacagctag gtgggaaagt cccatcactg agggagccta accatgtccc 2220
tgaacaaaaa ttgggcactc atctattcct tttctcttgt gtccctactc attgaaacca 2280
aactctggaa aggacccaat gtaccagtat ttatacctct aatgaagcac agagagagga 2340
agagagctgc ttaaactcac acaacaatga actgcagaca cagctgttct ctccctctct 2400
ccttcccaga gcaatttata ctttaccctc aggctgtcct ctggggagaa ggtgccatgg 2460
tcttaggtgt ctgtgcccca ggacagaccc taggacccta aatccaatag aaaatgcata 2520

tctttgctcc actttcagcc aggctggagc aaggtacctt ttcttaggat cttgggaggg 2580
aatggatgcc cctctctgca tgatcttgtt gaggcattta gctgccatgc acctgtcccc 2640
ctttaatact gggcatttta aagccatctc aagaggcatc ttctacatgt tttgtacgca 2700
ttaaaataat ttcaaagata tctgagaaaa gccgatattt gccattcttc ctatatcctg 2760
gaatatatct tgcatcctga gtttataata ataaataata ttctaccttg gaaaaaaaaa 2820
aaaaaa 2826

<210> 106
<211> 1669
<212> DNA
<213> Homo sapiens

<400> 106

ctccctcagc aaggacagca gaggaccagc taagagggag agaagcaact acagaccccc 60
cctgaaaaca accctcagac gccacatccc ctgacaagct gccaggcagg ttctcttcct 120
ctcacatact gacccacggc tccaccctct ctcccctgga aaggacacca tgagcactga 180
aagcatgatc cgggacgtgg agctggccga ggaggcgctc cccaagaaga caggggggcc 240
ccagggctcc aggcggtgct tgttcctcag cctcttctcc ttcctgatcg tggcaggcgc 300
caccacgctc ttctgcctgc tgcactttgg agtgatcggc ccccagaggg aagagttccc 360
cagggacctc tctctaatca gccctctggc ccaggcagtc agatcatctt ctcgaacccc 420
gagtgacaag cctgtagccc atgttgtagc aaaccctcaa gctgaggggc agctccagtg 480
gctgaaccgc cgggccaatg ccctcctggc caatggcgtg gagctgagag ataaccagct 540
ggtggtgcca tcagagggcc tgtacctcat ctactcccag gtcctcttca agggccaagg 600
ctgcccctcc acccatgtgc tcctcaccca caccatcagc cgcatcgccg tctcctacca 660
gaccaaggtc aacctcctct ctgccatcaa gagcccctgc cagagggaga ccccagaggg 720
ggctgaggcc aagccctggt atgagcccat ctatctggga ggggtcttcc agctggagaa 780
gggtgaccga ctcagcgctg agatcaatcg gcccgactat ctcgactttg ccgagtctgg 840
gcaggtctac tttgggatca ttgccctgtg aggaggacga acatccaacc ttcccaaacg 900
cctcccctgc cccaatccct ttattacccc ctccttcaga caccctcaac ctcttctggc 960
tcaaaaagag aattgggggc ttagggtcgg aacccaagct tagaacttta agcaacaaga 1020
ccaccacttc gaaacctggg attcaggaat gtgtggcctg cacagtgaag tgctggcaac 1080
cactaagaat tcaaactggg gcctccagaa ctcactgggg cctacagctt tgatccctga 1140
catctggaat ctggagacca gggagccttt ggttctggcc agaatgctgc aggacttgag 1200
aagacctcac ctagaaattg acacaagtgg accttaggcc ttcctctctc cagatgtttc 1260
cagacttcct tgagacacgg agcccagccc tccccatgga gccagctccc tctatttatg 1320
tttgcacttg tgattattta ttatttattt attatttatt tatttacaga tgaatgtatt 1380
tatttgggag accggggtat cctgggggac ccaatgtagg agctgccttg gctcagacat 1440

gttttccgtg aaaacggagc tgaacaatag gctgttccca tgtagccccc tggcctctgt 1500
gccttctttt gattatgttt tttaaaatat ttatctgatt aagttgtcta aacaatgctg 1560
atttggtgac caactgtcac tcattgctga gcctctgctc cccaggggag ttgtgtctgt 1620
aatcgcccta ctattcagtg gcgagaaata aagtttgctt agaaaagaa 1669

<210> 107
<211> 948
<212> DNA
<213> Homo sapiens

<400> 107

attgtggtgc cttgtagctg tcccgggagc cctcagcagc agttggagct ggtgcacagg 60
aaggatgagg aagaccaggc tctgggggct gctgtggatg ctctttgtct cagaactccg 120
agctgcaact aaattaactg aggaaaagta tgaactgaaa gaggggcaga ccctggatgt 180
gaaatgtgac tacacgctag agaagtttgc cagcagccag aaagcttggc agataataag 240
ggacggagag atgcccaaga ccctggcatg cacagagagg ccttcaaaga attcccatcc 300
agtccaagtg gggaggatca tactagaaga ctaccatgat catggtttac tgcgcgtccg 360
aatggtcaac cttcaagtgg aagattctgg actgtatcag tgtgtgatct accagcctcc 420
caaggagcct cacatgctgt tcgatcgcat ccgcttggtg gtgaccaagg gtttttcagg 480
gacccctggc tccaatgaga attctaccca gaatgtgtat aagattcctc ctaccaccac 540
taaggccttg tgcccactct ataccagccc cagaactgtg acccaagctc cacccaagtc 600
aactgccgat gtctccactc ctgactctga aatcaacctt acaaatgtga cagatatcat 660
cagggttccg gtgttcaaca ttgtcattct cctggctggt ggattcctga gtaagagcct 720
ggtcttctct gtcctgtttg ctgtcacgct gaggtcattt gtaccctagg cccacgaacc 780
cacgagaatg tcctctgact tccagccaca tccatctggc agttgtgcca agggaggagg 840
gaggaggtaa aaggcaggga gttaataaca tgaattaaat ctgtaatcac cagctatttc 900
taaagtcagc gtctcacctt aaaaaaaaaa aaaaaaaaaa aaaaaaaa 948

## Beschreibung

Die vorliegende Erfindung betrifft ein Verfahren zur Normalisierung einer mRNA Menge in mehreren Proben gemäß Anspruch 1, einen Kit mit einer Auswahl von Sequenzen gemäß Anspruch 9, eine Verwendung von Nucleinsäuren als Kontrollgene zur Normalisierung von Genexpressionsanalysedaten gemäß Anspruch 10 sowie Kontrollgen-Sätze zur Normalisierung von Genexpressionsanalysen gemäß Anspruch 11 und 12.

Nach wie vor besteht ein Bedarf, Gene, insbesondere aus Blutzellen, zu identifizieren, welche in ihrer Expression unter verschiedenen Bedingungen nur minimale Variation zeigen. Diese sogenannten "Housekeeper" oder "Housekeeping"-Gene finden Anwendung als Referenzen, interne Kontrollen und Bezugswerte bei der Quantifikation der Genexpression und von RNA und mRNA mit Methoden wie Northern Blotting, Ribonuklease Protection assay, Kapillarelektrophorese, Microarrays und quantitativer real-time PCR sowie mittels weiterer Verfahren zur direkten Messung der Transkription und Messung nach vorheriger Amplifikation.

Im Folgenden werden die Begriffe Housekeeper, Housekeeping-Gene und Expressionskontrollgene unter dem Begriff Kontrollgene zusammengefasst. Diese Vereinfachung wird aus Gründen der Lesbarkeit vorgenommen und stellt keine Einschränkung der Erfindung dar.

Eine Normalisierung quantitativer Daten mittels Kontrollgenen hat zahlreiche Anwendungsmöglichkeiten. Die Kontrollgene ermöglichen eine Identifikation von Genen deren Aktivität bei verschiedenen Krankheitszuständen differentiell reguliert wird sowie die Entwicklung darauf basierender Diagnostika.

Ein Kontrollgen ist ein Gen, welches minimale Änderung der Expression und Transkription über verschiedene RNA Proben zeigt und damit als Kontrolle zur Messung veränderlicher Genaktivitäten über verschiedene Proben dient. Kein Gen zeigt unveränderte Aktivität über alle Gewebe. Daher besteht ein hoher Bedarf an neuen Kontrollgenen, insbesondere für Blut, da Expressionswerte aus Blut diagnostisch angewendet werden.

Obwohl verschiedene Kontrollgene literaturbekannt sind [1], sind keine Kontrollgene und deren Transkripte sowie deren kombinierte Verwendung zur Normalisierung der Genexpression und Transkription aus Vollblutproben und Blutzellen bekannt. Transkripte (auch mRNA und microRNA sowie weitere RNA) mit konstanter Konzentration in Blutzellen und in Zellen aus Organen und peripherem Gewebe welche in Vollblut lokalisiert sind, stellen eine Voraussetzung zur Normalisierung von Genaktivitäten und zur Ermittlung der Veränderungen anderer Genaktivitäten dar und somit eine Voraussetzung für Blut-basierte Diagnostik. Ebenso sind bereits verschiedene Studien zur Messung der Genaktivität für die Diagnose/Prognose von SIRS und Sepsis publiziert, beispielsweise [2,3], eine Verwendung und Quantifizierung dieser Genaktivitätssignale mittels Kontrollgenen aus Blut wurde jedoch noch nicht beschrieben.

Es besteht somit ein Bedarf an robusten und über eine Stabilität verfügender Kontrollgene aus Blut und Blutzellen, die eine Normalisierung und Quantifizierung der Genexpression von krankheitsspezifischen Genen oder Genclustern ermöglicht.

Ausgangspunkt für die in der vorliegenden Patentanmeldung offenbarten Erfindung ist die Erkenntnis, dass Genaktivitäten verschiedener Gene, welche in Blutzellen vorkommen, in Proben eines Individuums bei dem Sepsistypische Krankheitserscheinungen (entsprechend der Definition in [4]) festgestellt werden, sich von den Genaktivitäten der gleichen Gene von Individuen, bei denen keine Sepsis diagnostiziert wurde nicht unterscheiden und gemeinsam oder einzeln als Kontrollgene zur Normalisierung von Genaktivitäten aus Blutzellen und zur Konzentrationsbestimmung von Transkripten aus Blut verwendet werden können. Dies erlaubt die Normalisierung und relative Quantifizierung der Aktivitäten anderer Gene, was zur Diagnose, Prognose, Therapie und Verlaufskontrolle genutzt werden kann.

Der vorliegenden Erfindung liegt somit die Aufgabe zugrunde, Mittel und Verfahren zur Verfügung zu stellen, welche einen Bezugspunkt zur Unterscheidung krankheitsbedingter Genexpressionsänderungen und damit eine Diagnose oder Verlaufskontrolle der Therapie ermöglicht.

Verfahrenstechnisch wird diese Aufgabe durch die Merkmale des Anspruchs 1 gelöst.

Die Aufgabe wird ferner durch einen Kit gemäß Anspruch 9 sowie eine Verwendung gemäß Anspruch 10 und durch Kontrollgen-Sätze gemäß den Ansprüchen 11 und 12 gelöst.

Die Erfindung beschreibt die Identifikation von neuen Kontrollgenen aus Blut, geeignete Mikroarray-Sonden und PCR-Primer und ihre Verwendung, auch in Kombination, zur Normalisierung von quantitativen Expressionsdaten aus Blut und Blutzellen in Microarrays, real-time PCR assays und anderen Systemen mit oder ohne Amplifikation und mit verschiedenen Visualisierungsmöglichkeiten zur Bestimmung sowie deren Anwendung zur Diagnose krankheitsbedingter Veränderungen bei lokalen Entzündungen unterschiedlicher Lokalisation und bei der systemischen Reaktion darauf wie SIRS, Sepsis, schwere Sepsis mit Organversagen.

Bei diesen Untersuchungen ist die Normalisierung von Genexpressionsanalysen von entscheidender Bedeutung. Für die Zwecke der vorliegenden Erfindung soll unter Normalisierung folgendes verstanden werden:

"Unter einer Normalisierung versteht man, die Messungen von verschiedenen Arrays bzw. PCR oder insbesondere RT-PCR Experimenten vergleichbar zu machen, indem man die technische Variabilität vermindert bzw. entfernt. Innerhalb dieser Experimente gibt es eine Vielzahl von Quellen, welche die Messungen verfälschen können. Mögliche technische Störquellen sind eine unterschiedliche Effizienz bei der reversen Transkription, dem Labelling oder den Hybridisierungsreaktionen sowie Probleme mit den Arrays, Chargeneffekte bei den Reagenzien oder laborspezifische Bedingungen."

Das erfindungsgemäße Verfahren ist dadurch gekennzeichnet, dass man in einer Blutprobe eines Individuums die Aktivität von einem oder mehreren zu untersuchenden Genen durch Feststellung der Anwesenheit und der Menge des Genprodukts relativ zu den Mengen der Genprodukte der Kontrollgenen zwischen SIRS und Sepsis unterscheiden kann.

Offenbart werden hierzu Kontrollgene und Gensequenzen aus Blut und Blutzellen sowie daraus abgeleitete Primer und Sonden, welche zur Bestimmung, Visualisierung und Normalisierung und Quantifizierung von Genaktivitäten und Transkripten verwendet werden können. Die Sequenzen der Oligonukleotidsonden in bevorzugter Ausführung sind in Tabelle 1 dargelegt und entsprechen der im beigefügten Sequenzprotokoll Seq-ID 1 bis Seq-ID 7, verwendete Primersequenzen in Tabelle 2 entsprechen der im beigefügten Sequenzprotokoll Seq-ID 8 bis Seq-ID 21. Dabei können die Sequenzen der Oligonukleotidsonden auch weitere Sequenzen, in bevorzugter Ausführung von einer Länge von 50-100 Nukleotide annehmen, welche spezifisch Transkripte der in Tabelle 3 dargelegten Gene mit Sequenzen Seq-ID 22 bis Seq-ID 97 binden. Die Länge der in Amplifikationsverfahren wie PCR verwendeten Sequenzen kann beliebig sein soweit sie die gewünschte enzymatische Manipulation und Amplifikation unterstützen.

Tabelle 1: DNA-Oligonukleotidsonden

| Gene Symbol | SEQ-ID |
|---|---|
| ITGAL | 1 |
| SNAPC1 | 2 |
| CASP8 | 3 |
| C7 | 4 |
| PPARD | 5 |
| IL18 | 6 |
| F3 | 7 |

Tabelle 2: Forward und Reverse DNA-Primer.

| Gene Symbol | Forward Primer SEQ-ID | Reverse Primer SEQ-ID |
|---|---|---|
| ITGAL | 8 | 15 |
| SNAPC1 | 9 | 16 |
| CASP8 | 10 | 17 |
| C7 | 11 | 18 |
| PPARD | 12 | 19 |
| IL18 | 13 | 20 |
| F3 | 14 | 21 |

Tabelle 3: Kontrollgene (RNA-Sequenzen)

| GenBank Accession Nummer | SEQ-ID |
|---|---|
| NM_024081 | 22 |
| AA398364 | 23 |
| N34546 | 24 |
| AA659421 | 25 |
| AA682479 | 26 |
| AK024118 | 27 |

(fortgesetzt)

| GenBank Accession Nummer | SEQ-ID |
|---|---|
| AA923316 | 28 |
| BM309952 | 29 |
| AI093653 | 30 |
| AI131415 | 31 |
| AI263527 | 32 |
| AA282242 | 33 |
| CR740270 | 34 |
| BG191861 | 35 |
| AI301257 | 36 |
| AI310464 | 37 |
| AW964023 | 38 |
| AI351933 | 39 |
| AA100540 | 40 |
| AI362368 | 41 |
| AI817134 | 42 |
| AI381377 | 43 |
| AI520967 | 44 |
| AA253470 | 45 |
| AI559304 | 46 |
| AI565002 | 47 |
| AI587389 | 48 |
| AI609367 | 49 |
| AI635278 | 50 |
| AI702056 | 51 |
| AI707917 | 52 |
| AI733176 | 53 |
| AI769053 | 54 |
| AI798545 | 55 |
| AI801425 | 56 |
| AI801595 | 57 |
| AI809873 | 58 |
| AI862063 | 59 |
| AI923251 | 60 |
| AI925556 | 61 |
| AI932551 | 62 |
| AI932884 | 63 |
| AI933797 | 64 |
| AI933967 | 65 |

(fortgesetzt)

| GenBank Accession Nummer | SEQ-ID |
|---|---|
| AI935874 | 66 |
| H06263 | 67 |
| H22921 | 68 |
| H54423 | 69 |
| N22551 | 70 |
| N73510 | 71 |
| R06107 | 72 |
| R42511 | 73 |
| R43088 | 74 |
| NM_181705 | 75 |
| R92455 | 76 |
| R93174 | 77 |
| T77995 | 78 |
| T79815 | 79 |
| T83946 | 80 |
| T95909 | 81 |
| T98779 | 82 |
| AK127462 | 83 |
| W80744 | 84 |
| W86575 | 85 |
| AJ297560 | 86 |
| NM_001562 | 87 |
| BU629240 | 88 |
| NM_001228 | 89 |
| NM_001993 | 90 |
| NM_002209 | 91 |
| NM_002392 | 92 |
| NM_000587 | 93 |
| NM_004379 | 94 |
| BC002715 | 95 |
| NM_003082 | 96 |
| AA664688 | 97 |

Die Primer in Tabelle 2 können verwendet werden, um Amplifikationsprodukte herzustellen, welche die gewünschte Region (Sequenz) der genannten Gene enthält. In üblicher Ausführung ist das Produkt 150-200 Nukleotide lang.

Die Kontrollgene können einzeln oder in Kombination von mehreren verwendet werden. Üblicherweise kann die Aktivität von Kontrollgene wie hier beschrieben mit Hybridisierungssonden für Microarrays oder PCR Primern und real-time PCR bestimmt werden. Die Kontrollgene und ihre Expressionsprodukte können aber auch nach Amplifikation mit anderen dem Fachmann bekannten Methoden wie beispielsweise NASBA (Nucleic Acid Sequence-based Amplification) und in verschiedener Kombination ermittelt werden. Sie können auch mit einer Reihe weiterer Methoden oder Visualisierungsmöglichkeiten wie beispielsweise mit Hilfe monoklonaler Antikörper bestimmt werden. Primer und Sonden können für das Gen, das Expressionsprodukt (mRNA) oder Expressionszwischenprodukte, welche nicht komplett zu mRNA prozessiert werden, eingesetzt werden.

In weiteren Ausführungen binden die Primer und Sonden eine spezifische Region der hier offenbarten Kontrollgene oder ihrer Transkripte. Die Sonden und Primer können aber mit jeder Region der hier offenbarten Gensequenzen oder daraus transkribierten Sequenzen wechselwirken. Die Primer und Sonden können über fortlaufende Basenpaarung wechselwirken müssen aber nicht kontinuierlich mit der kompletten komplementären Sequenz wechselwirken. Die Pufferzusammensetzungen, Salzkonzentrationen, Waschschritte und Temperaturen können hier variabel gewählt werden.

Ebenso können diese Veränderungen der Kontrollgene und der Testgene mit den Expressionswerten (oder daraus ableiteten Daten wie z.B. Durchschnittswerten) einer oder mehrerer Referenzproben verglichen werden, welche nicht gleichzeitig mit der Zielprobe ermittelt werden.

Eine Ausführungsform der Erfindung ist dadurch gekennzeichnet, dass man Expressionswerte unter Anwendung von Kontrollgenen der Tabelle 3 sowie Nukleinsäuren und Transkripte dieser Kontrollgene aus Blut und aus Blutzellen als Kontrollgene durch Vergleich der Expressionswerte mit einer oder mehreren Testnukleinsäuren und durch Quantifizierung in Bezug zur Testnukleinsäure ermittelt.

Eine weitere Ausführungsform der Erfindung ist dadurch gekennzeichnet, dass Nukleinsäuren und DNA Sonden mit den Sequenzen nach Tabelle 1 und deren Bindung von RNA inklusive microRNA und von Transkripten (RNA oder mRNA) in Blut oder aus Blutzellen von Genen nach Tabelle 3 in Lösung oder immobilisiert auf Oberflächen oder Partikeln oder Beads und die Verwendung der gebundenen Transkripte dieser Gene zur Normalisierung durch Vergleich der gebundenen Mengen (Expressionswerte) der Nukleinsäuren mit einer oder mehreren an Sonden gebundenen Testnukleinsäuren und zur Quantifizierung in Bezug zur gebundenen Testnukleinsäure verwendet werden.

Eine Ausführungsform der Erfindung ist dadurch gekennzeichnet, dass das Verfahren zur *ex vivo, in vitro* Unterscheidung zwischen SIRS und Sepsis (beides entsprechend [4]) basierend durch in-Bezug-setzen der RNA-Mengen aus Kontrollgen und Testgen, folgende Schritte erfasst:

a) Isolieren von Kontrollgen-RNA sowie Testgen-RNA aus einer Blutprobe

b) Markieren der Kontrollgen- und Testgen-RNA mit einem detektierbaren Marker und In-Kontakt-Bringen mit der DNA unter Hybridisierungsbedingungen, wobei die DNA ein Genfragment oder Oligonukleotid ist, welches spezifisch Transkripte, Amplifikationsprodukte oder *in vitro* Transkripte von Kontrollgenen bindet.

c) quantitatives Erfassen der Markierungssignale der Kontrollgen und Testgen-RNA entsprechend b) und

d) Vergleichen der quantitativen Daten der Markierungssignale, um eine Aussage zu treffen, ob ein spezifisches Gen oder Genfragment in Vergleich zu den Signalen der Kontrollgene stärker oder schwächer exprimiert sind.

Eine weitere Ausführungsform der Erfindung ist dadurch gekennzeichnet, dass man die Kontrollgen-RNA vor dem Messen der Testgen-RNA mit der DNA hybridisiert und die Markierungssignale des Kontroll-RNA/DNA-Komplexes erfasst, ggf. weiter transformiert und gegebenenfalls in Form einer Kalibrierkurve oder -tabelle ablegt.

Eine weitere Ausführungsform der Erfindung ist dadurch gekennzeichnet, dass RNA der Kontrollgene oder Teile davon über Sequenzierung oder teilweise Sequenzierung beispielsweise über Pyrosequenzierung identifiziert und quantifiziert werden.

Eine weitere Ausführungsform der Erfindung ist dadurch gekennzeichnet, dass als Kontrollgen-RNA mRNA oder microRNA verwendet wird.

Eine weitere Ausführungsform der Erfindung ist dadurch gekennzeichnet, dass die DNA zur spezifischen Bindung der Kontrollgen-RNA oder deren in vitro Transkripte an vorbestimmten Bereichen auf einem Träger in Form eines Microarrays angeordnet, insbesondere immobilisiert, wird.

Eine weitere Ausführungsform der Erfindung ist dadurch gekennzeichnet, dass es sich bei der biologischen Probe um die eines Menschen handelt.

Diese Sequenzen mit der Sequenz-ID: 1 bis zur Sequenz-ID: 97 sind durch den Umfang der vorliegenden Erfindung mit umfasst und sind dem angefügten 70-seitigen, 107 Sequenzen umfassenden, Sequenzprotokoll, das somit Teil der Erfindung ist, im Einzelnen offenbart.

Eine weitere Ausführungsform der Erfindung ist dadurch gekennzeichnet, dass die immobilisierten oder freien Sonden mit Sequenzen entsprechend Tabelle 1 markiert werden. Für diese Ausführungsform finden selbstkomplementäre Oligonukleotide, so genannte Molecular beacons, als Sonden Verwendung. Sie tragen an ihren Enden ein Fluorophor/Quencher-Paar, so dass sie in Abwesenheit einer komplementären Sequenz in einer gefalteten Haarnadelstruktur vorliegen und erst mit einer entsprechenden Probensequenz ein Fluoreszenzsignal liefern. Die Haarnadelstruktur der Molecular Beacons ist so lange stabil, bis die Probe an der spezifischen Fängersequenz hybridisiert, was zu einer Konformationsänderung und damit auch Freisetzung der Reporterfluoreszenz führt.

Eine weitere Ausführungsform der Erfindung ist dadurch gekennzeichnet, dass wenigstens 1 bis 14 Nukleinsäuresonden oder deren Komplementäre zur Bindung der Transkripte oder deren Komplementäre der Kontrollgene verwendet werden.

Eine weitere Ausführungsform der Erfindung ist dadurch gekennzeichnet, dass die synthetischen Analoga der Kontrollgene bzw. die synthetischen Oligonukleotide welche die Transkripte der Kontrollgene binden insbesondere ca. 60 Basenpaare umfassen.

Eine weitere Ausführungsform der Erfindung ist dadurch gekennzeichnet, dass die als DNA von in den Ansprüchen aufgelisteten Gene ersetzt werden durch von deren RNA abgeleiteten Sequenzen, synthetische Analoga, Aptamere sowie Peptidonukleinsäuren.

Eine weitere Ausführungsform der Erfindung ist dadurch gekennzeichnet, dass als detektierbarer Marker ein radioaktiver Marker, insbesondere $^{32}P$, $^{14}C$, $^{125}I$, $^{33}P$ oder $^{3}H$ verwendet wird.

Eine weitere Ausführungsform der Erfindung ist dadurch gekennzeichnet, dass als detektierbarer Marker ein nicht radioaktiver Marker, insbesondere ein Farb- oder Fluoreszenzmarker, ein Enzymmarker oder Immunmarker, und/oder quantum dots oder ein elektrisch messbares Signal, insbesondere Potential- und/oder Leitfähigkeits- und/oder Kapazitätsänderung bei Hybridisierungen, verwendet wird.

Eine weitere Ausführungsform der Erfindung ist dadurch gekennzeichnet, dass die Proben-RNA und Kontrollgen-RNA und/oder enzymatische oder chemische Derivate dieselbe Markierung tragen.

Eine weitere Ausführungsform der Erfindung ist dadurch gekennzeichnet, dass die Testgen-RNA und Kontrollgen-RNA und/oder enzymatische oder chemische Derivate unterschiedliche Markierungen tragen.

Eine weitere Ausführungsform der Erfindung ist dadurch gekennzeichnet, dass die DNA-Sonden auf Glas oder Kunststoff, immobilisiert werden.

Eine weitere Ausführungsform der Erfindung ist dadurch gekennzeichnet, dass die einzelnen DNA-Moleküle über eine kovalente Bindung an das Trägermaterial immobilisiert werden.

Eine weitere Ausführungsform der Erfindung ist dadurch gekennzeichnet, dass die einzelnen DNA Moleküle mittels elektrostatischer- und/oder Dipol-Dipol- und/oder hydrophober Wechselwirkungen und/oder Wasserstoffbrücken an das Trägermaterial immobilisiert werden.

Eine weitere Ausführungsform der Erfindung besteht in der Verwendung von rekombinant oder synthetisch hergestellten, spezifischen Kontrollgen-Nukleinsäuresequenzen, Partialsequenzen einzeln oder in Teilmengen als Kalibrator in Sepsis-Assays und/oder zur Bewertung der Wirkung und Toxizität beim Wirkstoffscreening und/oder zur Herstellung von Therapeutika und von Stoffen und Stoffgemischen, die als Therapeutikum vorgesehen sind, zur Vorbeugung und Behandlung von SIRS und Sepsis.

Es ist dem Fachmann klar, dass die in den Ansprüchen dargelegten einzelnen Merkmale der Erfindung ohne Einschränkung beliebig miteinander kombinierbar sind.

Als Kontrollgene im Sinne der Erfindung werden alle abgeleiteten DNA-Sequenzen, Partialsequenzen und synthetischen Analoga (beispielsweise Peptidonukleinsäuren, PNA) verstanden. Die auf Bestimmung der Genexpression auf RNA-Ebene bezogene Beschreibung der Erfindung stellt keine Einschränkung sondern nur eine beispielhafte Anwendung dar.

Eine Anwendung des erfindungsgemäßen Verfahrens liegt in der Normalisierung von Messdaten der differentiellen Genexpression aus Vollblut, beispielsweise zur Unterscheidung zwischen SIRS und Sepsis und deren Schweregrade (beides entsprechend [4]). Hierzu wird die RNA der Kontrollgene aus dem Vollblut von entsprechenden Patienten und eine Kontrollprobe eines gesunden Probanden oder nicht-infektiösen Patienten isoliert. Die RNA wird anschließend markiert, beispielsweise radioaktiv mit $^{32}P$ oder mit Farbstoffmolekülen (Fluoreszenz). Als Markierungsmoleküle können alle im Stand der Technik zu diesem Zwecke bekannten Moleküle und/oder Detektionssignale eingesetzt werden. Entsprechende Moleküle und/oder Verfahren sind dem Fachmann ebenfalls bekannt.

Die so markierte RNA wird anschließend mit auf einem Microarray immobilisierten DNA-Molekülen hybridisiert. Die auf dem Microarray immobilisierten DNA-Moleküle stellen eine spezifische Auswahl der Gene gemäß der vorliegenden Erfindung zur Normalisierung von Genexpressionsdaten bei der Unterscheidung von SIRS und Sepsis dar.

Die Intensitätssignale der hybridisierten Moleküle werden im Anschluss durch geeignete Messgeräte (Phosporimager, Microarray-Scanner) gemessen und durch weitere softwaregestützte Auswertungen analysiert. Aus den gemessenen Signalintensitäten werden die Expressionsverhältnisse zwischen den Testgenen der Patientenprobe und den Kontrollgenen bestimmt. Aus den Expressionsverhältnissen der unter- und/oder überregulierten Gene lassen sich, wie in den nachstehend dargestellten Experimenten, Rückschlüsse auf die Unterscheidung SIRS und Sepsis ziehen.

Eine weitere Anwendung der über Microarrayanalyse mit nachfolgender Quantifizierung ermittelten Genaktivitäten zur Normalisierung von Genexpressionsdaten besteht in der Anwendung zur Unterscheidung von SIRS und Sepsis für die elektronischen Weiterverarbeitung zum Zweck der Herstellung von Software für Diagnosezwecke (z.B. für die Ermittlung der Lokalisation einer Entzündung und zur Einschätzung der Krankheitsschwere einer individuellen Immunantwort insbesondere bei Infektionen, auch im Rahmen von Patientendatenmanagementsystemen oder Expertensystemen) oder zur Modellierung zellulärer Signalübertragungswegen.

Für die Durchführung der Auswertung der Mikroarrays für die Zwecke der vorliegenden Patentanmeldung gilt folgendes:

**Mikroarray-Experimentbeschreibung**

(Nach der Minimum Information About a Microarray Experiment [MIAME] Checkliste - Neuauflage Januar 2005, basierend auf Brazma A et al., Minimum information about a microarray experiment (MIAME)-toward standards for microarray data, Nature Genetics 29, 365 - 371 (2001) [17), auf welches hiermit vollinhaltlich Bezug genommen wird)

Einlesen der Slides / technische Spezifikationen des Scanners

| | | |
|---|---|---|
| a) Scanner: Scanner | GenePix 4000B konfokaler Auflichtfluoreszenz-(Axon Instruments) | |
| b) Software zum Scannen: | GenPix Pro 4.0 | |
| c) Scan-Parameter : | Laser-Power: | Cy3 Kanal -100% |
| | | Cy5 Kanal -100% |
| | PMT-Spannung: | Cy3 Kanal -700 V |
| | | Cy5 Kanal - 800 V |
| d) räumliche Auflösung (pixel space) -10 $\mu$m. | | |

Auslesen und Prozessieren der Daten

Im Rahmen der Experimente wurden über 1000 Blutproben von Patienten hybridisiert. Jedes RNA-Paar (Patient gegen Vergleichs-RNA) wurde auf einem Mikroarray cohybridisiert. Dabei wurde die Patienten-RNA mit einem rot fluoreszierenden und die Vergleichs-RNA mit einem grün fluoreszierenden Farbstoff markiert. Die digitalisierten Bilder des hybridisierten Arrays wurden mit der GenePix Pro 4.0 bzw. 5.0 Software von Axon Instruments ausgewertet. Zur Spot-Detektion, Signalquantifizierung und Bewertung der Spot-Qualität wurde die GenePix™ Analysis Software verwendet. Die Spots wurden entsprechend der Einstellungen in der GenePix™ Software mit 100 = "good", 0 = "found", -50 = "not found", -75 = "absent", -100 = "bad" markiert. Die Rohdaten werden in einer entsprechenden *.gpr-Datei abgelegt.

Normalisierung, Transformation und Datenauswahlverfahren

e) Transformation und Normalisierung der Signaldaten

Zur Normalisierung und varianzstabilisierten Transformation der Rohdaten wurde das Verfahren von Huber et al. [5] angewendet, bei der die additiven und multiplikativen Fehler Block für Block geschätzt werden. Dazu werden etwa 75% aller Spots herangezogen. Die Signale werden anschließend mit der Funktion arsinh transformiert. (so entspricht das transformierte Verhältnis von $\pm 0.4$ etwa einer 1.5-fachen Veränderung {für große Zahlen ist arsinh (x) nahezu identisch mit dem Ln (2x)}.

Rocke DM, Durbin B, A model for measurement error for gene expression arrays., J Comput Biol. 2001;8(6):557-69 [18] haben ein Modell zur Abschätzung des Messfehlers in Genexpressionsarrays als Funktion des Expressionsniveaus entwickelt, auf dieses hiermit vollinhaltlich Bezug genommen wird. Dieses Fehlermodell gestattet zusammen mit weiteren Analyseverfahren, Datentransformationen und Gewichtungen bereits einen genaueren Vergleich der Genexpressionsdaten und liefert Richtlinien für Hintergrundanalyse, Bestimmung von Vertrauensintervallen und Aufbereitung der Analysedaten für deren multivariate Weiterverarbeitung bzw. Analyse.

Aufgrund des oben erwähnten Fehlermodells von Rocke und Durbin [18] haben Huber W, Heydebreck A, und Sueltmann H, Variance stabilization applied to microarray data calibration and to the quantification of differential expression., Bioinformatics. 2002;18 Suppl 1:S96-104 [19], ein statistisches Modell für Mikroarray-Genexpressionsdaten entwickelt, auf welches hiermit vollinhaltlich Bezug genommen wird. Das Modell umfasst eine Datenkalibrierung, die Quantifizierung unterschiedlicher Expressionsniveaus sowie die Quantifizierung des Messfehlers. Huber et al. [19] haben hierzu eine Datentransformation für Signalintensitätsmessungen und eine Differenzstatistik hergeleitet, welche unter Verwendung der Areafunktion arsinh zu einer Varianzstabilisierung und Normalisierung eines Signaldatensatzes über dessen gesamten Intensitätsbereich führt. Dieses Verfahren wurde insbesondere anhand von Mikroarray-Genexpressionsdaten gezeigt, ist jedoch im Rahmen der vorliegenden Erfindung auch auf andere Verfahren zur Messung der Genexpression übertragbar.

Somit wird durch die genannte Transformation mittels der Areafunktion die häufig bei der Auswertung von Signalen beobachtete Abhängigkeit der Varianz von der Signalintensität ausgeglichen..

f) Filtern

Die technischen Replikate (mehrfache Spots derselben Probe) auf dem Mikroarray werden aus den korrigierten und transformierten Signalintensitäten abhängig von ihrer Spot-Qualität herausgefiltert. Für jeden Spot werden die Replikate mit der höchsten Kennzeichnung ausgewählt und die zugehörige Signalintensität gemittelt. Die Expression von Spots mit ausschließlich nicht messbaren Replikaten werden mit "NA" (not available) gekennzeichnet.

Eine weitere Anwendung des erfindungsgemäßen Verfahrens besteht in der Messung der differentiellen Genexpression für die therapiebegleitende Bestimmung der Wahrscheinlichkeit, dass Patienten auf die geplante Therapie ansprechen werden, und/oder für die Bestimmung des Ansprechens auf eine spezialisierte Therapie und/oder auf die Festlegung des Therapieendes im Sinne eines "drug monitoring" bei Patienten mit SIRS und Sepsis und deren Schweregrade. Hierzu wird aus den in zeitlichen Abständen gesammelten Blutproben des Patienten die RNA (Test-RNA und Kontroll-RNA) isoliert. Die verschiedenen RNA-Proben werden zusammen markiert und mit ausgewählten Testgenen sowie Kontrollgenen welche auf einem Microarray immobilisiert sind, hybridisiert. Aus den Expressionsverhältnissen zwischen einzelnen oder mehreren Kontrollgenen und Testgenen wie zB. TNF alpha lässt sich somit beurteilen, welche Wahrscheinlichkeit besteht, dass Patienten auf die geplante Therapie ansprechen werden und/oder ob die begonnene Therapie wirksam ist und/oder wie lange die Patienten noch entsprechend therapiert werden müssen und/oder ob der maximale Therapieeffekt mit der verwendeten Dosis und Dauer schon erreicht worden ist.

Eine weitere Anwendung des erfindungsgemäßen Verfahrens besteht in der Verwendung der RNA der erfindungsgemäßen Gene zur Gewinnung von quantitativen Informationen durch Hybridisierungs-unabhängige Verfahren, insbesondere enzymatische oder chemische Hydrolyse, Surface Plasmon ResonanzVerfahren (SPR-Verfahren), anschließende Quantifizierung der Nukleinsäuren und/oder von Derivaten und/oder Fragmenten derselben

Die mittels PCR (auch weitere Amplifikationsverfahren wie beispielsweise NASBA) amplifizierten und quantifizierten Transkripte von Kontrollgenen stellen eine weitere Ausführungsform gemäß der vorliegenden Erfindung zur Normalisierung von Genexpressionsdaten bei der Unterscheidung von SIRS und Sepsis und deren Schweregrade dar. Die Intensitätssignale der amplifizierten Transkripte werden im Anschluss durch geeignete Messgeräte (PCR-Fluoreszenzdetektor) gemessen und durch weitere softwaregestützte Auswertungen analysiert. Aus den gemessenen Signalintensitäten werden die Expressionsverhältnisse zwischen den Testgenen der Patientenprobe und den Kontrollgenen bestimmt. Aus den Expressionsverhältnissen der unter- und/oder überregulierten Gene lassen sich, wie in den nachstehend dargestellten Experimenten, Rückschlüsse auf die Unterscheidung SIRS und Sepsis und deren Schweregrade ziehen.

Eine weitere Anwendung des erfindungsgemäßen Verfahrens besteht in der Verwendung der über PCR oder andere Amplifikationsverfahren mit nachfolgender Quantifizierung ermittelten Genaktivitäten zur Normalisierung von Genexpressionsdaten zur Unterscheidung von SIRS und Sepsis und deren Schweregrade für die elektronischen Weiterverarbeitung zum Zweck der Herstellung von Software für Diagnosezwecke (z.B. für die Ermittlung des Focus einer Entzündung und zur Einschätzung der Schwere einer individuellen Immunantwort insbesondere bei bakterieller Infektion, auch im Rahmen von Patientendatenmanagementsystemen oder Expertensystemen) oder zur Modellierung zellulärer Signalübertragungswegen.

Eine weitere Anwendung des erfindungsgemäßen Verfahrens besteht in der Bestimmung einer mRNA Menge in einer Probe umfassend a) Isolation der Nukleinsäuren, b) eine Messung des Expressionswertes einer oder mehrerer Nukleinsäuren ausgewählt aus SEQ ID 22 bis SEQ-ID 97; c) einen Vergleich der Expressionswerte der ausgewählten Nukleinsäuren mit bekannten Prozentwerten der Nukleinsäuren an der Gesamtmenge an mRNA; d) Extrapolation der Expressionswerte einer oder mehrerer Nukleinsäuren ausgewählt aus SEQ ID 22 bis SEQ-ID 97 auf die Gesamtmenge an mRNA und d) Bestimmung der Gesamtmenge an mRNA in der Probe.

Eine weitere Anwendung des erfindungsgemäßen Verfahrens besteht in der Normalisierung einer ggf. amplifizierten mRNA Menge in mehreren Proben umfassend a) einen Vergleich der Expressionswerte einer oder mehrerer Nukleinsäuren ausgewählt aus SEQ ID 22 bis SEQ-ID 97 über verschiedene Proben; b) Ableitung eines Wertes zur Normalisierung von Expressionswerten einer oder mehrerer Nukleinsäuren ausgewählt aus SEQ ID 22 bis SEQ-ID 97 über mehrere Proben und c) eine Normalisierung der Expression anderer Nukleinsäuren, welche aus mehreren Proben isoliert wurden, basierend auf Schritt b)

Die Erfindung kann ferner einen Kit betreffen, der eine Auswahl von Sequenzen gemäß SEQ-ID 22 bis SEQ-ID 97 und/oder Genfragmenten davon mit wenigstens 1-100, in bevorzugter Ausführung 1-5 und 1-10 Nukleotiden zur Bestimmung von Genexpressionsprofilen in vitro in einer Patientenprobe, für die Verwendung als Kontrollgene enthält.

Die Erfindung kann ferner auch einen Kit betreffen, der eine Auswahl von Hybridisierungssonden gemäß SEQ-ID No. 1 bis SEQ-ID No. 7 und/oder Genfragmenten davon mit wenigstens 50 Nukleotiden zur Bestimmung von Genexpressionsprofilen *in vitro* in einer Patientenprobe enthält, für die Verwendung als Kontrollgene.

Die Erfindung kann ebenso einen Kit betreffen, der eine Auswahl von Primersonden gemäß SEQ-ID No. 8 bis SEQ-ID No. 21 und/oder Genfragmenten davon mit wenigstens 15 Nukleotiden zur Bestimmung von Genexpressionsprofilen *in vitro* in einer Patientenprobe, enthält, für die Verwendung als Kontrollgene.

In ihrer breitesten und allgemeinsten Fassung betrifft die vorliegende Erfindung folgende Ausführungsformen :

A) Wenigstens ein Kontrollgen zur Normalisierung von Genexpressionsanalysedaten aus Blutproben eines Patienten, wobei das Kontrollgen ausgewählt ist aus folgenden RNA-Sequenzen : SEQ-ID 22 bis SEQ-ID 97, insbesondere SEQ-ID 87, SEQ-ID 89, SEQ-ID 90, SEQ-ID 91, SEQ-ID 93, SEQ-ID 95 und SEQ-ID 96.

B) Wenigstens einen Primer, abgeleitet aus den Kontrollgenen gemäß A) zur Normalisierung von auf Nukleinsäureamplifikation basierenden Genexpressionsanalysedaten, aus Blutproben eines Patienten, wobei der Primer ausgewählt ist aus folgenden DNA-Sequenzen: SEQ-ID 8 bis SEQ-ID 21.

C) Wenigstens eine Sonde, abgeleitet aus den Kontrollgenen gemäß B) zur Normalisierung von Genexpressionsanalysedaten aus Blutproben eines Patienten, wobei der Sondensatz folgende DNA-Sequenzen umfasst: Seq-ID 1 bis Seq-ID 7 sowie deren komplementäre Nukleinsäuresequenzen.

D) Ein Verfahren zur Normalisierung von Genexpressionsanalysedaten mit wenigstens einer Kontrollnukleinsäure, ausgewählt aus den Kontrollgenen gemäß A) oder einem Primer-Satz gemäß B) oder einem Sonden-Satz gemäß C), wobei

a) wenigstens ein Genexpressionsanalyse-Assay an Blutproben eines Patienten *in vitro* durchgeführt wird;
b) als Basis für die Normalisierung der Genexpressionsanalysedaten der zu untersuchenden Proben wenigstens eine Kontrollnukleinsäure im selben Assay mit untersucht wird;
c) Signale aus den Genexpressionsanalysen erfasst werden, welche das Ausmaß der Genexpression einer Mehrzahl von Genen sowie der wenigstens einen Kontrollnukleinsäure wiedergeben;
d) die in Schritt c) erhaltenen Signaldaten einer mathematischen Transformation unterzogen werden, um die technische Variabilität der Signaldaten wenigstens abzuschwächen; und somit
e) die Signaldaten der zu untersuchenden Proben zu normalisieren.

E) Bevorzugte Ausführungsformen des Verfahrens gemäß D) sind:

Ein Verfahren nach D), wobei die mathematische Transformation der Signaldaten mittels des arsinh oder mittels eines logarithmischen Ansatzes durchgeführt wird;

und/oder

das Genexpressions-Assay ausgewählt wird aus:

f) Isolation von Nukleinsäuren aus einer Blutprobe;
g) ggf. einer Co-Amplifikation eines Kontrollnukleinsäuresatzes sowie den zu testenden Nukleinsäuren; und
h) Sondenhybridisierung;

und/oder

die Nukleinsäuren mRNA oder microRNA umfassen;

und/oder

die Nukleinsäuren mittels PCR, real time-PCR, NASBA, TMA oder SDA amplifiziert werden;

und/oder

die Expressionswerte der Kontroll- und Testnukleinsäuren mittels Hybridisierungsverfahren ermittelt werden;

und/oder

die Messung der Expressionswerte der Kontroll- und/oder Testnukleinsäuren in Lösung oder an Nukleinsäuren, die an einem Träger immobilisiert sind, erfolgt;

und/oder

der Träger ein Microarray, Partikel, Bead, Glas, Metall oder Membran ist;

und/oder

die Kontroll- und/oder Test-Nukleinsäuren indirekt über andere Bindungspartner wie Antikörper, Antigene, Oligonukleotide, Molecular beacons oder Enzyme an den Träger gekoppelt sind;

und/oder

die *in vitro* aus einer Patientenprobe ermittelten Expressionswerte der Kontroll- und Testnukleinsäuren als Inputparameter für die Herstellung von Software für die Beschreibung der individuellen Prognose eines Patienten, für Diagnosezwecke, für Therapieentscheidungen und/oder Patientendatenmangementsysteme, eingesetzt werden.

F) Eine Verwendung wenigstens einer Kontrollnukleinsäure, ausgewählt aus den Kontrollgenen gemäß A) oder einem Primer gemäß B) oder einer Sonde gemäß C), zur Normalisierung eines Genexpressionsanalyse-Verfahrens zur Diagnose von Erkrankungen mit systemischer Immunreaktion.

G) Bevorzugte Ausführungsformen der Verwendung gemäß F) sind:

Eine Verwendung nach F), wobei die Erkrankungen ausgewählt sind aus: Sepsis, schwerer Sepsis, septischem Schock oder Multiorganversagen;

und/oder

in einem Verfahren zur *in vitro* Diagnose von SIRS, Sepsis, schwerer Sepsis, septischem Schock oder Multiorganversagen in einem Individuum unter Verwendung von Kontrollnukleinsäuresätzen und Testnukleinsäuren, deren Expression spezifisch für SIRS oder Sepsis sind, die folgenden Schritte umfassend:

a) gleichzeitige Isolation der Kontroll- und Testnukleinsäuren aus einer Probe des Individuums,
b) ggf. Amplifikation der Kontroll- und Testnukleinsäuren,
c) Bestimmung der Expressionswerte der Kontroll- und Testnukleinsäuren
d) eine Normalisierung der Genexpression der Testnukleinsäuren basierend auf den Expressionswerten der Kontrollnukleinsäuren
e) Bestimmung ob die normalisierten Expressionswerte der Testnukleinsäure einen spezifischen Wert für SIRS, Sepsis, schwerer Sepsis, septischem Schock oder Multiorganversagen erreicht haben.

Grundsätzlich gilt für Datentransformation/Normalisierung im Rahmen der vorliegenden Erfindung auch Folgendes:

1. Variante: (wird bei PCR-Experimenten oder auch bei kleinen diagnostischen Arrays als Normalisierung vorgeschlagen)
Die Signale der Kontrollgene werden aggregiert und anschließend das Verhältnis der Signale der Testgene zum aggregierten Signal der Kontrollgene berechnet. Im Fall von logarithmierten Signalen besteht das Verhältnis dann aus der Differenz.
2. Variante: (z.B. Huber et al. [19] bei "whole genome"-Ansätzen bzw. großen Arrays) Die Signale der Kontrollgene werden verwendet, um die Parameter einer geeigneten Transformation oder die Transformation selbst zu schätzen.

Anschließend wird diese Transformation auf die Testgene angewendet
Weitere Vorteile und Merkmale der vorliegenden Erfindung ergeben sich aufgrund der Beschreibung von Ausführungsbeispielen.

Ausführungsbeispiel 1

**Identifizierung von Kontrollgenen aus Blut und aus Blutzellen**

Messung der Genexpression:

Es wurde die Genexpression von 372 Intensivstations-Patienten (ITS-Patienten) gemessen. Alle Patienten wurden intensivmedizinisch behandelt. Es wurden dabei pro Patient maximal sieben ITS-Tage berücksichtigt. Bei Patienten mit mehr als sieben ITS-Tagen wurden sieben Tage zufällig ausgewählt. Insgesamt gingen die Daten von 1261 Microarray-Experimenten in die Analysen ein.

Ausgewählte Charakteristika der Patienten sind in den Tabellen 4 und 5 dargestellt. Es werden Angaben zum Alter, Geschlecht, und ACCP/SCCM Kategorien gemacht. Als Referenzproben dienten die Gesamt-RNA aus Zelllinien SIG-M5. Alle Patientenproben wurden mit der Referenzprobe jeweils auf einem Microarray kohybridisiert.

Tabelle 4: Allgemeine Daten der Patienten

| | |
|---|---|
| Anzahl Patienten (Mikroarrays) | 372 (1261) |
| Sterblichkeit | 94 (25,3 %) |
| Geschlecht [W/M] | 113/259 |
| Alter in Jahren | 68 (15) |
| APACHE-II | 16 (9) |
| SAPS-II | 32 (15) |
| SOFA | 8 (4) |
| Liegedauer in Tagen | 8 (22) |
| Angegeben sind jeweils Median und in Klammern der Interquartilsabstand (IQR) | |

Tabelle 5: Operationsbedingte Indikationen zur IST-Aufnahme (Mehrfachnennungen möglich)

| Indikation | Anzahl Patienten |
|---|---|
| Herzkranzgefäße | 153 |
| Herzklappen | 65 |
| Gastrointestinal | 34 |
| Thorax | 17 |
| Polytrauma | 13 |
| Herzperipheriegefäße | 8 |
| Urogenital | 8 |
| Neurochirurgie | 6 |

Experimentelle Beschreibung:

Blutabnahme und RNA-Isolation

Das Vollblut der Patienten wurde auf der Intensivstation von den Patienten mittels des PAXGene Kits gemäß den Vorgaben des Herstellers (Qiagen) abgenommen. Nach Abnahme des Vollblutes wurde die Gesamt-RNA der Proben unter Anwendung des PAXGene Blood RNA Kit gemäß den Vorgaben des Herstellers (Qiagen) isoliert.

Zellkultivierung

Für die Zellkultivierung (Kontrollproben) wurden 19 Kryozellkulturen (SIGM5) (eingefroren in flüssigem Stickstoff) genutzt. Die Zellen wurden jeweils mit 2 ml Iscove's Medium (Biochrom AG) beimpft ergänzt mit 20% fetalen Kälber Serum (FCS). Die Zellkulturen wurden anschliessend für 24 Stunden bei 37° C unter 5% $CO_2$ in 12-well Platten inkubiert. Danach wurde der Inhalt von 18 Wells in 2 Teile mit jeweils dem gleichen Volumen geteilt, sodass schliesslich 3 Platten des gleichen Formats (insgesamt 36 Wells) zur Verfügung standen. Die Kultivierung wurde anschliessend für 24 Stunden unter den gleichen Bedingungen fortgeführt. Im Anschluss daran wurden die resultierenden Kulturen von 11 Wells jeder Platte vereint und zentrifugiert (1000 x g, 5 min, Raumtemperatur). Der Überstand wurde verworfen und das Zellpellet in 40 ml des o.g. Mediums gelöst. Diese 40 ml gelöste Zellen wurden in zwei 250 ml Kolben zu gleichen Teilen aufgeteilt und nach 48 Stunden Inkubation und Zugabe von 5 ml des o.g. Mediums wiederum inkubiert. Von den restlichen 2 ml der zwei verbleibendenden Platten wurden 80 $\mu$l in leere Wells der gleichen Platten gegeben, welche bereits vorher mit 1 ml des o.g. Mediums präpariert waren. Nach 48 Stunden Inkubation wurde nur eine der 12 Weil-Platten wie folgt prozessiert: Aus jedem Weil wurden 500 $\mu$l entnommen und vereint. Die daraus resultierenden 6 ml wurden in einen 250 ml Kolben gegeben, welcher ca. 10 ml frisches Medium enthielt. Dieses Gemisch wurde mit 1000 x g 5 Minuten bei Raumtemperatur zentrifugiert und in 10 ml des o.g. Mediums gelöst. Die anschliessende Zellzählung ergab folgendes Ergebnis: 1,5 x $10^7$ Zellen pro ml, 10 ml Gesamtvolumen, Gesamtzahl der Zellen: 1,5 x $10^8$. Da die Zellzahl noch nicht ausreichend war, wurden 2,5 ml des o.g. Zellsuspension in 30 ml des o.g. Mediums in einen 250 ml (75 $cm^2$) Kolben gegeben (insgesamt 4 Kolben). Nach 72 Sunden Inkubationszeit wurden jeweils 20 ml frischen Mediums in die Kolben gegeben. Nach folgender 24-stündiger Inkubation erfolgte die Zellzählung wie oben beschrieben, die eine Gesamtzellzahl von 3,8 x $10^8$ Zellen ergab. Um die gewünschte Zellzahl von 2 x $10^6$ Zellen zu errreichen wurden die Zellen in 47,5 ml des o.g. Mediums in 4 Kolben resuspendiert. Nach einer Inkubationszeit von 24 Stunden wurden die Zellen zentrifugiert und zweimal mit Phosphatpuffer ohne $Ca^{2+}$ und $Mg^{2+}$ (Biochrom AG) gewaschen.

Die Isolation der totalen RNA erfolgt mittels des NucleoSpin RNA L Kits (Machery&Nagel) entsprechend den Angaben des Herstellers. Die oben beschriebene Prozedur wurde wiederholt bis die erforderliche Zellzahl erreicht wurde. Dies war erforderlich, um die erforderliche Menge von 6 mg Gesamt-RNA zu erreichen, was etwa einer Effizienz von 600 $\mu$g RNA pro $10^8$ Zellen entspricht.

Reverse Transkription / Markierung / Hybridisierung

Nach Abnahme des Vollblutes wurde die Gesamt-RNA der Proben unter Verwendung des PAXGene Blood RNA Kits (PreAnalytiX) gemäss den Vorgaben des Herstellers isoliert und auf ihre Qualität geprüft. Von jeder Probe wurden 10 $\mu$g Gesamt-RNA aliquotiert und zusammen mit 10 $\mu$g total RNA aus SIGM5-Zellen als Referenz-RNA zu komplementärer DNA (cDNA) mit der reversen Transkriptase Superscript II (Invitrogen) umgeschrieben und die RNA anschließend durch alkalische Hydrolyse aus dem Ansatz entfernt. Im Reaktionsansatz wurde ein Teil des dTTP durch Aminoallyl-dUTP (AA-dUTP) ersetzt, um später die Kopplung des Fluoreszenzfarbstoffes an die cDNA zu ermöglichen.

Nach der Aufreinigung des Reaktionsansatzes wurden die cDNA der Proben und Kontrollen mit den Fluoreszenzfarbstoffen Alexa 647 und Alexa 555 kovalent markiert und auf einem Microarray der Firma SIRS-Lab hybridisiert. Auf dem verwendeten Microarray befinden sich 5308 immobilisierte Polynukleotide mit einer Länge von 55 - 70 Basenpaaren, die jeweils ein humanes Gen repräsentieren und Kontrollspots zur Qualitätssicherung. Ein Microarray unterteilt sich in 28 Subarrays mit einem Raster von 15x15 Spots.

Die Hybridisierung und das anschliessende Waschen bzw. Trocknen wurde in der Hybridisierungsstation HS 400 (Tecan) nach Angaben des Herstellers über 10,5 Stunden bei 42 °C durchgeführt. Die verwendete Hybridisierungslösung besteht aus den jeweiligen markierten cDNA-Proben, 3,5x SSC (1x SSC enthält 150 mM Natriumchlorid und 15 mM Natriumcitrat), 0,3% Natriumdodecylsulfat (V/V) 25% Formamid (V/V) und je 0,8 $\mu$g $\mu$l-1 cot-1 DNA, Hefe t-RNA und poly-A RNA. Das anschliessende Waschen der Mikroarrays wurde mit nachfolgendem Programm bei Raumtemperatur in durchgeführt: je 90 Sekunden spülen mit Waschpuffer 1 (2x SSC, 0,03% Natriumdodecylsulfat), mit Waschpuffer 2 (1x SSC) und abschließend mit Waschpuffer 3 (0,2x SSC). Danach wurden die Mikroarrays unter einem Stickstoffstrom mit einem Druck von 2,5 bar bei 30 °C über 150 Sekunden getrocknet.

Nach der Hybridisierung wurden die Hybridisierungssignale der Microarrays mit einem GenePix 4000B Scanner (Axon) ausgelesen und die Expressionsverhältnisse der differenziert exprimierten Gene mit der Software GenePix Pro 4.0 (Axon) bestimmt.

Auswertung:

Für die Auswertung wurde die mittlere Intensität eines Spots als der Medianwert der zugehörigen Spotpixel bestimmt.

Vorauswahl von Genproben:

Für eine erste Vorauswahl der Gensonden erfolgte die Korrektur systematischer Fehler nach dem Ansatz von Huber et al. [5]. Dabei wurden der additive und der multiplikative Bias innerhalb eines Microarrays aus 75% der vorhandenen Genproben geschätzt.

Es wurden anschliessend die normalisierten und transformierten Verhältnisse der Signale der Patientenproben gegen die allgemeine Kontrolle berechnet. D.h. für das j-te Gen des k-ten Arrays ergab die Berechnung den Wert

$$G_{j,k} = \text{arsinh}(Scy5(j,k)) - \text{arsinh}(Scy3(j,k))$$

wobei [Scy3(j,k), Scy5(j,k)] das zugehörige Fluoreszenzignalpaar bezeichnet. Für alle Gensonden wurde anschließend der Median der absoluten Abweichungen vom Median (MAD), d.h. MAD($G_{j,1}$, ..., $G_{j,1261}$), berechnet und die 10% Gensonden mit dem kleinsten MAD ausgewählt. Als zweites Kriterium für die Vorauswahl wurde die mittlere Signalintensität arsinh(Scy5(j,k)) + arsinh(Scy3(j,k)) herangezogen. Es wurden in den weiteren Analysen nur Gensonden berücksichtigt, deren Median der mittleren Signalintensität im sogenannten dynamischen Signalbereich, vorzugsweise zwischen 6 und 8 (auf der logarithmischen Skala) lag.

Auswahl der Kontrollgene:

Es wurden für die vorausgewählten Gensonden relative Quantitäten berechnet, indem der höchste Expressionswert auf 1 gesetzt wurde. Anschließend wurde das Genstabilitätsmaß M von Vandesompele et al. [6] berechnet. Mittels der ebenfalls in Vandesompele et al. beschriebenen schrittweisen Prozedur, bei der in jedem Schritt das Gen mit der geringsten Stabilität entfernt wird, wurden die Gensonden nach ihrer Stabilität angeordnet. Als oberen Schwellenwert für die Auswahl der Gensonden wurde der (gerundete) Wert 0.6 für den Mittelwert des Stabilitätsmaßes M zugrunde gelegt (Tabelle 6).

Die mathematische Definition für das Genstabilitätsmaß M lautet gemäß Vandesompele et al.:

Für jede Kombination zweier interner Kontrollgene j und k, ist ein Array $A_{jk}$ von m Elementen gegeben, welches aus den $\log_2$-transformierten Expressionsverhältnissen $a_{ij}/a_{ik}$ (Gleichung 1) besteht. Die paarweise Variation $V_{jk}$ für die Kontrollgene j and k wird ferner als Standardabweichung der Elemente $A_{jk}$ definiert (Gleichung 2), wobei SD die Standardabweichung ist. Das Genstabilitätsmaß $M_j$ für das Kontrollgen j ist dann das arithmetische Mittel sämtlicher paarweisen Variationen $V_{jk}$ (Gleichung 3):

(Für alle j,k gilt $\in$ [1 ,n] und j $\neq$ k):

$$A_{jk} = \left[\log_2\left(\frac{a_{1j}}{a_{1k}}\right), \log_2\left(\frac{a_{2j}}{a_{2k}}\right), \ldots, \log_2\left(\frac{a_{mj}}{a_{mk}}\right)\right] = \left[\log 2\left(\frac{a_{ij}}{a_{ik}}\right)\right]_{i=1\rightarrow m} \qquad (1)$$

$$V_{jk} = SD(A_{jk}) \qquad (2)$$

$$Mj = \frac{\sum_{k=1}^{n} V_{jk}}{n-1} \qquad (3)$$

Es wurde ein Cluster an 76 spezifischen Sequenzen mit unveränderter Genaktivität entsprechend den SEQ-ID No. 22 bis SEQ-ID No. 97 ermittelt, die Bestandteil des angefügten Sequenzprotokolls sind.

Tabelle 6: Ermittelte Kontrollgene (RNA-Basis) und deren Stabilitätswerte

| Seq-ID | GenBank Accession Nummer | MAD der Signalratios | Median der mittleren Intensitäten | Stabilität M |
|---|---|---|---|---|
| 22 | NM_024081 | 0,200 | 7,190 | 0,368 |

(fortgesetzt)

| Seq-ID | GenBank Accession Nummer | MAD der Signalratios | Median der mittleren Intensitäten | Stabilität M |
|---|---|---|---|---|
| 23 | AA398364 | 0,179 | 6,730 | 0,385 |
| 24 | N34546 | 0,171 | 6,265 | 0,401 |
| 25 | AA659421 | 0,212 | 7,127 | 0,380 |
| 26 | AA682479 | 0,218 | 6,209 | 0,373 |
| 27 | AK024118 | 0,172 | 6,601 | 0,457 |
| 28 | AA923316 | 0,197 | 6,891 | 0,374 |
| 29 | BM309952 | 0,205 | 7,533 | 0,417 |
| 30 | AI093653 | 0,156 | 7,120 | 0,355 |
| 31 | AI131415 | 0,156 | 6,881 | 0,413 |
| 32 | AI263527 | 0,173 | 6,614 | 0,379 |
| 33 | AA282242 | 0,181 | 6,758 | 0,381 |
| 34 | CR740270 | 0,191 | 6,360 | 0,346 |
| 35 | BG191861 | 0,191 | 6,292 | 0,377 |
| 36 | AI301257 | 0,244 | 6,039 | 0,401 |
| 37 | AI310464 | 0,202 | 6,229 | 0,423 |
| 38 | AW964023 | 0,204 | 6,776 | 0,380 |
| 39 | AI351933 | 0,171 | 6,478 | 0,414 |
| 40 | AA100540 | 0,196 | 7,180 | 0,365 |
| 41 | AI362368 | 0,199 | 6,967 | 0,397 |
| 42 | AI817134 | 0,167 | 6,592 | 0,362 |
| 43 | AI381377 | 0,193 | 6,179 | 0,401 |
| 44 | AI520967 | 0,188 | 6,534 | 0,386 |
| 45 | AA253470 | 0,182 | 7,002 | 0,365 |
| 46 | AI559304 | 0,195 | 7,408 | 0,369 |
| 47 | AI565002 | 0,182 | 7,149 | 0,381 |
| 48 | AI587389 | 0,197 | 7,006 | 0,355 |
| 49 | AI609367 | 0,206 | 6,648 | 0,354 |
| 50 | AI635278 | 0,200 | 6,629 | 0,427 |
| 51 | AI702056 | 0,208 | 6,370 | 0,391 |
| 52 | AI707917 | 0,177 | 6,392 | 0,414 |
| 53 | AI733176 | 0,209 | 6,211 | 0,411 |
| 54 | AI769053 | 0,210 | 7,570 | 0,383 |
| 55 | AI798545 | 0,167 | 7,289 | 0,394 |
| 56 | AI801425 | 0,174 | 6,780 | 0,406 |
| 57 | AI801595 | 0,188 | 7,061 | 0,409 |
| 58 | AI809873 | 0,200 | 7,207 | 0,413 |
| 59 | AI862063 | 0,173 | 7,001 | 0,347 |

(fortgesetzt)

| Seq-ID | GenBank Accession Nummer | MAD der Signalratios | Median der mittleren Intensitäten | Stabilität M |
|---|---|---|---|---|
| 60 | AI923251 | 0,197 | 7,085 | 0,359 |
| 61 | AI925556 | 0,178 | 6,924 | 0,329 |
| 62 | AI932551 | 0,177 | 7,191 | 0,415 |
| 63 | AI932884 | 0,182 | 7,430 | 0,409 |
| 64 | AI933797 | 0,204 | 6,834 | 0,423 |
| 65 | AI933967 | 0,193 | 7,007 | 0,443 |
| 66 | AI935874 | 0,203 | 7,166 | 0,388 |
| 67 | H06263 | 0,169 | 7,140 | 0,337 |
| 68 | H22921 | 0,241 | 6,445 | 0,408 |
| 69 | H54423 | 0,175 | 7,046 | 0,385 |
| 70 | N22551 | 0,205 | 6,830 | 0,387 |
| 71 | N73510 | 0,181 | 7,084 | 0,388 |
| 72 | R06107 | 0,164 | 7,067 | 0,352 |
| 73 | R42511 | 0,212 | 6,110 | 0,371 |
| 74 | R43088 | 0,215 | 6,067 | 0,398 |
| 75 | NM_181705 | 0,208 | 6,821 | 0,383 |
| 76 | R92455 | 0,203 | 6,629 | 0,410 |
| 77 | R93174 | 0,211 | 7,164 | 0,358 |
| 78 | T77995 | 0,201 | 7,251 | 0,423 |
| 79 | T79815 | 0,197 | 7,270 | 0,417 |
| 80 | T83946 | 0,196 | 7,388 | 0,363 |
| 81 | T95909 | 0,177 | 7,109 | 0,414 |
| 82 | T98779 | 0,186 | 6,964 | 0,416 |
| 83 | AK127462 | 0,198 | 6,784 | 0,367 |
| 84 | W80744 | 0,194 | 6,995 | 0,364 |
| 85 | W86575 | 0,236 | 6,761 | 0,438 |
| 86 | AJ297560 | 0,175 | 7,063 | 0,380 |
| 87 | NM_001562 | 0,192 | 7,021 | 0,516 |
| 88 | BU629240 | 0,214 | 6,696 | 0,401 |
| 89 | NM_001228 | 0,235 | 6,286 | 0,423 |
| 90 | NM_001993 | 0,192 | 6,874 | 0,451 |
| 91 | NM_002209 | 0,201 | 7,676 | 0,425 |
| 92 | NM_002392 | 0,197 | 6,969 | 0,431 |
| 93 | NM_000587 | 0,199 | 6,848 | 0,334 |
| 94 | NM_004379 | 0,222 | 7,135 | 0,415 |
| 95 | BC002715 | 0,182 | 6,685 | 0,502 |
| 96 | NM_003082 | 0,214 | 6,327 | 0,469 |

(fortgesetzt)

| Seq-ID | GenBank Accession Nummer | MAD der Signalratios | Median der mittleren Intensitäten | Stabilität M |
|---|---|---|---|---|
| 97 | AA664688 | 0,192 | 6,610 | 0,396 |

Ausführungsbeispiel 2

Stabilitätsuntersuchung der Kontrollgene anhand von Genexpressionsuntersuchungen von Patienten mit und ohne Sepsis.

Wir zeigen in diesem Ausführungsbeispiel, dass die im ersten Ausführungsbeispiel ermittelten Kontrollgene auch bei intensivmedizinisch behandelten Patienten mit und ohne Sepsis stabil sind. Wir betrachteten hierzu Microarray-Daten von 118 Patienten. Insgesamt wurden 394 Patiententage (Microarrays) analysiert, wobei maximal sieben Tage pro Patient berücksichtigt wurden.

Tabelle 7: Allgemeine Daten der Patienten

| | |
|---|---|
| Anzahl Patienten (Microarrays) | 118 (394) |
| Sterblichkeit | 31 (26,3 %) |
| Geschlecht [W/M] | 41/77 |
| Alter in Jahren [Median (IQR)] | 68,5 (14,8) |

Tabelle 8: Einteilung der Patiententage nach ACCP/SCCM Kategorie sowie weitere diagnostische Parameter

| | ITS Patienten* | SIRS | Sepsis | Schwere Sepsis | Septischer Schock |
|---|---|---|---|---|---|
| Anzahl Tage | 33 | 158 | 24 | 90 | 89 |
| SOFA Score | 7 (3) | 7 (4) | 6 (3,25) | 8 (4) | 10 (3) |
| Anzahl ODFs | 2 (2) | 2 (1) | 1,5 (1) | 3 (2) | 3 (2) |
| PCT [ng/ml] | 1,6 (3,8) | 1,8 (5,4) | 1,2 (5,1) | 2,5 (4,9) | 6,4 (11,5) |
| CRP [mg/l] | 144 (53,9) | 112,5 (106,4) | 141 (87,1) | 133 (105,9) | 170 (146) |
| WBC [no/l] | 7750 (4075) | 11100 (7100) | 13350 (8800) | 12900 (6675) | 16100 (10600) |
| * intensivmedizinisch behandelte Patienten, die kein SIRS oder Sepsis entwickelt haben Angegeben ist jeweils der Median und in Klammern der Interquartilsabstand (IQR). | | | | | |

Um die Anwendbarkeit der Kontrollgene anhand eines Vergleiches von SIRS- und Sepsis-Patienten zu demonstrieren, wurden folgende Testgene ausgewählt (vgl. Tabelle 9).

Tabelle 9: Testgene für den Vergleich von SIRS- und Sepsis-Patienten

| Name | GenBank Accession Nummer | Literatur | Seq-ID |
|---|---|---|---|
| CARD8 | NM_014959 | [7] | 98 |
| CCBP2 | NM_001296 | [8] | 99 |
| CCL26 | NM_006072 | [9] | 100 |
| FADD | NM_003824 | [10] | 101 |
| IL6R | NM_181359 | [11] | 102 |
| ITGB2 | NM_000211 | [12] | 103 |
| MAPK3 | MM_002746 | [13] | 104 |
| MYD88 | NM_002468 | [14] | 105 |

(fortgesetzt)

| Name | GenBank Accession Nummer | Literatur | Seq-ID |
|---|---|---|---|
| TNF | NM_000594 | [15] | 106 |
| TREM1 | NM_018643 | [16] | 107 |

Diese Testgene sind in der wissenschaftlichen Literatur im Zusammenhang mit Sepsis beschrieben.

Für die statistische Analyse wurden 6 Patienten mit schwerer SIRS (SIRS + Organdysfunktionen) und 9 Patienten mit schwerer Sepsis (Sepsis + Organdysfunktionen) ausgewählt (Tabelle 10).

Tabelle 10: Ausgewählte Charakteristika der SIRS- und Sepsispatienten

| | schwere SIRS | schwere Sepsis |
|---|---|---|
| Anzahl Patienten | 6 | 9 |
| Sterblichkeit | 0(0%) | 5(55,6%) |
| Geschlecht [M/W] | 4/2 | 7/2 |
| Alter [Jahre] | 70,5 (7) | 74 (7) |
| SOFA Score | 8 (2,25) | 10 (4) |
| Anzahl ODFs | 3,5 (1,75) | 3(1) |
| PCT [ng/ml] | 3,1 (5,5) | 28,2 (38,8) |
| CRP [mg/l] | 71,2 (15,6) | 206 (180) |
| WBC [no/l] | 14250 (3800) | 15800 (4600) |

Angegeben ist jeweils der Median und in Klammern der Interquartilsabstand (IQR).

Die Normalisierung der zehn Testgene erfolgte mittels der folgenden fünf, zufällig ausgewählten Kontrollgene. Es wurde hierzu die Methode von Vandesompele et al. [6] verwendet (Tabelle 11).

Tabelle 11: Ausgewählte Kontrollgene (Set 1)

| GenBank Accession Nummer | Seq.-ID |
|---|---|
| AI263527 | 32 |
| AW964023 | 38 |
| AI933797 | 64 |
| T98779 | 82 |
| NM_004379 | 94 |

Ein Vergleich mittels dem Zwei-Stichproben t-Test liefert folgendes Ergebnis (Tabelle 12)

Tabelle 12: Genaktivität der Testgene normalisiert mit Set 1 der Kontrollgene

| Gene symbol | Seq-ID | Mean SIRS | Mean Sepsis | p-Wert |
|---|---|---|---|---|
| CARD8 | 98 | 1,85 | 4,32 | 0,045 |
| CCBP2 | 99 | 1,25 | 2,69 | 0,004 |
| CCL26 | 100 | 1,52 | 2,69 | 0,041 |
| FADD | 101 | 1,26 | 3,45 | 0,028 |
| IL6R | 102 | 1,58 | 2,15 | 0.175 |
| ITGB2 | 103 | 1,04 | 2,60 | 0.074 |
| MAPK3 | 104 | 1,26 | 2,49 | 0.052 |

(fortgesetzt)

| Gene symbol | Seq-ID | Mean SIRS | Mean Sepsis | p-Wert |
|---|---|---|---|---|
| MYD88 | 105 | 1,11 | 2,34 | 0.025 |
| TNF | 106 | 1,41 | 2,47 | 0.055 |
| TREM1 | 107 | 1,09 | 1,52 | 0.154 |

Um die Reproduzierbarkeit der Ergebnisse zu demonstrieren, wurde der statistische Vergleich wiederholt, wobei erneut fünf Kontrollgene (Set 2) zufällig ausgewählt wurden (Tabelle 13)

Tabelle 13 : Kontrollgene (Set 2)

| GenBank Accession Nummer | Seq.ID |
|---|---|
| AI609367 | 49 |
| AI862063 | 59 |
| H06263 | 67 |
| R92455 | 76 |
| BC002715 | 95 |

Nach der Normalisierung mittels der Methode von Vandesompele et al. erhalten wir folgende Ergebnisse für den Zwei-Stichproben t-Test (Tabelle 14):

Tabelle 14: Genaktivität der Testgene normalisiert mit Set 2 der Kontrollgene

| Gene symbol | Seq-ID | Mean SIRS | Mean Sepsis | p-Wert |
|---|---|---|---|---|
| CARD8 | 98 | 1,67 | 3,71 | 0,029 |
| CCBP2 | 99 | 1,15 | 2,35 | 0,001 |
| CCL26 | 100 | 1,37 | 2,34 | 0,033 |
| FADD | 101 | 1,15 | 2,98 | 0,015 |
| IL6R | 102 | 1,44 | 1,88 | 0,210 |
| ITGB2 | 103 | 0,97 | 2,27 | 0,050 |
| MAPK3 | 104 | 1,15 | 2,34 | 0,065 |
| MYD88 | 105 | 1,03 | 2,05 | 0,028 |
| TNF | 106 | 1,28 | 2,20 | 0,057 |
| TREM1 | 107 | 0,99 | 1,34 | 0.145 |

Die Ergebnisse zeigen eine sehr gute Reproduzierbarkeit der Ergebnisse. Bei beiden Vergleichen sind identische Marker auf dem 5% bzw. 10% Niveau signifikant.

**Ausführungsbeispiel 3**

Ermittelung der Stabilitätswerte ausgewählter Kontrollgene durch deren spezifische Primer mittels real-time PCR

<u>RNA-Isolation</u>

<u>Aus Vollblut wurde RNA mit Hilfe des PAXgene-Kits (PreAnalytiX) nach Angaben des Hersteller isoliert.</u>

Quantitative Reverse Transkriptase-PCR (RT-PCR)

Durch Reverse Transkription wurde mit Hilfe eines Oligo-dT- Primers mRNA unabhängig von ihrer Sequenz in cDNA umgeschrieben. Die dabei komplementärer zu der eingesetzten mRNA entstandenen cDNA-Stränge, wurden anschließend als Template für verschiedenen PCR-Reaktionen eingesetzt.

a) Für den Ansatz wurden folgende Bestandteile zusammen pipettiert:

- 5 µg eingeengte RNA
- 10µl H2O
- 1µl dNTP (dGTP,dATP,dCTP,dTTP)
- 1µl Oligo dT (0,5µg/µl)

b) 5min bei 70°C, anschließend 5min auf Eis
c) Folgender Mix wurde anschließend zugegeben:

- 4µl RT-Puffer
- 2µl 0,1 M DTT
- 1µl RNase out (RNase Inhibitor)
- 1µl SuperScript Reverse Transkriptase

d) 1 h bei 42 °C inkubieren
e) 15min bei 70°C inkubieren

Polymerase-Kettenreaktion

Mit Hilfe der PCR wurde der ausgewählte DNA-Abschnitt amplifiziert, und anschließend quantifiziert und damit die Stärke der Genexpression der Kontrollgene ermittelt:
Für die PCR wurde das AccuPrime Taq DNA Polymerase System von invitrogen verwendet.
Für einen 25µl Ansatz werden folgende Bestandteile in ein 200µl Tube zusammen pipettiert:

| | |
|---|---|
| 2,5µl | 10X AccuPrime PCR Puffer I |
| 20µl | RNase free $H_2O$ |
| 1µl | Template DNA 1:10 verdünnt (ca. 0,82ng/µl) |
| 1µl | Primermix (je 0,5µl forward-/reverse-Primer entsprechend Tabelle 2) |
| 0,5µl | AccuPrime Taq DNA Polymerase |

Folgendes Programm wird im real-time PCR-Thermocycler (corbett research RG 3000) durchgeführt:

| | | |
|---|---|---|
| 94°C | 2min | |
| 94°C | 30sec | 30 Zyklen |
| 58°C | 30sec | |
| 68°C | 1min | |
| 68°C | 2min | |

Zuerst wurde bei 94°C die Template-DNA vollständig denaturiert und das Enzym aktiviert. Anschließend folgten 30 Amplifikationssyklen bestehend aus Denaturierung bei 94°C, Annealing bei 58°C und Elongation bei 68°C. Im Anschluss an die PCR wurden die Proben auf ein 1,5%iges Agarosegel aufgetragen, um über die Größe der Fragmente die Richtigkeit der Produkte zu überprüfen.

Tabelle 15: Stabilitätswerte ausgewählter Kontrollgene (RNA-Basis) ermittelt durch spezifische Primer und real-time PCR

| Seq-ID | GenBank Accession Nummer | Stabilität M |
|---|---|---|
| 87 | NM_001562 | 1,1028295 |
| 89 | NM_001228 | 1,0377301 |
| 90 | NM_001993 | 1,9214240 |
| 91 | NM_002209 | 1,1226082 |
| 93 | NM_000587 | 1,1679851 |
| 95 | BC002715 | 1,1285312 |
| 96 | NM_003082 | 0,9456845 |

Referenzen

[1] Warrington JA, Nair A, Mahadevappa M, et al., Comparison of human adult and fetal expression and identification of 535 housekeeping/maintenance genes., Physiol Genomics. 2000 Apr 27;2(3):143-7

[2] US 10/551,874, Method for recognising acute generalized inflammatory conditions (SIRS), Sepsis, Sepsis-like conditions and systemic infections

[3] O'Dwyer MJ. Mankan AK, Stordeur P, The occurrence of severe sepsis and septic shock are related to distinct patterns of cytokine gene expression.Shock. 2006 Dec;26(6):544-50.

[4] Bone RC, Balk RA, Cerra FB, et al. (1992) The ACCP/SCCM Consensus Conference Committee (1992) Definitions for Sepsis and organ failure and guidelines for the use of innovative therapies in Sepsis. Chest 101:1656-1662; und Crit Care Med 1992; 20: 864-874.

[5] Huber W, Heydebreck A, Sueltmann H, et al. (2003) Parameter estimation for the calibration and variance stabilization of microarray data. Stat. Appl. in Gen. and Mol. Biol.. Vol. 2, Issue 1, Article 3

[6] Vandesompele J, De Preter K, Pattyn F, et al., Accurate normalization of real-time quantitative RT-PCR data by geometric averiging of multiple internal control genes. Genome Biology 2002, 3(7):research0034.1-0034.11

[7] Razmara M, Srinivasula SM, Wang L, et al., CARD-8 protein, a new CARD family member that regulates caspase-1 activation and apoptosis. J Biol Chem. 2002 Apr 19;277(16):13952-8. Epub 2002 Jan 30.

[8] Coelho AL, Hogaboam CM, Kunkel SL. Chemokines provide the sustained inflammatory bridge between innate and acquired immunity. Cytokine Growth Factor Rev. 2005 Dec;16(6):553-60. Epub 2005 Jun 20.

[9] Yamamoto T, Umegae S, Kitagawa T, Matsumoto K. Intraperitoneal cytokine productions and their relationship to peritoneal sepsis and systemic inflammatory markers in patients with inflammatory bowel disease. Dis Colon Rectum. 2005 May;48(5):1005-15.

[10] Oberholzer C, Oberholzer A, Clare-Salzler, M, Moldawer LL. Apoptosis in sepsis: a new target for therapeutic exploration. FASEB J. 2001 Apr;15(6):879-92.

[11] Andrejko K.M., Chen J., and Deutschman C.S. Intrahepatic STAT-3 activation and acute phase gene expression predict outcome after CLP sepsis in the rat. Am J Physiol Gastrointest Liver Physiol 275: G1423-G1429, 1998.

[12] Piguet P.F., Vesin C., Rochat A. β2 Integrin modulates platelet caspase activation and life span in mice. European Journal of Cell Biology, Volume 80, Number 2, February 2001, pp. 171-177(7).

[13] Riedemann NC, Guo RF, Hollmann TJ, et al., Regulatory role of C5a in LPSinduced IL-6 production by neutrophils during sepsis. FASEB J. 2004 Feb;18(2):370-2. Epub 2003 Dec 19.

[14] Weighardt H, Kaiser-Moore S, Vabulas RM, et al., Cutting edge: myeloid differentiation factor 88 deficiency improves resistance against sepsis caused by polymicrobial infection. J Immunol. 2002 Sep 15;169(6):2823-7.

[15] Hedberg CL, Adcock K, Martin J, et al., Tumor necrosis factor alpha -- 308 polymorphism associated with increased sepsis mortality in ventilated very low birth weight infants. Pediatr Infect Dis J. 2004 May;23(5):424-8.

[16] Gibot S, Kolopp-Sarda MN, Bene MC, et al., A soluble form of the triggering receptor expressed on myeloid cells-1 modulates the inflammatory response in murine sepsis. J Exp Med. 2004 Dec 6;200(11):1419-26.

[17] Brazma A, Hingamp P, Quackenbush J et al., Minimum information about a microarray experiment (MIAME)-toward standards for microarray data, Nature Genetics 29, 365 - 371 (2001)

[18] Rocke DM, Durbin B, A model for measurement error for gene expression arrays., J Comput Biol. 2001;8(6):557-69

[19] Huber W, Heydebreck A, Sueltmann H, Variance stabilization applied to microarray data calibration and to the quantification of differential expression., Bioinformatics. 2002;18 Suppl 1:S96-104.

## Patentansprüche

1. Verfahren zur Normalisierung einer Expression von mRNA in mehreren Blutproben umfassend:

   a) einen Vergleich der Expressionswerte einer oder mehrerer Nukleinsäuren ausgewählt aus SEQ ID 22 bis SEQ-ID 97 über verschiedene Blutproben;
   b) Ableitung eines Genstabilitätmaßes zur Normalisierung von Expressionswerten einer oder mehrerer Nukleinsäuren, ausgewählt aus SEQ ID 22 bis SEQ-ID 97 über mehrere Blutproben; und
   c) eine Normalisierung der Expression anderer Nukleinsäuren, welche aus mehreren Blutproben isoliert wurden, basierend auf Schritt b).

2. Verfahren nach Anspruch 1, **dadurch gekennzeichnet, dass** die mRNA amplifiziert wird, wobei durch reverse Transkription mit Hilfe eines oligo-dT-Primers mRNA in cDNA umgeschrieben wird und die komlementär zu der eingesetzten mRNA entstandenen cDNA-Stränge als Template fürPolymerasekettenreaktionen (PCR) eingesetzt werden.

3. Verfahren nach Anspruch 2, wobei die Nukleinsäuren mittels PCR, oder real time-PCR amplifiziert werden.

4. Verfahren nach einem der Ansprüche 1-3, wobei die Expressionswerte der Nukleinsäuren mittels Hybridisierungsverfahren ermittelt werden.

5. Verfahren nach einem der Ansprüche 1-4, wobei die Messung der Expressionswerte der Nukleinsäuren in Lösung oder an Nukleinsäuren, die an einem Träger immobilisiert sind, erfolgt.

6. Verfahren nach Anspruch 5, wobei der Träger ein Microarray, Partikel, Bead, Glas, Metall oder Membran ist.

7. Verfahren nach einem der Ansprüche 1-6, wobei die Nukleinsäuren indirekt über andere Bindungspartner wie Antikörper, Antigene, Oligonukleotide, Molecular beacons oder Enzyme an den Träger gekoppelt sind.

8. Verfahren nach einem der Ansprüche 1-7, wobei die in vitro aus einer Patientenprobe ermittelten Expressionswerte der Nukleinsäuren als Inputparameter für die Herstellung von Software für die Beschreibung der individuellen Prognose eines Patienten, für Diagnosezwecke, für Therapieentscheidungen und/oder Patientendatenmangementsysteme, eingesetzt werden.

9. Verwendung von Nucleinsäuren, ausgewählt aus der Gruppe bestehend aus: SEQ-ID 22-86, 88, 92, 94 und 97 als Kontrollgene zur Normalisierung von Genexpressionsanalysedaten aus Blutproben.

10. Verwendung eines Kontrollgen-Satzes zur Normalisierung von Genexpressionsanalysedaten aus Blutproben eines Patienten, wobei der Kontrollgen-Satz folgende Nucleinsäuresequenzen umfasst: SEQ-ID 32, SEQ-ID 38, SEQ-ID 64, SEQ-ID 82, und SEQ-ID 94.

11. Verwendung eines Kontrollgen-Satzes zur Normalisierung von Genexpressionsanalysedaten aus Blutproben eines Patienten, wobei der Kontrollgen-Satz folgende Nucleinsäuresequenzen umfasst: SEQ-ID 49, SEQ-ID 59, SEQ-ID 67, SEQ-ID 76, und SEQ-ID 95.

## Claims

1. A method for the normalization of an expression of mRNA in several blood samples, including:

   a) a comparison of the expression values of one or several nucleic acids selected from SEQ ID NO: 22 to SEQ ID NO: 97 across various blood samples;
   b) deriving a gene stability measure for the normalization of expression values of one or several nucleic acids selected from SEQ ID NO: 22 to SEQ ID NO: 97 across several blood samples; and
   c) a normalization of the expression of other nucleic acids that were isolated from several blood samples, based on step b).

2. The method according to claim 1, **characterized in that** the mRNA is amplified, wherein mRNA is rewritten to cDNA by means of reverse transcription with the aid of an oligo-dT primer, and the cDNA strands formed in the process complementarily to the mRNA used are subsequently used as templates for polymerase chain reaction (PCR) reactions.

3. The method according to claim 2, wherein the nucleic acids are amplified by means of PCR or real-time PCR.

4. The method according to one of the claims 1-3, wherein the expression values of the nucleic acids are determined by means of hybridization methods.

5. The method according to one of the claims 1-4, wherein the measurement of the expression values of the nucleic acids takes place in solution or on nucleic acids immobilized on a support.

6. The method according to claim 5, wherein the support is a microarray, particle, bead, glass, metal, or membrane.

**7.** The method according to one of the claims 1-6, wherein the nucleic acids are indirectly coupled to the support through other binding partners such as antibodies, antigenes, oligonucleotides, molecular beacons, or enzymes.

**8.** The method according to one of the claims 1-7, wherein the expression values of the nucleic acids determined in vitro from a patient sample are used as input parameters for the production of software for the description of a patient's individual prognosis, for diagnostic purposes, for therapy decisions, and/or patient data management systems.

**9.** Use of nucleic acids, selected from the group consisting of: SEQ ID NO: 22-86, 88, 92, 94, and 97 as reference genes for the normalization of gene expression analysis data from blood samples.

**10.** Use of a set of reference genes for the normalization of gene expression analysis data from blood samples of a patient, wherein the set of reference genes comprises the following nucleic acid sequences: SEQ ID NO: 32, SEQ ID NO: 38, SEQ ID NO: 64, SEQ ID NO: 82, and SEQ ID NO: 94.

**11.** Use of a set of reference genes for the normalization of gene expression analysis data from blood samples of a patient, wherein the set of reference genes comprises the following nucleic acid sequences: SEQ ID NO: 49, SEQ ID NO: 59, SEQ ID NO: 67, SEQ ID NO: 76, and SEQ ID NO: 95.

**Revendications**

**1.** Procédé pour la normalisation d'une expression d'ARNm dans plusieurs échantillons sanguins, comprenant :

a) une comparaison des valeurs de l'expression d'un ou plusieurs acides nucléiques choisis parmi SEQ ID NO:22 à SEQ ID NO:97, sur différents échantillons sanguins ;
b) la déduction d'une mesure de la stabilité génique pour la normalisation de valeurs de l'expression d'un ou plusieurs acides nucléiques choisis parmi SEQ ID NO:22 à SEQ ID NO:97, sur plusieurs échantillons sanguins ; et
c) une normalisation de l'expression d'autres acides nucléiques, qui ont été isolés à partir de plusieurs échantillons sanguins, en se fondant sur l'étape b).

**2.** Procédé selon la revendication 1, **caractérisé en ce que** l'ARNm subit une amplification, au cours de laquelle un ARNm est transcrit en ADNc par rétro-transcription à l'aide d'une amorce oligo-dT, et les brins d'ADNc, créés d'une manière complémentaire à l'ARNm utilisé, étant utilisés en tant que matrices pour la réaction en chaîne par polymérase (PCR).

**3.** Procédé selon la revendication 2, dans lequel les acides nucléiques sont amplifiés par PCR ou par une PCR en temps réel.

**4.** Procédé selon l'une des revendications 1 à 3, dans lequel les valeurs de l'expression des acides nucléiques sont déterminées par des procédés d'hybridation.

**5.** Procédé selon l'une des revendications 1 à 4, dans lequel la mesure des valeurs de l'expression des acides nucléiques a lieu en solution ou sur des acides nucléiques qui sont immobilisés sur un support.

**6.** Procédé selon la revendication 5, dans lequel le support est un micro-réseau, une particule, une perle, du verre, du métal ou une membrane.

**7.** Procédé selon l'une des revendications 1 à 6, dans lequel les acides nucléiques sont couplés au support d'une manière indirecte par l'intermédiaire d'autres partenaires de liaison tels que des anticorps, des antigènes, des oligonucléotides, des balises moléculaires ou des enzymes.

**8.** Procédé selon l'une des revendications 1 à 7, dans lequel les valeurs de l'expression des acides nucléiques, déterminées *in vitro* à partir d'un échantillon provenant d'un patient, sont utilisées en tant que paramètres d'entrée pour la fabrication d'un logiciel destiné à décrire le pronostic individuel d'un patient, à des fins de diagnostic, pour des décisions thérapeutiques et/ou pour des systèmes de gestion des données patients.

**9.** Utilisation d'acides nucléiques choisis dans le groupe consistant en SEQ ID NO:22-86, 88, 92, 94 et 97 en tant que gènes de contrôle pour la normalisation de données d'analyse de l'expression génique provenant d'échantillons sanguins.

**10.** Utilisation d'un groupe de gènes de contrôle pour la normalisation de données d'analyse de l'expression génique provenant d'échantillons sanguins d'un patient, le groupe de gènes de contrôle comprenant les séquences d'acides nucléiques suivantes : SEQ ID NO:32, SEQ ID NO:38, SEQ ID NO:64, SEQ ID NO:82 et SEQ ID NO:94.

**11.** Utilisation d'un groupe de gènes de contrôle pour la normalisation de données d'analyse de l'expression génique provenant d'échantillons sanguins d'un patient, le groupe de gènes de contrôle comprenant les séquences d'acides nucléiques suivantes : SEQ ID NO:49, SEQ ID NO:59, SEQ ID NO:67, SEQ ID NO:76 et SEQ ID NO:95.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 10551874 B


### In der Beschreibung aufgeführte Nicht-Patentliteratur


- **BRAZMA A et al.** Minimum information about a microarray experiment (MIAME)-toward standards for microarray data. *Nature Genetics,* 2001, vol. 29, 365-371
- **ROCKE DM ; DURBIN B.** A model for measurement error for gene expression arrays. *J Comput Biol.,* 2001, vol. 8 (6), 557-69
- **HUBER W ; HEYDEBRECK A ; SUELTMANN H.** Variance stabilization applied to microarray data calibration and to the quantification of differential expression. *Bioinformatics,* 2002, vol. 18 (1), 96-104
- **WARRINGTON JA ; NAIR A ; MAHADEVAPPA M et al.** Comparison of human adult and fetal expression and identification of 535 housekeeping/maintenance genes. *Physiol Genomics,* 27. April 2000, vol. 2 (3), 143-7
- **O'DWYER MJ ; MANKAN AK ; STORDEUR P.** The occurrence of severe sepsis and septic shock are related to distinct patterns of cytokine gene expression. *Shock,* Dezember 2006, vol. 26 (6), 544-50
- **BONE RC ; BALK RA ; CERRA FB et al.** The ACCP/SCCM Consensus Conference Committee (1992) Definitions for Sepsis and organ failure and guidelines for the use of innovative therapies in Sepsis. *Chest,* 1992, vol. 101, 1656-1662
- *Crit Care Med,* 1992, vol. 20, 864-874
- **HUBER W ; HEYDEBRECK A ; SUELTMANN H et al.** Parameter estimation for the calibration and variance stabilization of microarray data. *Stat. Appl. in Gen. and Mol. Biol.,* 2003, vol. 2 (1
- **VANDESOMPELE J ; DE PRETER K ; PATTYN F et al.** Accurate normalization of real-time quantitative RT-PCR data by geometric averiging of multiple internal control genes. *Genome Biology,* 2002, vol. 3 (7), 0034.1-0034.11
- **RAZMARA M ; SRINIVASULA SM ; WANG L et al.** CARD-8 protein, a new CARD family member that regulates caspase-1 activation and apoptosis. *J Biol Chem.,* 30. Januar 2002, vol. 277 (16), 13952-8
- **COELHO AL ; HOGABOAM CM ; KUNKEL SL.** Chemokines provide the sustained inflammatory bridge between innate and acquired immunity. *Cytokine Growth Factor Rev.,* 20. Juni 2005, vol. 16 (6), 553-60
- **YAMAMOTO T ; UMEGAE S ; KITAGAWA T ; MATSUMOTO K.** Intraperitoneal cytokine productions and their relationship to peritoneal sepsis and systemic inflammatory markers in patients with inflammatory bowel disease. *Dis Colon Rectum.,* Mai 2005, vol. 48 (5), 1005-15
- **OBERHOLZER C ; OBERHOLZER A ; CLARE-SALZLER, M ; MOLDAWER LL.** Apoptosis in sepsis: a new target for therapeutic exploration. *FASEB J.,* April 2001, vol. 15 (6), 879-92
- **ANDREJKO K.M. ; CHEN J. ; DEUTSCHMAN C.S.** Intrahepatic STAT-3 activation and acute phase gene expression predict outcome after CLP sepsis in the rat. *Am J Physiol Gastrointest Liver Physiol,* 1998, vol. 275, G1423-G1429
- **PIGUET P.F. ; VESIN C. ; ROCHAT A.** β2 Integrin modulates platelet caspase activation and life span in mice. *European Journal of Cell Biology,* Februar 2001, vol. 80 (2), 171-177
- **RIEDEMANN NC ; GUO RF ; HOLLMANN TJ et al.** Regulatory role of C5a in LPSinduced IL-6 production by neutrophils during sepsis. *FASEB J.,* 19. Dezember 2003, vol. 18 (2), 370-2
- **WEIGHARDT H ; KAISER-MOORE S ; VABULAS RM et al.** Cutting edge: myeloid differentiation factor 88 deficiency improves resistance against sepsis caused by polymicrobial infection. *J Immunol.,* 15. September 2002, vol. 169 (6), 2823-7
- **HEDBERG CL ; ADCOCK K ; MARTIN J et al.** Tumor necrosis factor alpha -- 308 polymorphism associated with increased sepsis mortality in ventilated very low birth weight infants. *Pediatr Infect Dis J,* Mai 2004, vol. 23 (5), 424-8
- **GIBOT S ; KOLOPP-SARDA MN ; BENE MC et al.** A soluble form of the triggering receptor expressed on myeloid cells-1 modulates the inflammatory response in murine sepsis. *J Exp Med.,* 06. Dezember 2004, vol. 200 (11), 1419-26

- **BRAZMA A ; HINGAMP P ; QUACKENBUSH J et al.** Minimum information about a microarray experiment (MIAME)-toward standards for microarray data. *Nature Genetics,* 2001, vol. 29, 365-371
- **HUBER W ; HEYDEBRECK A ; SUELTMANN H.** Variance stabilization applied to microarray data calibration and to the quantification of differential expression. *Bioinformatics,* 2002, vol. 18 (1), S96-104